(19) Europäisches Patentamt
European Patent Office
Office européen des brevets

(11) EP 2 340 271 B1

(12) # EUROPEAN PATENT SPECIFICATION

(45) Date of publication and mention of the grant of the patent:
**05.12.2018 Bulletin 2018/49**

(21) Application number: **09818692.7**

(22) Date of filing: **09.10.2009**

(51) Int Cl.:
*C08G 63/00* (2006.01) *A61L 17/10* (2006.01)
*C08G 18/00* (2006.01) *A61L 27/34* (2006.01)
*C08G 18/73* (2006.01) *C08G 18/77* (2006.01)
*C08G 18/34* (2006.01) *C08G 18/38* (2006.01)
*C08G 18/42* (2006.01) *C08G 63/02* (2006.01)
*A61K 47/58* (2017.01) *A61K 47/59* (2017.01)

(86) International application number:
**PCT/AU2009/001341**

(87) International publication number:
**WO 2010/040187 (15.04.2010 Gazette 2010/15)**

(54) **POLYMER-BIOACTIVE AGENT CONJUGATES**
KONJUGATE AUS POLYMER-BIOAKTIVEN STOFFEN
CONJUGUÉS POLYMÈRE-AGENT BIOACTIF

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **10.10.2008 AU 2008905262**
**22.12.2008 US 139722 P**

(43) Date of publication of application:
**06.07.2011 Bulletin 2011/27**

(73) Proprietor: **PolyActiva Pty Ltd.**
**Melbourne, VIC 3000 (AU)**

(72) Inventors:
- **O'SHEA, Michael Shane**
  **Mulgrave**
  **Victoria 3170 (AU)**
- **GRAICHEN, Florian Hans Maximilian**
  **Richmond**
  **victoria 3121 (AU)**
- **TAIT, Russell John**
  **Balwyn**
  **Victoria 3103 (AU)**
- **TAING, Heng Chy**
  **Chadstone**
  **Victoria 3148 (AU)**
- **JEFFERY, Justine Leigh**
  **Mitcham**
  **Victoria 3132 (AU)**

(74) Representative: **Barker Brettell LLP**
**100 Hagley Road**
**Edgbaston**
**Birmingham B16 8QQ (GB)**

(56) References cited:
**WO-A1-2005/089778 WO-A1-2009/102795**
**WO-A1-2009/102795 WO-A1-2010/040188**
**WO-A2-02/26867 WO-A2-2004/014973**
**WO-A2-2006/024953 WO-A2-2008/060780**
**US-A- 5 798 115**

- **DAVARAN, S. ET AL.: ''Synthesis and Hydrolysis of Polyurethanes Containing Ibuprofen Pendent Groups' J. BIOACTIVE AND COMPATIBLE POLYMERS vol. 12, 1997, pages 47 - 58, XP008137236**
- **OUCHI, T. ET AL.: 'Design of Poly(a-malic acid)-SFU Conjugate Exhibiting Antitumor Activity' BRITISH POLYMER JOURNAL vol. 23, 1990, pages 221 - 228, XP008137095**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 340 271 B1

## Description

### FIELD OF THE INVENTION

The present invention relates in general to polymer - bioactive agent conjugates. In particular, the invention relates to a bioerodible polymer - bioactive agent conjugate, to a method of preparing a bioerodible polymer - bioactive agent conjugate and to a monomer-bioactive bioactive agent conjugate suitable for preparing a bioerodible polymer - bioactive agent conjugate.

### BACKGROUND OF THE INVENTION

The targeted and controlled delivery of bioactive agents (such as drugs) is an area of considerable current interest. The site-specific delivery of a bioactive agent is a highly desirable feature for the treatment of many different conditions. The use of devices, implanted in the body of humans or animals, which contain bioactive agents is desirable to increase the efficacy and safety of the bioactive agent.

One mode of delivering a bioactive agent to a biological target involves the use of a polymer to carry/retain the bioactive agent to/at a specific location.

An example of such a delivery mode utilises an admixture of a polymer with a bioactive agent. Such admixtures generally result in poor control over the release of the bioactive agent and a significant change in the physical properties of the admixture as the bioactive agent is released.

A further example of such a delivery mode involves the polymerisation of the bioactive agent with other monomers or itself so as to incorporate the bioactive agent as part of the backbone polymer chain. Such polymerised bioactive agents generally result in inefficient release of the bioactive agent as the release of the bioactive agents occurs *via* inactive intermediates. Furthermore, the resulting polymer material generally has quite poor physical properties.

Still a further example of such a delivery mode utilises a bioactive agent covalently bound to a polymer so as to form a polymer -bioactive agent conjugate. Such polymer -bioactive agent conjugates are typically formed by covalently attaching a bioactive agent to a preformed polymer backbone. However, the synthesis of such covalently bound systems can be problematic. In particular, steric and thermodynamic constraints can affect the amount of bioactive agent that can be covalently attached, and also impact on the distribution of the bioactive agent along the polymer backbone, which in turn can reduce control over the release of the bioactive agent.

Yet another example of such a delivery mode utilises a bioactive agent covalently bound to a polymer which has been formed from polymerisation of an ethylenically unsaturated monomer to which the bioactive agent has been previously appended. Such polymers are typically not bioerodible and following administration to a subject may require physical extraction at some later stage. Similar bioerodible polymer - bioactive agent conjugates have been prepared, but these typically bioerode to yield relatively toxic products.

WO 2008/060780 relates to a conjugate containing a PEG moiety and a peptide having in vivo activity which are linked via a glycosyl group.WO2006/024953 relates to glycerol branched PEG conjugates with human growth hormone.WO02/26867 relates to polyester and polyamide dendrimers which act as carriers for active agents.Davaran and Entezami (Journal of Bioactive and Compatible Polymers Vol. 12. January 1997, 47-58) relates to synthesis and hydrolysis of polyurethanes containing pendent ibuprofen groups.Ouchi et al. (British Polymer Journal 23 (1990) 221-228) relates to a macromolecular prodrug of 5-fluorouracil (5-FU) in which 5-FU is covalently attached to poly($\alpha$-malic acid). WO 2005/089778 A1 discloses biocompatible, biodegradable thermoplastic polyurethane or polyurethane/ureas comprising isocyanate, polyol and a conventional chain extender and/or a chain extender having a hydrolysable linking group.

An opportunity therefore remains to develop new polymer - bioactive agent conjugates which address or ameliorate one or more disadvantages or shortcomings associated with existing materials and/or their method of manufacture, or to at least provide a useful alternative to such materials and their method of manufacture.

### SUMMARY OF THE INVENTION

In one aspect, the invention provides a bioerodible polymer - bioactive agent conjugate comprising as part of its polymer backbone a moiety of general formula (I):

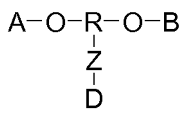

(I)

wherein:

A and B, which may be the same or different, represent the remainder of the polymer backbone and are (i) attached to the -O-R(ZD)-O- moiety as shown in formula (I) via a bioerodible moiety, and (ii) comprise a poly(urethane-ester) having monomeric units coupled via bioerodible urethane or ester moieties;
R represents a linear or branched optionally substituted hydrocarbon;
Z is a linking group; and
D is a releasable bioactive agent.

For avoidance of any doubt, the "moiety of general formula (I)" is intended to be a reference to:

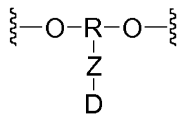

with A and B being presented in formula (I) to (i) more clearly depict that the "moiety" forms part of the polymer backbone, and (ii) define the nature of the remainder of the polymer backbone.

As used herein the expression forming "part of the polymer backbone" means that the moiety of formula (I) (i.e. excluding A and B) is part of the string of atoms that are each connected so as to form the polymer chain (i.e. including A and B). In other words, the moiety of formula (I) is not pendant from the polymer backbone. Having said this, it will be appreciated that groups Z and D in the moiety of formula (I) will be pendant from the polymer backbone.

Examples of A and B are discussed in more detail below, but comprise a poly(urethane-ester) having monomeric units coupled via bioerodible urethane or ester moieties.

Depending on the application, the bioerodible polymer - bioactive agent conjugate may have a single moiety of formula (I), but more typically the conjugate will comprise a plurality of moieties of formula (I). In polymers comprising a plurality of moieties of formula (I), each group represented by A, B, R, Z and D may be the same or different.

For example, the moiety of general formula (I) may in conjunction with a suitable comonomer form a repeat unit of a polyester or polyurethane as illustrated below in general formula (Ia) and (Ib), respectively:

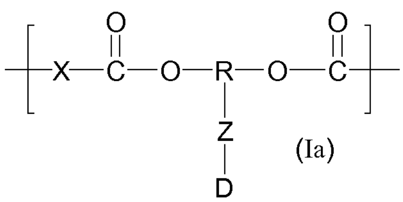

(Ia)

where R, Z and D are as herein defined and X is an optionally substituted alkyl, aryl or alkylaryl group, wherein for each repeat unit of the polyester each R, Z, D and X may be the same or different;

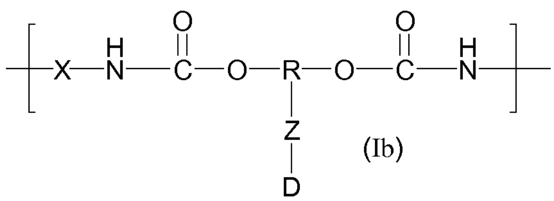

(Ib)

where R, Z and D are as herein defined and X is an optionally substituted alkyl, aryl or alkylaryl group, wherein for each repeat unit of the polyurethane each R, Z, D and X may be the same or different.

An important feature of the polymer - bioactive agent conjugates in accordance with the invention is that they are "bioerodible". By being "bioerodible" is meant that the conjugates have a molecular structure that is susceptible to break down (i.e. a reduction in molecular weight) by chemical decomposition in a biological environment (e.g. within a subject or in contact with biological material such as blood, tissue etc), as opposed to physical degradation. Such chemical decomposition will typically be via the hydrolysis of labile moieties that form part of the molecular structure of the conjugates. In other words, the conjugates will comprise moieties that are susceptible to hydrolytic cleavage. The rate of hydrolysis of the bioerodable polymer may vary over time, or be activated by any number of extrinsic or intrinsic factors (e. g. light, heat, radiation, pH, enzymatic or nonenzymatic cleavage, etc.).

Reference herein to biological material such as "biological tissue" is intended to include cells or tissue *in vivo*

(e. g. cells or tissue of a subject) and *in vitro* (e.g. cultured cells).

By being bioerodible, the conjugates in accordance with the invention can advantageously be used to release a bioactive agent "D", for example within a subject, without the need to subsequently remove the remaining conjugate structure from the subject.

An important feature of the bioerodible properties of the conjugates is that (i) the -OR(ZD)-O- moiety as shown in formula (I) is attached to the remainder of the polymer backbone (represented by A and B) via a bioerodible moiety, and (ii) comprise a poly(urethane-ester) having monomeric units coupled via bioerodible urethane or ester moieties. By having such characteristics, the conjugates in accordance with the invention can advantageously bioerode into substantially non-toxic residues.

As used herein the expression "bioerodible moiety" is intended to mean a moiety that can undergo chemical decomposition in a biological environment. Such chemical decomposition will typically be via hydrolysis. In other words, the bioerodible moiety with be susceptible to hydrolytic cleavage. In the context of the present invention, the bioerodible moieties function to link or couple the monomeric units that form the polymer backbone of the conjugates. Accordingly, it will be appreciated that the bioerodible moieties give rise to the bioerodible property of the conjugates.

Those skilled in the art will appreciate the type of moieties that are typically susceptible to hydrolytic cleavage in a biological environment. Such moieties may include anhydride, amide, urethane (carbamate), and ester. The conjugates in accordance with the invention may include a combination of such moieties.

Those skilled in the art will appreciate the type of moieties that are not typically susceptible to hydrolytic cleavage in a biological environment. Such moieties may include carbonyl, siloxane, sulfone, ether, olefin (i.e. C-C, e.g. alkylene, alkenylene and alkynylene) and halogeninated olefin.

In accordance with the invention, A and B, which may be the same or different, represent the remainder of the polymer backbone and are "attached to the -O-R(ZD)-O- moiety as shown in formula (I) via a bioerodible moiety". By this is meant that the O atoms in the - O-R(ZD)-O- moiety each form part of a bioerodible moiety. For example, the O atoms in the -O-R(ZD)-O- may each independently form part of an ester or urethane moiety as illustrated below where O* represents the O atom in the -O-R(ZD)-O- moiety:

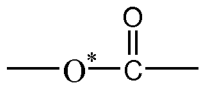
ester

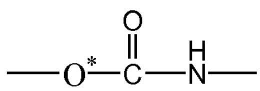
urethane

In one embodiment, the O atoms in the -O-R(ZD)-O- each independently form part of an ester or urethane moiety.

In addition to A and B being attached to the -O-R(ZD)-O- moiety via a bioerodible moiety, A and B are also "each formed from monomeric units that are coupled via a bioerodible moiety". By this is meant that the polymer segments A and B only include monomeric units that are coupled to each other via a bioerodible moiety. By "monomeric units" is meant the building blocks that are polymerised to form the polymer segments A and B. The monomeric units may in their own right be macro-monomeric units (i.e. monomeric units that are typically low molecular weight polymers and contain monomeric units in their own right). Where the monomeric units are macro-monomeric units, they too must be formed from monomeric units that are coupled via a bioerodible moiety.

A and/or B comprise a poly(urethane-ester) having monomeric units coupled via bioerodible urethane or ester moieties. A and/or B may also be a poly(urethane-ester) formed by polymerising a diisocyanate with a polyester macromonomer or macromer. In that case, the polyester macromer will be formed from monomeric units that are coupled via a bioerodible moiety (as discussed above), and the polymerisation of it with the diisocyanate will give rise to the poly(urethane-ester) having monomeric units that are all coupled via a bioerodible urethane or ester moiety.

Accordingly, it will be appreciated that the present invention is not intended to embrace the situation where A and/or B comprise monomeric units that are coupled to each other via a non-bioerodible moiety. For example, A and/or B can not be a polyether. A and/or B also can not be a polymer comprising a polyether such as a poly(urethane-ether) or poly(ester-ether) formed by polymerising a diisocyanate and diacid, respectively, with a polyether macromer (e.g. polyalkylene glycols such as polyethylene glycol and polypropylene glycol). In that case, the polyether macromer will be formed from monomeric units (e.g.-$(OR)_n$-) that are coupled via a non-bioerodible moiety (i.e. an ether), and the polymerisation of it with the diisocyanate or diacid will give rise to the poly(urethane-ether) or poly(ester-ether), respectively, that has monomeric units coupled via non-bioerodible moieties.

The bioerodible characteristics of the conjugates in accordance with the invention advantageously enable its polymer backbone to degrade into substantially non-toxic residues. This is in contrast with polymer - bioactive agent conjugates that comprise within their polymer backbone a non-bioerodible polymer segment such as a polyether or

polyvinyl that may give rise to toxic residues. For example, some polyethers are known to be toxic to humans and animals.

Furthermore, low molecular weight diols such as $C_{2-10}$, $C_{2-6}$, $C_{2-3}$, $C_2$ diols (e.g. ethylene glycol and propylene glycol) can also be toxic to humans and animals. Low molecular weight diols are commonly used as comonomers or chain extenders in the manufacture of polymers such polyurethanes and polyesters. If such diols are used in preparing the bioerodible polymers of the invention they can subsequently be liberated upon its bioerosion. Accordingly, it can be desirable to limit their use in the bioerodible polymers of the invention. In contrast, higher alcohols such as triols are typically less toxic to humans and animals.

In one embodiment, the bioerodible polymers of the invention comprise less than 25 mol%, less than 10 mol%, or less than 5 mol% of polymerised residues that are derived from a low molecular weight diol ($C_{2-10}$, $C_{2-6}$, $C_{2-3}$, or $C_2$ diol).

In a further embodiment, the bioerodible polymers of the invention comprise substantially no polymerised residues of a low molecular weight diol ($C_{2-10}$, $C_{2-6}$, $C_{2-3}$, or $C_2$ diol).

In still a further embodiment, the bioerodible polymers of the invention are prepared using less than 25 mol%, less than 10 mol%, or less than 5 mol% of a low molecular weight diol ($C_{2-10}$, $C_{2-6}$, $C_{2-3}$, or $C_2$ diol).

In yet a further embodiment, the bioerodible polymers of the invention are prepared using substantially no low molecular weight diol ($C_{2-10}$, $C_{2-6}$, $C_{2-3}$, or $C_2$ diol).

The mol% diol/diol residue values referred to herein are those relative to the total moles of diol/diol residue.

The bioerodible polymer - bioactive agent conjugates in accordance with the invention may form part of or be formed into an article or device *per se* or can be presented as a coating on an existing article or device.

The bioerodible polymer - bioactive agent conjugates provide an effective and efficient means for delivering bioactive agents to a subject.

Disclosed is a method of delivering a bioactive agent to a subject, the method comprising administering to the subject a bioerodible polymer - bioactive agent conjugate described.

Through the bioactive agent release function of the bioerodible polymer - bioactive agent conjugates, the conjugates can also advantageously function as or form part of a sustained bioactive agent delivery system.

It is also disclosed a sustained bioactive agent delivery system, the system comprising a bioerodible polymer - bioactive agent conjugate described.

By "sustained bioactive agent delivery" is meant that the bioactive agent is released from the bioerodible polymer - bioactive agent conjugate over a period of time, for example over a period of 10 or more minutes, 30 or more minutes, 60 or more minutes, 2 or more hours, 4 or more hours, 12 or more hours, 24 or more hours, 2 or more days, 5 or more days, 10 or more days, 30 or more days, 2 or more months, 4 or more months or over 6 or more months.

In another aspect, the invention provides a monomer - bioactive agent conjugate that is suitable for use in preparing a bioerodible polymer - bioactive agent conjugate, the monomer - bioactive agent conjugate having a structure of general formula (II):

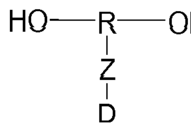

(II)

wherein:

R represents a linear or branched optionally substituted hydrocarbon;
Z is a linking group; and
D is a releasable bioactive agent selected from fluoroquinolone antibiotics.

The monomer - bioactive agent conjugate has been found to be particularly versatile and can advantageously be polymerised with one or more other monomers using techniques well known in the art.

The skilled worker will be familiar with techniques that may be used to promote reactions between functional groups of complementary reactivity, such as between a carboxylic acid and a hydroxy group. For example, coupling agents such as those mentioned in Tetrahedron Volume 60, Issue 11, 8 March 2004, pages 2447-2467, could be employed (see further detail below).

In another aspect, the invention provides a process for preparing a bioerodible polymer - bioactive agent conjugate as defined above

said process comprising the step of polymerising a monomer - bioactive agent conjugate of formula (II):

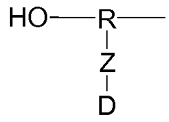

(II)

wherein:

R, Z and D are as defined above;
with a polyisocyanate and a polyester polyol.

Monomers that are polymerised with the monomer - bioactive agent conjugate of formula (II) to form the bioerodible polymer - bioactive agent conjugates of the invention will not only comprise compatible chemical functionality to react with the monomer - bioactive agent conjugate but that reaction will of course afford or give rise to a bioerodible moiety.
Through the polymerisation of a monomer - bioactive agent conjugate of formula (II), the process of the invention may advantageously be used to synthesise a bioerodible polymer - bioactive agent conjugate with a high loading of one or more bioactive agents.
Further aspects of the invention appear below in the detailed description of the invention.

## DETAILED DESCRIPTION OF THE INVENTION

The bioerodible polymer - bioactive agent conjugates in accordance with the invention can advantageously be prepared such that they are suitable for administration to a subject (i.e. suitable for *in vivo* applications).
By the conjugates being "suitable" for administration to a subject is meant that administration of the conjugate to a subject will not result in unacceptable toxicity, including allergenic responses and disease states.
By the term "subject" is meant either an animal or human subject. By "animal" is meant primates, livestock animals (including cows, horses, sheep, pigs and goats), companion animals (including dogs, cats, rabbits and guinea pigs), and captive wild animals (including those commonly found in a zoo environment). Laboratory animals such as rabbits, mice, rats, guinea pigs and hamsters are also contemplated as they may provide a convenient test system. Generally, the subject will be a human subject.
By "administration" of the conjugate to a subject is meant that the composition is transferred to the subject such that the bioactive agent will be released. Provided the bioactive agent can be released, there is no particular limitation on the mode of administration, but this will generally be by way of oral, parenteral (including subcutaneous, intradermal, intramuscular, intravenous, intrathecal, and intraspinal), inhalation (including nebulisation), buccal, pulmonary, aural, ocular, nasal, topical, rectal and vaginal modes.
The conjugates may be provided in particulate form and blended with a pharmacologically acceptable carrier to facilitate administration. By "pharmacologically acceptable" is meant that the carrier is suitable for administration to a subject in its own right. In other words, administration of the carrier to a subject will not result in unacceptable toxicity, including allergenic responses and disease states. The term "carrier" refers to the vehicle with which the conjugate is contained prior to being administered.
As a guide only, a person skilled in the art may consider "pharmacologically acceptable" as an entity approved by a regulatory agency of a federal or state government or listed in the US Pharmacopeia or other generally recognised pharmacopeia for use in animals, and more particularly humans.
Suitable pharmacologically acceptable carriers are described in Martin, Remington's Pharmaceutical Sciences, 18th Ed., Mack Publishing Co., Easton, PA, (1990), and include, but are not limited to, liquids that may be sterilised such as water and oils, including those of petroleum, animal, vegetable or synthetic origin, such as peanut oil, soya bean oil, mineral oil, sesame oil, and the like.
The conjugate may also form part of or be formed into an article or device, or be applied as a coating on an article or device, and implanted in a subject. By being "implanted" is meant that the article or device is totally or partly introduced medically into a subject's body, or by medical intervention into a natural orifice of a subject, and which is intended to remain there after the procedure.
Suitable dosage amounts of the bioactive agent and dosing regimens of the conjugate can be determined by a physician and may depend on the particular condition being treated, the rate of release of the agent form the polymer backbone, the severity of the condition as well the general age, health and weight of the subject.
Dosing may occur at intervals of minutes, hours, days, weeks, months or years or continuously over any one of these periods. Suitable dosages of the bioactive agent *per se* (i.e. that which is to be release from the polymer backbone within a given time frame) may lie within the range of about 0.1 ng per kg of body weight to 1 g per kg of body weight per dosage. The dosage may be in the range of 1 μg to 1 g per kg of body weight per dosage, such as is in the range of 1 mg to 1 g per kg of body weight per dosage. In one embodiment, the dosage may be in the range of 1 mg to

500 mg per kg of body weight per dosage. In another embodiment, the dosage may be in the range of 1 mg to 250 mg per kg of body weight per dosage. In yet another embodiment, the dosage may be in the range of 1 mg to 100 mg per kg of body weight per dosage, such as up to 50 mg per body weight per dosage.

The bioerodible polymer - bioactive agent conjugates in accordance with the invention may be administered in a single dose or a series of doses.

Dosage forms adapted for administration by the above modes may also be formulated with the bioerodable polymer - bioactive agent conjugate as one of the components of the dose formulation. In the case of ocular, aural or nasal administration the bioerodable polymer - bioactive agent conjugate may be a component of a drop, cream or ointment or take the form of an implant, ointment or gel. In the case of oral administration the bioerodable polymer - bioactive agent conjugate could take the form of or be a component in a tablet, capsule or liquid. In the case of topical administration the bioerodable polymer - bioactive agent conjugate could take the form of or be a component in a cream, ointment, gel or liquid (e.g eye drop). Parenteral administration would involve the bioerodable polymer - bioactive agent conjugate to be part of or take the form of an injectable product or implantable device (e.g coating on a pacemaker) that can be administered subcutaneously, intramuscularly, intravenously or by direct surgical placement within the body.

The form of the bioerodable polymer - bioactive agent conjugate may be adjusted to be suited to the required application such as a coating, film, pellet, capsule, fibres, laminate, foam etc. The difference in the form of the bioerodable polymer - bioactive agent conjugate provides a means to alter the release profile of the bioactive agent. For example the amount of polymer and bioactive agent may be the same in two different structures e.g. (a) polymer film, and (b) woven multifilament mat. The differences in the surface area to volume, rates of hydration and diffusion paths from the different physical structures can result in different rates of bioactive release from essentially the same polymer.

The adjustment of the form of the polymer to suit the application and further to adjust the form to further control the bioactive agent release profile provide an additional advantage over purely compositional and polymer structural means to control the release profile of the bioactive agent.

Some of the compositional / structural means to control the release of the bioactive agent include: controlling the loading of the bioactive; composition of the other comonomers to adjust criteria such as hydrophobicity, flexibility, susceptibility to degradation, ability of the fragments to autocatalyse the polymer degradation, thermal stability of the polymer, mouldability, polymer solubility to assist casting etc.

Additionally, the ability to produce the polymer - bioactive agent conjugates into a range of forms provide a means to produce three dimensional structures that can be beneficial is providing structural integrity to their application as well as providing a means to control cell growth by control of the placement of the conjugate in structures to allow release of the bioactive agent at specific places (e.g. fibre coating or fibres in a stent to prevent or control restinosis) Other examples include the ability of the structures to be formed into porous three dimensional structures to control cell phenotype as well as the filter or limit different types of cell bodily fluid ingress.

The conjugates in accordance with the invention may also be used in *in vitro* applications such as assays. Such in vitro applications could involve the use of the bioerodable polymer conjugates as a coating of a reaction chamber that would have an affect on chamber constituents (e.g. encourage cell growth, discourage growth of contaminating organisms, etc) or as a component within the assay to provide a source of the bioactive agent (e.g. as an antigen for an ELIZA test).

The use of the polymer - bioactive agent conjugates for *in vitro* applications could allow the use of the formed polymer to provide a host for bio-signals, essential additives drugs etc for the interaction controlled growth, cell segregation, controlled cell differentiation etc of cells / tissue components etc.

The formed conjugate could be made into a structure that is compatible with cell culture ware or *in vitro* devices used to act as indicators etc of certain conditions. The forms of the conjugate could range from simple pellets or films through to multilayer films, fibrous knitted, woven or electrospun structures, printed, spun cast, deposited or cast structures through to foams or composite type structures having multiple forms or components.

As used herein the term "conjugate" refers to the product formed through covalent bonding between the monomer or polymer and the bioactive agent as depicted in formulae (I) and (II).

The expression "bioactive agent" (represented as "D" in formulae (I) and (II)) is used to define any substance that is of medical or veterinary therapeutic, prophylactic or diagnostic utility capable of forming a conjugate in accordance with the invention. For example a bioactive agent may be a drug or therapeutically active agent, including pharmacologically active agents (eg receptor binding agonist or antagonists, cytotoxic agents), pharmacologically inactive agents (eg antibiotics) and prodrugs thereof. The bioactive agent will generally be a substance (e.g. pharmaceutical substance) for therapeutic use whose application (or one or more applications) involves: a chemical interaction, or physico-chemical interaction, with a subject's physiological system; or an action on an infectious agent, or on a toxin or other poison, in subject's body; or with biological material such as cells *in vitro.*

As used herein, a "therapeutic agent" refers to a bioactive agent that, when administered to a subject, will cure, or at least relieve to some extent, one or more symptoms of, a disease or disorder.

As used herein, a "prophylactic agent" refers to a bioactive agent that, when administered to a subject either

prevents the occurrence of a disease or disorder or, if administered subsequent to a therapeutic agent, prevents or retards the recurrence of the disease or disorder.

By the bioactive agent being "releasable" is meant that the agent is capable of being released or cleaved from the Z group defined in general formulae I and II above. Upon being released, the agent is bioactive or will be converted *in vivo* or *in vitro* to a bioactive agent (e.g. as in the case of a prodrug). Despite the bioactive agent being releasable from the monomer of formula II, it will be appreciated that the intention of the present invention is for the agent to be released after the monomer has been polymerised to form polymer.

In one embodiement, the bioactive agents are relased such that they do not comprise a residue derived from the polymer backbone or linker goup (Z). By this it is meant that the bioactive agents are released in their substantially original form (i.e. before being conjugated) and are essentially free from, for example, fragments of oligomer or polymer derived from the polymer backbone.

The bioactive agent may be released from the polymer - bioactive agent conjugate such that it provides for a sustained bioactive delivery system. Such a delivery system may in its simplest form be the conjugate provided in a desired shape, for example a pellet or more intricate shape. To promote surface area contact of the conjugate with a biological environment, the conjugate may also be provided in the form of a foamed product or a coating on substrate.

In order for the bioactive agent (denoted by D) to be released, the covalent bond between D and the Z group will of course need to be cleaved. In one embodiment the covalent bond between D and the Z group is not a carbon-carbon bond. In such an embodiment, the covalent bond between D and the Z group is part of a functional group selected from: esters; amides; anhydrides; imides; carbonates; peroxides; peroxyesters; phosphates; thioesters; sulfates; disulfides; carbamates; azo; and boronate esters. Of these functional groups, anhydrides, esters, imides, carbonates, carbamates, and boronate esters are preferred.

Cleavage of the covalent bond between the D and Z group can be promoted hydrolytically (i.e. hydrolytic cleavage) and may take place in the presence of water and an acid or a base. In some embodiments the cleavage may take place in the presence of one or more hydrolytic enzymes or other endogenous biological compounds that catalyze or at least assist in the cleavage process. For example, an ester bond may be hydrolytically cleaved to produce a carboxylic acid and an alcohol, and an amide bond may be hydrolytically cleaved to produce a carboxylic acid and an amine. Those skilled in the art will appreciate that such cleavage amounts to the hydrolytic cleavage of the bioerodible moieties discussed herein. Accordingly, the bioactive agent (D) may also be described as (a) being coupled to the linking group (Z) via a bioerodible moiety, or (b) forming together with the linking group (Z) a bioerodible moiety.

At the very least the bioactive agent will be releasable from the Z group of the polymer conjugate *per se.* However, the polymer may also bioerode *in vivo* or *in vitro* such that the polymer backbone fragments, with the agent remaining tethered to such a fragment(s) via the Z group or even just to a lone Z group as the fragment. In that case, the agent will nevertheless still be capable of being released or cleaved from the Z group, which may or may not still be associated with the polymer conjugate *per se.*

In one embodiment, the bioactive agent is releasable where the polymer backbone undergoes substantially no bioeroison during the release time frame.

As indicated above, on being released the bioactive agent may be in the form of a prodrug. As used herein, the term "prodrug" refers to a derivative of a bioactive agent, wherein the derivative may have little or none of the activity of the bioactive agent *per se* yet is capable of being converted *in vivo* or *in vitro* into a bioactive agent. An example of such derivatisation is the acetylation of one or more hydroxyl groups on a bioactive agent, such that subsequent to being released *in vivo* the released prodrug is deactylated to produce the bioactive agent.

Techniques that can be used to quantify a bioactive agent release profile of a given bioerodible polymer - bioactive agent conjugate are well known to those skilled in the art. For example, the release of an agent from a given conjugate may be quantified using analytical techniques such as HPLC and GC or by using activity tests.

In the moieties of formulae (I) and (II), the bioactive agent (D) is coupled to R through a linking group denoted by Z. As used herein "linking group" refers to a substituent which is generally divalent and that couples D to R. As outlined above, the covalent bond between the linking group (Z) and the bioactive agent (D) is cleavable so that the bioactive agent is releasable.

Examples of suitable linking groups include the divalent form of a group selected from oxy (-O-), alkyl, alkenyl, alkynyl, aryl, acyl (including -C(O)-), carbocyclyl, heterocyclyl, heteroaryl, alkyloxy, alkenyloxy, alkynyloxy, aryloxy, acyloxy, carbocyclyloxy, heterocyclyloxy, heteroaryloxy, alkylthio, alkenylthio, alkynylthio, arylthio, acylthio, carbocyclylthio, heterocyclylthio, heteroarylthio, alkylalkenyl, alkylalkynyl, alkylaryl, alkylacyl, alkylcarbocyclyl, alkylheterocyclyl, alkylheteroaryl, alkyloxyalkyl, alkenyloxyalkyl, alkynyloxyalkyl, aryloxyalkyl, alkylacyloxy, alkyloxyacylalkyl, alkylcarbocyclyloxy, alkylheterocyclyloxy, alkylheteroaryloxy, alkylthioalkyl, alkenylthioalkyl, alkynylthioalkyl, arylthioalkyl, alkylacylthio, alkylcarbocyclylthio, alkylheterocyclylthio, alkylheteroarylthio, alkylalkenylalkyl, alkylalkynylalkyl, alkylarylalkyl, alkylacylalkyl, arylalkylaryl, arylalkenylaryl, arylalkynylaryl, arylacylaryl, arylacyl, arylcarbocyclyl, arylheterocyclyl, arylheteroaryl, alkenyloxyaryl, alkynyloxyaryl, aryloxyaryl, arylacyloxy, arylcarbocyclyloxy, arylheterocyclyloxy, arylheteroaryloxy, alkylthioaryl, alkenylthioaryl, alkynylthioaryl, arylthioaryl, arylacylthio, arylcarbocyclylthio, arylheterocyclylthio, and arylheteroarylthio, wherein where present the or each $-CH_2-$ group in any alkyl chain may be replaced by a divalent group independently selected from -O-, $-OP(O)_2-$, $-OP(O)_2O-$ -S-, -S(O)-, $-S(O)_2O-$, $-OS(O)_2O-$, -N=N-, $-OSi(OR^a)_2O-$, $-Si(OR^a)_2O-$, $-OB(OR^a)O-$, $-B(OR^a)O-$, $-NR^a-$, -C(O)-, -C(O)O-,-OC(O)O-, $-OC(O)NR^a-$ and $-C(O)NR^a-$, where the or each $R^a$ may be independently selected from hydrogen, alkyl, alkenyl, alkynyl, aryl, carbocyclyl, heteroaryl, heterocyclyl, arylalkyl, and acyl. The or each $R^a$ may also be independently selected from hydrogen, $C_{1-18}$alkyl, $C_{1-18}$alkenyl, $C_{1-18}$alkynyl, $C_{6-18}$aryl, $C_{3-18}$carbocyclyl, $C_{3-18}$heteroaryl, $C_{3-18}$heterocyclyl, and $C_{7-18}$arylalkyl.

The linking group (Z) may also comprise one or more terminal polyvalent groups such as -N= (so as to provide an imino group or azo group for example) or -CH= (so as to provide an imino group for example).

The linking group may be branched. In some embodiments where the linking group is branched, two or more releasable bioactive agents may be appended to the linking group.

In the lists above defining groups (generally divalent) from which the or each linking group may be selected, each alkyl, alkenyl, alkynyl, aryl, carbocyclyl, heteroaryl, and heterocyclyl moiety may be optionally substituted. For avoidance of any doubt, where a given linking group contains two or more of such moieties (e.g. alkylaryl), each of such moieties may be optionally substituted with one, two, three or more optional substituents as herein defined.

In the lists above defining groups (generally divalent) from which the or each linking group may be selected, where a given linking group contains two or more subgroups (e.g. [group A] [group B]), the order of the subgroups is not intended to be limited to the order in which they are presented. Thus, a linking group with two subgroups defined as [group A][group B] (e.g. alkylaryl) is intended to also be a reference to a linking group with two subgroups defined as [group B][group A] (e.g. arylalkyl).

The present invention advantageously provides the skilled worker with an ability to construct monomer - bioactive agent conjugates of formula (II) using readily available reagents. Examples of such reagents that may be used to construct the linking group include linear or branched hydrocarbons substituted with two or more reactive functional groups such as alcohols, primary and secondary amines, carboxylic acids and combinations thereof. Examples of such substituted hydrocarbons are diols, diacids, diamines, hydroxyacids, amino acids and amino alcohols. The substituted hydrocarbons may be $\alpha,\omega$-substituted alkylenes or $\alpha,\omega$-substituted (poly)oxycarbonylalkylenes [ie $\alpha,\omega$-substituted polyesters]. The skilled person will appreciate that when such $\alpha,\omega$-substituted alkylenes or $\alpha,\omega$-substituted (poly)oxycarbonylalkylenes are used as the linking group, the monomer - bioactive agent conjugates of formula (II), and polymers produced therefrom, will typically comprise at least two functional groups that may be susceptible to cleavage so as to release the bioactive agent D from the hydrocarbon R. In these compounds, it is preferable that the functional group closest to the bioactive agent D is as susceptible to, or more susceptible to, cleavage than the functional group closest to the hydrocarbon R as defined herein.

Specific examples of linking groups include: -O-; -C(O)-; and optionally substituted: -OC(O)-$C_{1-18}$alkylene-C(O)-; -C(O)O-$C_{1-18}$alkylene-C(O)-; $-NR^aC(O)-$$C_{1-18}$alkylene-C(O)-; -C(O)O-$C_{1-18}$alkylene-O-; -O-$C_{1-18}$alkylene-O-; -O-$C_{1-18}$alkylene-$NR^a$-; -OC(O)-$C_{1-18}$alkylene-$NR^a$-; -C(O)-$C_{1-18}$alkylene-$NR^a$-; -OC(O)-$C_{1-18}$alkylene-O-; -C(O)-$C_{1-18}$alkylene-O-; and -C(O)$NR^a$-$C_{1-18}$alkylene-$NR^a$- where $R^a$ is as defined above.

Preferred examples of linking groups include: -O-; -C(O)-; and -OC(O)-$C_{1-18}$alkylene-C(O)-, such as -OC(O)-$C_{2-3}$alkylene-C(O)-.

An example of a monomer - bioactive agent conjugate of formula (II) that comprises a -OC(O)-$C_{1-18}$alkylene-C(O)- linking group (Z) is shown below:

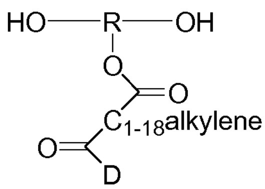

wherein:

R represents a linear or branched optionally substituted hydrocarbon; and
D is a releasable bioactive agent selected from fluoroquinolone antibiotics.

Use of the linking group can provide facile coupling of the bioactive agent to the R group. In particular, the linking group may provide the skilled worker with the ability to couple the bioactive agent at a sterically hindered position that could not otherwise be achieved by directly coupling the agent to the R group.

The choice of linking group will determine the spacing of the D from the OH groups in the monomers of formula (II). In this respect, the use of a linking group can provide a means to distance D from the OH groups. This can facilitate polymerisation of the monomers by reducing steric crowding around the OH groups.

In forming the monomer of formula (II), prior to conjugation the bioactive agent (denoted by D) necessarily comprises compatible functionality so as to promote coupling of the bioactive agent to the monomer through Z.

Examples of such compatible functionalities in D can include:

(i) carboxylic acids, sulfates and phosphates (e.g. for reacting with a linking precursor group (Z' or Z") comprising a primary amino, secondary amino or hydroxy group to couple the bioactive agent to the monomer through a nitrogen atom or an oxygen atom containing linking group (Z)); and
(ii) carboxylic acids, hydroxyls, amines (primary and secondary) thiols and phosphates (e.g. for reacting with a linking precursor group (Z' or Z") comprising a carboxylic acid or acid halide group to couple the bioactive agent to monomer through a carbonyl group containing linking group (Z)).

Those skilled in the art will appreciate that the process of conjugation of D to Z' or Z", or the process of conjugation of D-Z" to Z', will typically result in expulsion of a small molecule such as water or hydrogen halide (e.g. HCl). For example, the formation of an ester bond through reaction of a carboxylic acid group with a hydroxy group liberates a water molecule.

In some embodiments, the bioactive agent comprises a carboxylic acid, hydroxyl group, thiol group, amine group, or phosphate group, or combinations thereof for conjugate coupling. When conjugated through such groups, a part or the whole of the Z group can form part of an ester, an amide, an anhydride, an azo, an imide, a carbonate, a peroxyester, a thioester, a carbamate, a boronate ester, a sulfate or a phosphate linkage group. The skilled worker will recognise that each of these linkage groups comprises a covalent bond that is capable of being cleaved (for example hydrolytically, enzymatically and/or by a radical mechanism). Generally, such linkage groups will comprise a covalent bond that is capable of being cleaved hydrolytically so as to release the bioactive agent.

Where a given bioactive agent comprises more than one compatible functional group capable of conjugate coupling, the skilled worker may routinely adopt an appropriate protecting group strategy so that the bioactive agent couples through Z in a preselected fashion. Protection group chemistry, and strategy, is well-known in the art in, for example, T.W. Greene and P.G.M. Wuts in "Protective Groups in Organic Chemistry", John Wiley and Sons, 1991. For example, where a bioactive moiety comprises a primary alcohol and a primary amine, the skilled worker may protect the primary amine prior to coupling the bioactive agent through Z (which may be, or may comprise, a carbonyl group derived from a linking precursor group Z' or Z" comprising a carboxylic acid) so that the bioactive moiety couples through an ester moiety rather than an amide moiety. In another example a primary alcohol may be protected selectively over a secondary alcohol, so that the bioactive moiety may be coupled through an ester moiety (where Z is, or comprises, a carbonyl group derived from a linking precursor group Z' or Z" comprising a carboxylic acid) derived from the secondary alcohol. Such protecting groups may or may not be removed following coupling. For example, the protecting group may be an acetyl group, and the acetylated amine or alcohol may undergo cleavage *in vivo* to return the unprotected amine or alcohol.

Provided that a suitable conjugate can be formed, there is no particular limitation on the type of bioactive agent that may be used in accordance with the invention. The bioactive agent may be exemplified by 5-alpha-reductase inhibitors, amebicides, aminosalicylates, anaesthetics (general and local), analgesics, angiotensin inhibitors, anorexiants, antacid agents, anti-angiogenic agents, antianginal agents, antiarrythmic agents, antiarthritic agents, antibiotics, antibacterial agents, antibodies, anticoagulants, anticonvulsants, antidepressants, antiepileptic agents, antifungals, anthelmintics, antihistamines, antihypertensives, antihyperlipidemic agents, antiinfectives, antiinflammatories, antiemetics, antimalarial, antimetabolites, antimigraine, antimitotics, antiparasitic agents, antiparkinson agents, antipsychotics, antiprotozoals, antitussives, antiulcer agents, antivirals, anxiolytics, bronchodilators, decongestants and expectorants, cancer therapy and related pharmaceuticals, cardiovascular pharmaceuticals, central nervous system pharmaceuticals, benzopidazepines, beta-adrenergic blocking agents, bisphosphonates, calcium channel blockers, carbonic anhydrase inhibitors, chemokine receptor antagonist, coumarins and indadiones, cox-2 inhibitors, contraceptives, cytotoxics, diuretics, diabetes therapies, growth hormones, fertility pharmaceuticals, hematinics, glucose modifying agents, growth promoters, H2 antagonists, heparin and heparin antagonists, hormone replacement therapies, hemostatics, immunosuppressants, immunostimulants, inotropic agents, interferons, hormones and analogs, impotence agents, kinase inhibitors, laxatives, leukotriene modifiers, macrolides, mast cell stabilizers, muscle relaxants/stimulants, mydiratics, neuromuscular blocking agents, obesity therapeutics, ophthalmic pharmaceuticals, osteoporosis drugs, pain therapeutics (including paracetamol, opiates, nonsteroidal antinflammatory agents, tramadol), peptides and polypeptides, peripheral vasodilators, platelet inhibitors/stimulating agents, prolactin inhibitors, protease inhibitors, protein therapeutics, proton pump inhibtors, radiopharmaceuticals, respiratory pharmaceuticals, sedatives, spermicides, steroids (including androgens, anabolic and adrenal cortical), smoking cessation agents, statins, stimulants and tranquilizers, sulphonamides, thyroid drugs, urinary acidifiers/alkalinisers, and vasodilators.

Specific examples of bioactive agents that can be used in the present invention are listed below in categories according to their functional groups that may be used in conjugate formation. This list is in no way limiting the scope of drugs covered in this invention, but given as representative examples. All the amino- (including amide-NH and sulfonamide-NH, carbamate-NH, sulfamate-NH, hydrazone-NH, semicarbazone-NH, thiosemicarbazone-NH, urea-NH, phosphoramide-NH and the like), carboxyl- and hydroxyl-(including oxime-OH), containing drugs under various therapeutic categories as listed in Merck Index (13.sup.th editions) and other data bases such as prous science's ensemble, integrity, and the like and also all the qualified (i.e., amino-, and /or hydroxyl-, and/or carboxyl-containing) investigational drugs as listed in databases such Merck Index (14th. edition), iddb, ensemble, integrity, and the like, are covered under this invention without any limitation.

ANTI-INFLAMMATORY DRUGS:

Amino-containing: Ampiroxicam, Bucolome, Celecoxib, Difenpiramide, Mofebutazone, Nimesulide, Paranyline, Parecoxib, Parsalmide, Piketoprofen, Talniflumate, Tenidap, Terofenamate, and Valdecoxib.

Hydroxyl-containing: 21-Acetoxypregnenolone, Alclometasone, Betamethasone, alfa-Bisabolol, Budesonide, Clobetasone, Cyclosporin, Deflazacort, Dexamethasone, Diflorasone, Desonide, Desoximetasone, Diflorasone, Diflucortolone, Difluprednate, Ditazol, Everolimus, Fluazacort, Fludrocortisone, Flumethasone, Fluocinolone, Fluocinonide, Fluocortin Butyl, Fluocortolone, Fluprednidene Acetate, Glucametacin, Halcinonide, Halobetasol Propionate, Halometasone, Halopredone Acetate, Hydrocortisone, Ibuproxam, Loteprednol Etabonate, Mazipredone, Memetasone, Methylprednisolone, Mometasone Furoate, Oxyphenbutazone, Perisoxal, Pimecrolimus, Prednisolone, Prednisone, Rimexolone, Sirolimus, Triamcinolone and Tacrolimus.

Hydroxyl-, and Amino-containing: Bufexamac, Etofenamate, Fepradinol, Ibuproxam, Isoxicam, Lornoxicam, Meloxicam, Oxametacine, Piroxicam, and Tenoxicam.

Carboxyl- and Amino-containing: Aceclofenac, Alminoprofen, Amfenac, 3-Amino-4-hydroxybutyric Acid, Carprofen, Diclofenac, Enfenamic Acid, Etodolac, Flufenamic Acid, Meclofenamic Acid, Mefenamic Acid, Niflumic Acid, and Tolfenamic Acid.

Carboxyl-containing: Acemetacin, Acetamidocaproic Acid, Bendazac, Benoxaprofen, Bermoprofen, Bucloxic Acid , Butibufen, Cinmetacin, Clidanac, Clopirac, Felbinac, Fenbufen, Fenclozic Acid, Fenoprofen, Fentiazac, Flunoxaprofen, Flurbiprofen, Ibuprofen, Indomethacin, Isofezolac, Isoxepac, Ketoprofen, Lonazolac, Loxoprofen, Metiazinic Acid, Mofezolac, Naproxen, Oxaprozin, Pirazolac, Pirprofen, Pranoprofen, Protizinic Acid, Sulindac, Suprofen, Suxibuzone, Tiaprofenic Acid, Tolmetin, and Tropesin. Bermoprofen, Bucloxic Acid, Isoxepac, Ketoprofen, Loxoprofen, and Zaltoprofen.

Carboxyl- and Hydroxyl-containing: Balsalazide, Enoxolone, Fendosal, Olsalazine, Oxaceprol, and Ximoprofen.

Amino-, Carboxyl- and Hydroxyl-containing: 3-Amino-4-hydroxybutyric Acid, Mesalamine, and Sulfasalazine.

ANALGESIC AND/OR ANTIPYRETIC DRUGS:

Amino-containing: Aminochlorthenoxazin, Aminopropylon, Anileridine, Antrafenine, Benorylate, Benzpiperylon, p-Bromoacetanilide, Butacetin, Carsalam, Difenamizole, Etersalate, Ethenzamide, Ethoxazene, Flipirtine, Isonixin, Nifenazone, Phenacetin, Phenazopyridine, Phenocoll, Phenopyrazone, Piminodine, Piritramide, Propacetamol, Ramifenazone, Piperylone, Salverine, and Tinoridine.

Hydroxyl-containing: Aluminum bis(acetylsalicylate), Benzylmorphine, Buprenorphine, Butorphanol, Chlorobutanol, Ciramadol, Codeine, Desomorphine, Dihydrocodeine, Dihydromorphine, Dihydroxyaluminum acetylsalicylate, Dimepheptanol, Eptazocine, Ethylmorphine, Eugenol, Hydromorphone, Hydroxypethidine, Levorphanol, Meptazinol, Metazocine, Morphine, Nalbuphine, Oxycodone Pentazocine, Phenazocine, Phenoperidine, Phenylsalicylate, Salicin, Tramadol, and Viminol. Hydromorphone, Ketobemidone, Metopon, Oxycodone, and Oxymorphone.

Carboxyl-containing: Acetylsalicylsalicylic acid, Alclofenac, Aspirin, Benoxaprofen, 5-Bromosalicylic acid acetate, Cinchophen, Diacerein, Dipyrocetyl, Fosfosal, Ibufenac, Indoprofen, and Salicysulfuric acid. Clometacin, Ketorolac, and Zomepirac.

Amino- and Hydroxyl-containing: Acetaminophen, Acetaminosalol, Bucetin, Capsaicine, Dezocine, Floctafenine, Glafenine, Isoladol, p-Lactophenetide, Norlevorphanol, Normorphine, Phenylramidol, Salacetamide, and Salicylamide.

Amino- and Carboxyl-containing: Actarit, Bumadizone, Clonixin, and Salicylamide O-acetic acid.

Carboxyl- and Hydroxyl-containing: Diflunisal, Gentisic acid, and Salsalate.

ANTIHYPERTENSIVE DRUGS:

Amino-containing: Alfuzosin, Benzylhydrochlorothiazide, Bethanidine, Bopindolol, Budralazine, Bunazosin, Ciclosidomine, Clonidine, Clopamide, Cyclopenthiazide, Debrisoquin, Edeserpidine, Diazoxide, Dihydralazine, Doxazosin, Endralazine, Guanabenz, Guanacline, Guanazodine, Guanethidine, Guanochlor, Guanadrel, Guanfacine, Guanoxan, Hydracarbazine, Hydralazine, Hydroflumethiazide, Indapamide, Indoramin, Irbesartan, Ketanserin, Lofexidine,

Mebutamate, Mecamylamine, Methyl 4-pridyl ketone thiosemicarbazone, Mibefradil, Minoxidil, Monatepil, Moxonidine, Pheniprazine, Pinacidil, Prazosin, Raubasine, Rescinnamine, Reserpiline, Reserpine, Rilmenidine, Syrosingopine, Tasosartan, Terazosin, Tiamenidine, Todralazine, Tolonidine, Tripamide, and Urapidil.

Hydroxy-containing: Ajmaline, Cicletanine, Levcromakalim, Naftopidil, Phenactropinium chloride, and Protoveratrines.

Carboxyl-containing: Eprosartan, Fosinopril, and Telmisartan, Captopril, and Omapatrilat.

Amino- and Carboxyl-containing: Alacepril, gama-Aminobutyric acid, Benazepril, Candesartan, Carmoxirole, Caronapril, Cilazapril, Delapril, Enalapril, Enalaprilat, Imidapril, Lisinopril, Moexipril, Moveltipril, Perindopril, Quinapril, Ramipril, Saralasin, Spirapril, Temocapril, Trandolapril, and Valsartan.

Amino- and Hydroxyl-containing: Acebutolol, Alprenolol, Amosulalol, Arotinolol, Atenolol, Betaxolol, Bisoprolol, Bosentan, Bucindolol, Bufeniode, Bunitrolol, Bupranolol, Butofilolol, Cadralazine, Celiprolol, Carazolol, Carteolol, Cetamolol, Carvedilol, Epanolol, Indenolol, Nadolol, Dilevalol, Fenoldopam, Guanoxabenz, Labetalol, Losartan, Mepindolol, Metipranolol, Metoprolol, Moprolol, Nebivolol, Olmesartan, Oxprenolol, Penbutolol, Phentolamine, Pildralazine, Pindolol, Propranolol, Rescimetol, Sulfinalol, Talinolol, Tertatolol, Timolol, and Trimazosin.

Amino-, Hydroxyl- and Carboxyl-containing: Methyldopa, and Sampatrilat.

ANTIBIOTICS:

All the known amino-, hydroxyl-, and carboxyl-containing antibiotics such as Amoxicillin, Ampicillin, Olivanic acid, Metronidazole, and the like as listed in Merck Index. 13.sup.th edition and other drug databases integrity, ensemble, iddb, and the like. These antibiotics can be used in combination with beta-lactamase inhibitor such as clavulanic acid, penicillinic acid sulfone and the like. The following lists of antibacterial and antifungal agnets are given for clarity.

ANTIBACTERIAL AGENTS:

Amino-containing: Acedapsone, Acetosulfone sodium, Ambazone, Bacampicillin, Benzylsulfamide, Brodimoprim, Cefcapene pivoxil, Cefpodoxime proxetil, Chloramine-B, Chloramine-T, Capreomycin, Clofazimine, Cyacetacide, Cycloserine, Dapsone, Ethionamide, Furazolium chloride, N2-Formylsulfisomidine, Furonazide, Isoniazid, Lenampicillin, Linezolide, Mafenide, 4'-(Methylsulfamoyl)sulfanilanilide, Morphazinamide, Nifuradene, Nitrofurantoin, Penamecillin, Penethamate hydriodide, Pexiganan, Pivampicillin, Pivcefalexin, Picloxydine, Protionamide, Pyrazinamide, Solasulfone, Subathizone, 4,4'-Sulfinyldianiline, Sulfoxone sodium, 4'-Sulfanilylsulfanilamide, Sulfoniazide, Sulfabenzamide, Sulfacetamide, Sulfachlorpyridazine, Sulfacytine, Sulfadiazine, Sulfadicramide, Sulfadimethoxine, Sulfadoxine, Sulfaethidole, Sulfaguanidine, Sulfaguanole, Sulfalene, Sulfamerazine, Sulfameter, Sulfamethazine, Sulfamethizole, Sulfamethomidine, Sulfamethoxazole, Sulfamethoxypyridazine, Sulfamethylthiazole, Sulfametrole, Sulfamidochrysoidine, Sulfamoxole, Sulfanilamide, p-Sulfanilylbenzylamine, Sulfanilylurea, N-Sulfanilyl-3,4-xylamide, Sulfaperine, Sulfaphenazole, Sulfaproxyline, Sulfapyrazine, Sulfasomizole, Sulfasymazine, Sulfathiazole, Sulfathiourea, Sulfisomidine, Sulfisoxazole, Sultamicillin, Sulfatolamide, Talampicillin, Taurolidine, Tetroxoprim, Thiazosulfone, Thiacetazone, Tiocarlide, and Trimethoprim.

Hydroxyl-containing: Azithromycin, Chloroxylenol, Chlorquinadol, Clindamycin, Clofoctol, Cloxyquin, Diathymosulfone, Doxycycline, Glucosulfone sodium, Nifurpirinol, Nifurtoinol, Nitroxoline, Roxarsone, Roxithromycin, Xanthocillin, and Xibornol. Carbomycin, Clarithromycin, Erythromycin, all erythromycin ester derivatives, Oleandomycin, and Telithromycin.

Carboxyl-containing (including sulfate, phosphate and phosphonate-containing): Amdinocillin, Cinoxacin, Difloxacin, Fosfomycin, and Hydnocarpic acid. Fleroxacin, Flumequine, Miloxacin, Nalidixic acid, Ofloxacin, Oxolinic acid, Pefloxacin, Piromidic acid, Prulifloxacin, Rosoxacin, and Rufloxacin.

Amino- and Carboxyl-containing (including sulfate-, sulfonic acid-, phosphate and phosphonate-containing): Acediasulfone, Amphomycin, Ampicillin, Azidocillin, Azlocillin, Aztreonam, Bacitracin, Balofloxacin, Betamipron, Carbenicillin, Carindacillin, Carumonam, Cefaclor, Cefazedone, Cefazolin, Cefclidin, Cefditoren, Cefepime, Cefetamet, Cefixime, Cefmenoxime, Cefmetazole, Cefodizime, Ceforanide, Cefotaxime, Cefotetan, Cefotiam, Cefoxitin, Cefozopran, Cefpimizole, Cefpirome, Cefroxadine, Cefsulodin, Ceftazidime, Cefteram, Ceftezole, Ceftibuten, Ceftizoxime, Ceftriaxone, Cefuroxime, Cefuzonam, Cephacetrile sodium, Cephalexin, Cephaloglycin, Cephaloridine, Cephalosporin C, Cephalothin, Cephapirin sodium, Cephradine, Cilastatin, Ciproflaxacin, Clinafloxacin, Clometocillin, Cyclacillin, Dicloxacillin, Enoxacin, Epicillin, Fenbenicillin, Floxacillin, Hetacillin, Loracarbef, Metampicillin, Methicillin, Mezlocillin, Nafcillin, Noprysulfamide, Opiniazide, Oxacillin, Penicillin(s), Penimepicycline, Phenethicillin, Phthalylsulfacetamide, Phthalylsulfathiazole, Piperacillin, Propicillin, Quinacillin, Succinylsulfathiazole, Succisulfone, Sulbenicillin, Sulfachrysoidine, Sulfanilic acid, Temocillin, Ticarcillin, and Tigemonam. Garenoxacin, Gatifloxacin, Gemifloxacin, Grepafloxacin, Lomefloxacin, Moxifloxacin, Norfloxacin, Pazufloxacin, Pipemidic acid, Sitafloxacin, Sparfloxacin, Tosufloxacin, and Trovafloxacin.

Amino- and Hydroxyl-containing: Amikacin, p-Aminosalicylic acid hydrazide, Arbekacin, Azidamfenicol, Bambermycins, 5-Bromosalicylhydroxamic acid, Butirosin, Clindamycin, Clomocycline, Chloramphenicol, Cloxacillin, Colistin, Demeclocycline, Deoxydihydrostreptomycin, Dibekacin, Dihydrostreptomycin, Dirithromycin, Doxycycline, Enviomycin, Ethambutol, Forimicins, Gentamycin, Glyconiazide, N4-beta-D-Glucosylsulfanilamide, Gramicidin(s), Isepamicin, Kanamycin(s), Lincomycin, Meclocycline, Methacycline, Micronomicin, Neomycin, Netilmicin, Novobiocin, Paromomycin, Phenyl aminosalicylate, Pipacycline, Polymyxin, Primycin, Ramoplanin, Ribostamycin, Rifabutin, Rifalazil, Rifamide, Rifamycin SV, Rifampin, Rifapentine, Rifaximin, Ristocetin, Salinazid, Sancycline, Sisomicin, Streptolydigin, Streptomycin, Streptonicozid, 2-p-Sulfanilylanilinoethanol, Thiamphenicol, Thiostrepton, Tobramycin, Tuberactinomycin, Viomycin, and Virginiamycin. Chlortetracycline, Dalfopristin, Guamecycline, Mikamycin, Minocycline, Oxytetracycline, Pristinamycin, Quinupristin, Rolitetracycline, Spectinomycin, and Trospectomycin.

Hydroxyl- and Carboxyl-containing (including sulfate, phosphate and phosphonate-containing): Fropenem, Nadifloxacin, Biapenem, Fusidic acid, and Merbromin.

Amino-, Hydroxyl-, and Carboxyl-containing (including sulfate, phosphate and phosphonate-containing): p-Aminosalicylic acid, Apicycline, Amoxicillin, Apalcillin, Aspoxicillin, Benzoylpas, Cefadroxil, Cefamandole, Cefatrizine, Cefbuperazone, Cefdinir, Cefminox, Cefonicid, Cefoperazone, Cefoselis, Cefpiramide, Cefprozil, Ertapenem, Flomoxef, Imipenem, Lymecycline, Meropenem, Moxalactam, Negamycin, Panipenem, Ritipenem, Salazosulfadimidine, Sulfaloxic acid, 4-Sulfanilamidosalicylic acid, Teicoplanin, Tyrocidine, and Vancomycin.

ANTIFUNGAL AGENTS:

Amino-containing: Chlordantoin, Exalamide, Flucytosine, Loflucarban.

Hydroxy-containing: Chlorphenesin, Ciclopirox, Dermostatin, Filipin, Fluconazole, Fungichromin, Pecilocin, Posaconazole, Ravuconazole, Rubijervine, Siccanin, 2,4,6-Tribromo-m-cresol and Voriconazole.

Carboxyl-containing: Undecylenic acid (10-undecenoic acid), and Propionic acid.

Amino- and Carboxyl-containing: Azaserine.

Amino- and Hydroxyl-containing: Salicylanilide, Acrisorcin (9-Aminoacrindine compound with 4-Hexylresorcinol (1:1)), Anidulafungin, Bromosalicylchloranilide, Buclosamide, Caspofungin, Micafungin, and Tubercidin.

Amino-, Carboxyl- and Hydroxyl-containing: Natamycin, Amphotericin B, Lucensomycin, and Nystatin.

ANTIVIRAL DRUGS:

Hydroxy-containing: Edoxudine, Floxuridine, Idoxuridine, Kethoxal, Podophyllotoxin, Sorivudine, Stavudine, Trifluridine, and Zidovudine.

Amino-containing: Amantadine, Amidinomycin, Atevirdine, Capravirine, Delavirdine, Efavirenz, Famciclovir, Imiquimod, Lamivudine, Methisazone, Moroxydine, Nevirapine, Oseltamivir, Rimantadine, Stallimycin, mantadine, and Valacyclovir.

Amino- and Hydroxyl-containing: Abacavir, Acyclovir, Adefovir, Amprenavir, Atazanavir, Cidofovir, Didanosine, Dideoxyadenosine, Emtricitabine, Entecavir, Indinavir, Lamivudine, Lopinavir, 5-(methylamino)-2-deoxyuridine (MADU), Nelfinavir, Penciclovir, Resiquimod, Ribavirin, Ritonavir, Saquinavir, Tenofovir, Tipranavir, Valganciclovir, Vidarabine, and Zalcitabine.

Carboxyl- and Hydroxyl-containing: Foscarnet sodium, and Ganciclovir.

Amino-, Carboxyl- and Hydroxyl-containing: Zanamivir.

ANTIMALARIAL:

Amino-containing: Chlorguanide, Chloroquine, Chlorproguanil, Cycloguanil, Pamaquine, Plasmocid, Primaquine, Quinocide, and Tafenoquine.

Hydroxyl-containing: Artemisinin alcohol, Bebeerines, Cinchonidine, Cinchonine, Dihydroartemisinin, Halofantrine, Lumefantrine, Quinine and Yingzhaosu A.

Carboxyl-containing: Arteflene and Artesunate.

Amino-, and Hydroxyl-containing: Amodiaquin, Hydroxychloroquine, Mefloquine, and Pyronaridine.

ANTINEOPLASTIC DRUGS:

Hydroxy-containing: Aclacinomycins, Arzoxifene, Batimastat, Broxuridine, Calusterone, Capecitabine, CC-1065, Chromomycins, Diethylstilbestrol, Docetaxel, Doxifluridine, Droloxifene, Dromostanolone, Enocitabine, Epitiostanol, Estramustine, Etanidazole, Etoposide, Fenretinide, Flavopiridol, Formestane, Fosfestrol, Fulvestrant, Gemcitabine, Irinotecan, Melengestrol, Menogaril, Miltefosine, Mitobronitol, Mitolactol, Mopidamol, Nitracrine, Nogalamycin, Nordihydroguaiaretic Acid, Olivomycins, Paclitaxel and other known paclitaxel analogs, Plicamycin, Podophyllotoxin, Retinoic acid (including all trans-retinioc acid), Roquinimex, Rubitecan, Seocalcitol, Temoporfin, Teniposide, Tenuazonic Acid, Topotecan, Valrubicin, Vinblastine, Vincristine, and Zosuquidar.

Amino-containing (including Amide-NH and Sulphonamide-NH, Carbamate-NH, Sulfamate-NH, and Phosphomide-NH): 9-Aminocamptothecin, Aminolevulinic Acid, Amsacrine, Bisantrene, Cactinomycin, Carboquone, Carmofur, Carmustine, Cyclophosphamide, Dacarbazine, Dactinomycin, Demecolcine, Diaziquone, 6-Diazo-5-oxo-L-norleucine (DON), Edatrexate, Efaproxiral, Eflornithine, Eniluracil, Erlotinib, Fluorouracil, Gefitinib, Gemcitabine, Goserelin, Histamine, Ifosfamide, Imatinib, Improsulfan, Lanreotide, Leuprolide, Liarozole, Lobaplatin, Cisplatin, Carboplatin, Lomustine, Lonafarnib, Mannomustine, Melphalan, Methotrexate, Methyl Aminolevulinate, Miboplatin, Mitoguazone, Mitoxantrone, Nilutamide, Nimustine, Nolatrexed, Oxaliplatin, Pemetrexed, Phenamet, Piritrexim, Procarbazine, Raltitrexed, Tariquidar, Temozolomide, Thiamiprine, Thioguanine, Tipifarnib, Tirapazamine, 3-Aminopyridine-2-carboxaldehyde thiosemicarbazone (3-AP)/ 3-Aminopyridine-4-methyl-2-carboxaldehyde thiosemicarbazone (3-AMP/Triapine /OCX-191/OCX-0191), Trimetrexate, Uracil Mustard, Uredepa ([Bis(1-aziridinyl)phosphinyl]carbamic acid ethyl ester, ethyl carbamate and Meturedepa.

Both Hydroxy- & Amino- containing (including Amide-NH and Sulphonamide-NH, Carbamate-NH, Sulfamate-NH, and Phosphomide-NH): Ancitabine, Anthramycin, Azacitidine, Bleomycins, Bropirimine, Buserelin, Carubicin, Chlorozotocin, Cladribine, Cytarabine, Daunorubicin, Decitabine, Defosfamide, Docetaxel, Doxorubicin, Ecteinascidins, Epirubicin, Gemcitabine, Hydroxyurea, Idarubicin, Marimastat, 6-Mercaptopurine, Pentostatin, Peplomycin, Perfosfamide, Pirarubicin, Prinomastat, Puromycin, Ranimustine, Streptonigrin, Streptozocin, Tiazofurin, Troxacitabine, Vindesine and Zorubicin.

Carboxyl-containing: Butyric acid.

NTIGLAUCOMA AGENTS:

Amino- containing: Acetazolamide, Brimonidine and Pilocarpine.

Amino- and Hydroxyl-containing: Bimatoprost and Timolol.

Hydroxyl-containing: Latanoprost, Bimatoprost and Travoprost.

BENZODIAZEPINE TRANQUILIZERS AND HYPNOTICS:

Diazepam, Triazolam, Alprazolam, and the like.

ANTIULCER AGENTS:

Amino-containing (including Amide NH and Sulphonamide NH and Phosphomide NH, etc.): Aldioxa, Benexate HCl, Cimetidine, Ebrotidine, Ecabapide, Esaprazole, Esomeprazole, Famotidine, Irsogladine, Lafutidine, Lansoprazole, Omeprazole, Pantoprazole, Pirenzepine, Polaprezinc, Rabeprazole, Ranitidine, Roxatidine, and Troxipide.

Hydroxyl-containing: Enprostil, Misoprostol, Ornoprostil, Plaunotol, Rioprostil, Trimoprostil, and Oryzanol A.

Carboxyl-containing: Acetoxolone, Carbenoxolone, Rebamipide, and Sofalcone.

Amino (or Hydroxyl) - and Carboxyl-containing: Cetraxate, Ecabet, S-Methylmethionine, Rosaprostol, and Rotraxate.

ANTICONVULSANTS:

Amino-containing (including Amide NH and Sulphonamide NH and Phosphomide NH, etc.): Acetylpheneturide, Albutoin, N-benzyl-3-chloropropionamide, Carbamazepine, Cinromide, Clonazepam, Decimemide, Dimethadione, Doxenitoin, Ethosuximide, Ethotoin, Felbamate, Fosphenytoin, Lamotrigine, Levetiracetam, Mephenytoin, Mephobarbital, Metharbital, Methetoin, Nitrazepam, Oxcarbazepine, Oxicarbamazepine, Phenacemide, Phenetharbital, Pheneturide, Phenobarbital, Phenylmethylbarbituric Acid, Phenytoin, Phethenylate Sodium, Primidone, Progabide, Remacemide, Rufinamide, Suclofenide, Sulthiame, Talampanel, Tetrantoin, Topiramate, Valpromide, Zonisamide, 5-Methyl-5-(3-phenanthryl)hydantoin, and 3-Methyl-5-phenylhydantoin.

Hydroxyl-containing: Ganaxolone.

Hydroxyl-, and Amino-containing (including Amide NH and Sulphonamide NH and Phosphomide NH): 4-Amino-3-hydroxybutyric Acid, Atrolactamide, and Buramate.

Carboxyl- and Amino-Containing (including Amide NH and Sulphonamide NH and Phosphomide NH): Gabapentin, Pregabalin, and Vigabatrin.

Carboxyl-containing: Tiagabine, and Valproic Acid.

ANTIPARKINSON

Levodopa and Carbidopa.

ANTIDEPRESSANT:

Amino-containing (including Amide NH and Sulphonamide NH and Phosphomide NH, etc.): Amoxapine, Caroxazone, Demexiptiline, Desipramine, Duloxetine, Fluoxetine, Fluvoxamine, Indalpine, Indeloxazine Hydrochloride, Iproclozide, Iproniazid, Isocarboxazid, Levophacetoperane, Maprotiline, Metapramine, Milnacipran, Minaprine, Moclobemide, Nialamide, Nomifensine, Nortriptyline, Octamoxin, Oxypertine, Paroxetine, Protriptyline, Reboxetine, Rolipram, Sertraline, Tofenacin, Tranylcypromine, Viloxazine, Benmoxine, and Rolicyprine.
Hydroxyl-containing: Befloxatone, Bupropion, Fenpentadiol, Hypericin, Opipramol, Pyrisuccideanol, Toloxatone, and Venlafaxine.
Hydroxyl-, and Amino-containing (including Amide NH and Sulphonamide NH and Phosphomide NH): S-Adenosylmethionine, 5-Hydroxytryptophan, and Roxindole.
Carboxyl- and Amino-Containing (including Amide NH and Sulphonamide NH and Phosphomide NH): Amineptine, and Tianeptine.

ANTIHISTAMINIC

Amino-containing (including Amide NH and Sulphonamide NH and Phosphomide NH, etc.): Antazoline, Astemizole, Clobenzepam, Desloratadine, Epinastine, Metron S, Mizolastine, and Tritoqualine.
Hydroxyl-containing: Terfenadine, and N-Hydroxyethylpromethazine Chloride.
Hydroxyl-, and Amino-containing (including Amide NH and Sulphonamide NH and Phosphomide NH, etc.): Cetoxime.
Carboxyl-containing: Acrivastine, Bepotastine, Cetirizine, and Levocabastine.
Carboxyl- and Hydroxyl-containing: Fexofenadine.

ANTICANCER, ANTIOXIDATIVE, ANTIINFLAMMATORY, AND CARDIOPROTECTIVE AGENT:

Trans-Resveratrol [(E)-3,4',5-trihydroxystilbene).

ANTIDIABETIC:

Metformin, and Nateglinide/Glipizide/Glibenclamide (Glyburide).

LOCAL ANAESTHETICS

Amino-containing: Benzocaine, Chloroprocaine, Proparacaine, Tetracaine,Cocaine, Propoxycaine, Procaine, Proparacaine, Tetracaine, Articaine, Bupivacaine, Carticaine, Cinchocaine, Etidocaine, Levobupivacaine, Lignocaine, Mepivacaine, Piperocaine, Prilocaine, Ropivacaine, Trimecaine
It should be understood that while the lists of names of various categories of drugs have been included above, such lists are presented in a way of illustration of the structural features of the qualifying drugs in this invention and therefore, the number and types of listed drugs are not necessarily limited thereto. In principal, any amino-, and /or carboxyl, and/or carbonyl-, and/or hydroxyl-containing drug (from both known and investigational drugs), irrespective of its therapeutic category and their mechanism of action, as listed in drug databases such as Merck Index, prous science's ensemble, integrity, iddb, and the like, are generally covered within the scope of the present invention. For clarity, in addition to the above lists of drugs, any amino-, and/or carboxyl-, and/or carbonyl-, and/or hydroxyl-containing drug(s) (both known and investigational drugs) from the following therapeutic areas are covered without any limitation:

CENTRAL NERVOUS SYSTEM:

Sedatives, Hypnotics, Antidepressants, Antipsychotics and Antimanics, Analgesics & Antipyretics, Antimigraine agents, Anticonvulsants, Drugs used in parkinsonism and movement disorders, Drug for dementia, Antiemtics, drugs for Vertigo, CNS Stimulants & activators. Quetiapine, Paliperidone (active metabolite of risperidone), Fluphenazine

EYE:

Antiinfective eye preparations, Antiinflammatory and antiallergic preparations, antiglucoma drugs and other preparations to cure eye diseases.

EAR, NOSE and OROPHARYNX:

Drugs used aural, nasal and oropharyngeal preparation.

CARDIOVASCULAR SYSTEM:

Antiarrhythemic drugs, Antihypertensives (including alfa/beta-blockers, channel blockers, ACE inhibitors, Angiotensin II receptor antagonists, diuretics, etc.), Antianginals (includinig nitrates, calcium channel blockers, etc.), Drugs for cardiac failure and shock, Vasodilators, Coagulants, Anticoagulants, Thrombolytics and antiplatelet drugs.

RESPIRATORY SYSTEM:

Respiratory stimulants, Antitussives, Expectorants, Mucolytics and Decongestants, Antihistamine agents, and antiasthmatics.

GASTRO INTESTINAL TRACT:

Antiulcer and Antisecretory drugs (including H.sub.2 receptor antagonists, Proton Pump Iinhibitors, Prostaglandin analogues, etc.), Antacids, Antispasmodics and drugs modifying intestinal motility, Antidiarrhoeals (including antimotility and antimicrobial drugs) and drugs acting on gall bladder.

GENITO URINARY SYSTEM:

Urinary antiinfectives, Diuretics, Urinary analgesics & antispasmodics, Antiinfective drugs acting on urethra and vagina, drugs acting on uterus, Drugs for prostatic hypertrophy (including alfa blockers and antiandrogens), Drugs for erectile dysfunction, and Spermicidal & nonhormonal contraceptives.

SKIN:

Keratolytics, topical antiinfectives, topical antifungals, topical parasiticidals, topical steroids, topical drugs for acne vulgaris, drugs for psoriasis, pigmentation disorders, and Antiseborrhoeics.

MUSCULO-SKELETAL DISORDERS:

Non Steroidal Anti Inflammatory Drugs (NSAIDs) including COX-2 inhibitors, Antiarthritic agents, Immunosuppressants, Topical analgesics, Muscle relaxants and Neuromuscular Drugs.

INFECTIONS AND INFESTATIONS:

Penicillin antibiotics, Cephalosporin antibiotics, Quinolone & Fluoroquinolone antibiotics, Macrolide antibiotics, Chloramphenicol, Tetracycline antibiotics, Sulfonamides, Antianaerobics such as Metronidazole, Antitubercular drugs, Antileprosy drugs, Antifungals, Antiprotozoals, Anthelminthics & Antiinfestive Drugs, Antimalarials and Antivirals.

ENDOCRINE SYSTEM:

Anabolic and androgenic steroids, Corticosteroids, Oestrogens, Progestogens and Hormonal contraceptives, Fertility Agents, Trophic hormones and related drugs, Thyroid and antithyroid drugs, Antidiabetics and hyperglycaemics.

METABOLISM:

Hypolipidaemic drugs (including fibric acid derivatives, statins [(i.e., HMG CoA reductase inhibitors), nicotinic acid group, etc.], Drugs used for Gout and Drugs affecting bone metabolism (including bisphosphonates).

NEOPLASTIC DISORDERS:

Anticancer drugs such as alkylating agents, cytotoxic antibiotics, antimetabolites such as cytarbine, Fludarbine, 5-Fluorouracil, Mercaptopurine, Thioguanine, etc., Vinca alkaloids and Etoposide, Taxanes, Topoisomerase 1 inhibitors, Cytotoxic immunosuppressants, Immunostmulants, Cytoprotectives such as Amifostine, Oestrogens, Progestogens, hormon antagonists and other antineoplastic drugs.

ALLERGY AND IMMUNOLOGY:

Antiallurgics such as non-sedative antihistamins (e.g., Cetirizine, Desloratadine, Terfenadine, Fexofenadine, etc.), sedative histamines and histamine receptor blockers.

ANAESTHETICS & SURGICALS:

Local anaesthetics, intravenous anaesthetics, inhalation anaesthetics and muscle relaxants.

In addition to the above list of drugs, the present invention also covers newer drugs with the above mentioned active functional groups as listed in the Merck index (14.th edition) and other drug databases such as Prous Science's ensemble, integrity and the investigational drugs as listed in databases such as iddb, ensemble, integrity, and the like without any limitation.

Preferred bioactive agents include the flouroquinolone antibiotics, local anesthetics and valproic acid. Preferred flouroquinolone antibiotics include; alatrofloxacin, balofloxacin, ciprofloxacin, clinafloxacin, danofoxacin, delafloxacin, dextrofloxacin, difloxacin, enoxacin, enrofloxacin, garenoxacin, gatifloxacin, gemifloxacin, grepafloxacin, levofloxacin, lomefloxacin, marbofloxacin, moxifloxacin, nadifloxacin, norfloxacin, ofloxacin, orbifloxacin, pefloxacin, sitafloxacin, sparfloxacin, temafloxacin, tosufloxacin, tosulfloxacin and trovafloxacin. Still further preferred are: danafloxacin, detrofloxacin, difloxacin, enrofloxacin, marbofloxacin, levofloxacin, ofloxacin, pefloxacin. Still further preferred is levofloxacin and dextrofloxacin. Most preferred is levofloxacin and dextrofloxacin. The preferred local anaesthetics are benzocaine and procaine, most preferred is benzocaine.

Preferred bioactive agents that can be utilised in the present invention are those containing one or more functional groups such as carboxylate, hydroxyl, amino, thiol, phosphate or sulphate.

As discussed above, the polymer - bioactive agent conjugate will typically have multiple bioerodible moieties in its polymer backbone through which bioerosion can occur. Those skilled in the art will appreciate that the rate at which a particular bioerodible moiety in the polymer backbone undergoes hydrolytic cleavage under given environment relative to another can vary depending on the nature of each moiety (e.g. type of functionality, steric and electronic effects etc).

The same rationale can also apply to the rate at which the polymer backbone erodes relative to the rate of release of the bioactive agent.

In some embodiments it may be desirable that the rate of release of the bioactive agent from the polymer backbone is faster than the rate of bioerosion of the polymer backbone. By providing the polymer - bioactive agent conjugate with such properties, structural integrity of the polymer backbone (and hence the physical form (e.g. shape and size etc) in which the conjugate is provided) can be maintained while the drug is being released.

By the "rate of release of the bioactive agent from the polymer backbone" being "faster than the rate of bioerosion of the polymer backbone" is meant that the polymer - bioactive agent conjugate is constructed such that in a given biological environment the measurable concentration of the released agent in its biologically active form (D) is greater than the measurable concentration of the corresponding monomer-bioactive agent conjugate from which the polymer is derived (formula (II)). Such concentration measurement may be readily conducted by those skilled in the art, for example by using HPLC or GC analytical techniques.

In one embodiment, the measurable concentration of the released agent in its biologically active form (D) is at least 5%, at least 10%, at least 20%, at least 40%, at least 50 %, at 1 x, at least 2 x at least 5 x greater than the measurable concentration of the corresponding monomer-bioactive agent conjugate from which the polymer is derived (formula (II)).

In connection with providing the polymer - bioactive agent conjugates with an appropriate structure to promote a desired rate of release of the bioactive agent, those skilled in the art will appreciate, for example, that hydrolysis of an ester moiety will typically occur more readily than that of an amide or carbamate moiety. Thus to promote a rate of release of the bioactive agent from the polymer backbone that is faster than the rate of bioerosion of the polymer backbone, one might construct the conjugate such that the polymer backbone comprises only amide and/carbamate bioerodible moieties and the drug is conjugated to the backbone via an ester moiety.

Those skilled in the art will also appreciate that a factors such as steric crowding and electronic effects around a given bioerodible moiety can alter its propensity to undergo hydrolytic cleavage. Both the polymer backbone and the pendant bioactive agent can be a source of such effects. For example, a bioerodible moiety having a non hydrogen substituent (e.g. alkyl, aryl, alkylaryl, carbocyclyl) located $\alpha$ and/or $\beta$ to it will typically be less susceptible to hydrolytic

cleavage relative to the same moiety having hydrogen substituents located α and/or β to it. Accordingly, steric crowing around a given bioerodible moiety can be used to influence the rate of release of the bioactive moiety and/or the rate bioerosion of the polymer backbone.

Generally, the rate of hydrolytic cleavage will be in the order: anhydride moiety, ester moiety (including carboxylic acid esters, sulphate esters and phosphate esters) > carbamate > amide.

By tailoring at least the rate at which the bioactive agent is released from the polymer backbone, the polymer - bioactive agent conjugates of the invention can advantageously function as a sustained bioactive agent delivery system.

In the polymer - bioactive agent conjugate comprising as part of its polymer backbone a moiety of general formula (I):

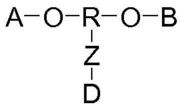

(I)

A and B, which may be the same or different, represent the remainder of the bioerodible polymer backbone and are (i) attached to the -O-R(ZD)-O- moiety as shown in formula (I) via a bioerodible moiety, and (ii) comprise a poly(urethane-ester) having monomeric units coupled via bioerodible urethane or ester moieties.

Depending upon the intended application, A and B may be selected for their biocompatible and/or their bioerodable properties. Those skilled in the art can readily select polymers to provide for such properties.

As used herein, "biocompatible polymer" refers to a polymer that both in its intact, that is, as synthesized, state and in its decomposed state (i.e. its degradation products), is compatible with living tissue in that it is not, or at least is minimally, toxic to living tissue; does not, or at least minimally and reparably does, injure living tissue; and/or does not, or at least minimally and/or controllably does, cause an immunological reaction in living tissue.

The moiety "R" present in the formulae herein represents a linear or branched optionally substituted hydrocarbon. In some embodiments the hydrocarbon may comprise between 1 and 12 carbon atoms, for example between 1 and 6 carbon atoms or 2 or 3 carbon atoms. The hydrocarbon may be partially or completely saturated or unsaturated (including moieties that are aromatic). Specific examples of R include a moiety having one of the following structures:

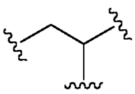 , 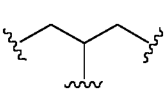 , 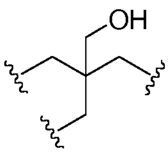 or 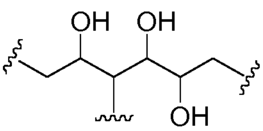

In accordance with the invention, the monomer - bioactive agent conjugate has general formula (II):

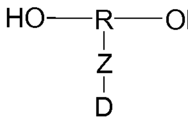

(II)

wherein:

R represents a linear or branched optionally substituted hydrocarbon;
Z is a linking group; and
D is a releasable bioactive agent selected from fluoroquinolone antibiotics.

In some embodiments, the monomer - bioactive agent conjugate may have a more specific structure of formula (III), (IV), (V) or (Va):

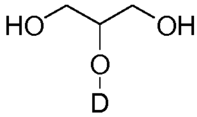 (III) , 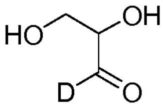 (IV) , 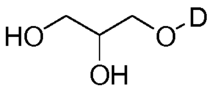 (V) or 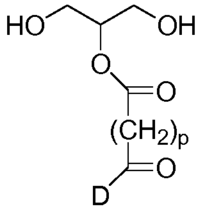 (Va)

wherein D is a releasable bioactive agent; and
p is between 1 and 18.

Disclosed is a process for preparing a monomer - bioactive agent conjugate of formula (II):

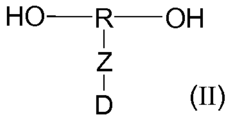 (II)

wherein:

R represents a linear or branched optionally substituted hydrocarbon;
Z is a linking group; and
D is a releasable bioactive agent,

said process comprising covalently coupling a linking precursor group Z' in a compound of formula (VI):

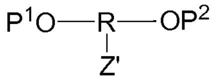
(VI)

wherein:

$P^1$ and $P^2$ are each independently H, a protecting group, or $P^1$ and $P^2$ together form a protecting group; and
R is as defined above,

with a group selected from D or D-Z",

wherein:
D is a bioactive agent in protected or unprotected form or prodrug thereof; and
Z" is a linking precursor group,

wherein the coupling of Z' with D, or the coupling of Z' with Z" in the group D-Z", forms the

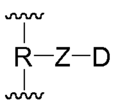

moiety in the conjugate of formula (II),
and where $P^1$ and/or $P^2$ are protecting groups said process further comprises the step of removing the protecting group or groups.

Z' and Z" (when present) are each linking precursor groups which afford the linking group Z in the monomer - bioactive agent conjugate of formula (II).

In some embodiments, Z' is a moiety comprising a functional group such as a carboxylic acid, acid halide, primary amino, secondary amino, hydroxy group, thiol group, phosphate group or sulphate group that couples to D or couples to Z" in the group D-Z".

In some embodiments, Z" is a moiety comprising a functional group such as a carboxylic acid, acid halide,

primary amino, secondary amino, hydroxy group, thiol group, phosphate group or sulphate group that couples to Z'.

For example, the compound of formula (VI) may be derived from a compound of formula (VII):

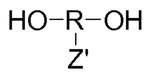

(VII)

wherein Z' and R are as defined above.

To promote reaction between D or D-Z" and the Z' moiety in general formula (VI), those skilled in the art will appreciate that the relevant coupling functional group on the bioactive agent may be suitably modified. For example, where Z' in formula (VI) comprises a hydroxy group, ester formation through reaction of this hydroxy group with a carboxylic acid group in D or a carboxylic acid group on Z" in D-Z" can in some cases prove difficult. Under these circumstances, ester formation may be facilitated by first converting the carboxylic acid group in D or D-Z" into an acid halide (e.g. acid chloride) group and then reacting the acid halide group with the hydroxy group. Coupling agents such as those mentioned in Tetrahedron Volume 60, Issue 11, 8 March 2004, pages 2447-2467, could also be employed. Examples of such coupling agents include those listed below in Table 1.

**Table 1:** Coupling agents for promoting reaction between carboxylic acid, acid halide and hydroxy groups.

| Acronym | Chemical name |
|---|---|
| AOMP | 5-(7-azabenzotriazol-1-yloxy)-3,4-dihydro-1-methyl 2H-pyrrolium hexachloroantimonate |
| AOP | (7-azabenzotriazol-1-yl)oxytris-(dimethylamino)phosphonium hexafluorophosphate |
| BDDC | bis[[4-(2,2-dimethyl-1,3-dioxolyl)]-methyl]carbodiimide |
| BDMP | 5-(1H-benzotriazol-1-yloxy)-3,4-dihydro-1-methyl 2H-pyrrolium hexachloroantimonate |
| BDP | benzotriazol-1-yl diethylphosphate |
| BEC | N-tert-butyl-N'-ethylcarbodiimide |
| BEMT | 2-bromo-3-ethyl-4-methyl thiazolium tetrafluoroborate |
| BEP | 2-bromo-1-ethyl pyridinium tetrafluoroborate |
| BEPH | 2-bromo-1-ethyl pyridinium hexachloroantimonate |
| BMC | N-tert-butyl-N'-methylcarbodiimide |
| BMP-Cl | N,N'-bismorpholinophosphinic chloride |
| BMPI | 2-bromo-1-methylpyridinium iodide |
| BMTB | 2-bromo-3-methyl-4-methyl thiazolium bromide |
| BOI | 2-(benzotriazol-1-yl)oxy-1,3-dimethyl-imidazolidinium hexafluorophosphate |
| BOMI | benzotriazol-1-yloxy-N,N-dimethyl-methaniminium hexachloroantimonate |
| BOP | benzotriazol-1-yloxytris(dimethyl-amino)phosphonium hexafluorophosphate |
| BOP-Cl | N,N'-bis(2-oxo-3-oxazolidinyl)-phosphinic chloride |
| BPMP | 1-(1H-benzotriazol-1-yloxy)phenyl-methylene pyrrolidinium hexachloroantimonate |
| BroP | bromotris(dimethylamino)phosphonium hexafluorophosphate |
| BTC | bis(trichloromethyl)carbonate |
| BTFFH | bis(tetramethylene)fluoroformamidinium hexafluorophosphate |
| Bts-Cl | benzothiazol-2-sulfonyl chloride |
| CBMIT | 1,1 '-carbonylbis(3 -methyl-imidazo lium)triflate |
| CDI | 1,1'-carbonyldiimidazole |
| CDMT | 2-chloro-4,6-dimethoxy-1,3,5-triazine |
| CIC | N-cyclohexyl-N'-isopropylcarbodiimide |

(continued)

| Acronym | Chemical name |
|---|---|
| CIP | 2-chloro-1,3-dimethylimidazolidinium hexafluorophosphate |
| CMBI | 2-chloro-1,3-dimethyl 1H-benzimidazolium hexafluorophosphate |
| CMPI | 2-chloro-1-methylpyridinium iodide |
| Cpt-Cl | 1-oxo-chlorophospholane |
| DBDMAP | 2,6-di-tert-butyl-4-(dimethylamino)pyridine |
| DCC | N,N'-dicyclohexylcarbodiimide |
| DEBP | diethyl 2-(3-oxo-2,3-dihydro-1,2-benzisosulfonazolyl)phosphonate |
| DECP | diethylcyanophosphonate |
| Deg | α,α-diethylglycine |
| DEPB | diethyl phosphorobromidate |
| DEPBO | N-diethoxyphosphoryl benzoxazolone |
| DEPBT | 3-(diethoxyphosphoryloxy)-1,2,3-benzotriazin-4(3H)-one |
| DEPC | diphenyl phosphorochloridate |
| DFIH | 1,3-dimethyl-2-fluoro-4,5-dihydro-1H-imidazolium hexafluorophosphate |
| DIC | N,N'-diisopropylcarbodiimide |
| DIEA(DIPE | diisopropylethylamine |
| A) | |
| DMTMM | 4-(4,6-dimethoxy[1,3,5]triazin-2-yl)-4-methylmorpholinium chloride |
| DOMP | 5-(3',4'-dihydro-4'-oxo-1',2',3'-benzotriazin-3'-yloxy)-3,4-dihydro-1-methyl 2H-pyrrolium hexachloroantimonate |
| DOPBO | N-(2-oxo-1,3,2-dioxaphosphorinanyl)-benzoxazolone |
| DOPBT | 3-[O-(2-oxo-1,3,2-dioxaphosphorinanyl)oxy]-1,2,3-benzotriazin-4(3H)-one |
| DPP-Cl | diphenylphosphinic chloride |
| DPPA | diphenylphosphoryl azide |
| DPTF | 2,2-dichloro-5-(2-phenylethyl)-4-(trimethylsilyl)-3-furanone |
| EDC | 1-[3-(dimethylamino)propyl]-3-ethylcarbodiimide hydrochloride |
| FDPP | pentafluorophenyl diphenyl phosphinate |
| FEP | 2-fluoro-1-ethyl pyridinium tetrafluoroborate |
| FEPH | 2-fluoro-1-ethyl pyridinium hexachloroantimonate |
| FOMP | 5-(pentafluorophenyloxy)-3,4-dihydro-1-methyl 2H pyrrolium hexachloroantimonate |
| HAMDU | O-(7-azabenzotriazol-1-yl)-1,3-dimethyl-1,3-dimethyleneuronium hexafluorophosphate |
| HAPipU | O-(7-azabenzotriazol-1-yl)-1,1,3,3-bis(pentamethylene)uronium hexafluorophosphate |
| HATU | O-(7-azabenzotriazol-1-yl)-1,1,3,3-tetramethyluronium hexafluorophosphate |
| HBPyU | O-(benzotriazol-1-yl)oxybis-(pyrrolidino)uronium hexafluorophosphate |
| HBTU | O-(benzotriazol-1-yl)-1,1,3,3-tetramethyluronium hexafluorophosphate |
| HDTU | O-(3,4-dihydro-4-oxo-1,2,3-benzotriazin-3-yl)-1,1,3,3-tetramethyluronium hexafluorophosphate |
| HIP | α-(α-hydroxyisovaleryl)propionic acid |

(continued)

| Acronym | Chemical name |
|---|---|
| HOAt | 1-hydroxy-7-azabenzotriazole |
| HOBt | 1-hydroxybenzotriazole |
| HOCt | ethyl-1-hydroxy-1H-1,2,3-triazole-4-carboxylate |
| HODhbt | 3,4-dihydro-3-hydroxy-4-oxo-1,2,3-benzotriazine |
| HODT | S-(1-oxido-2-pyridinyl)-1,3-dimethyl-1,3-trimethylenethiouronium hexafluorophosphate |
| HOSu | N-hydroxysuccinimide |
| HOTT | S-(1-oxido-2-pyridinyl)-1,1,3,3-tetramethylthiouronium hexafluorophosphate |
| HPyOPfp | N,N,N',N'-bis(tetramethylene)-O-pentafluorophenyluronium hexafluorophosphate |
| IBCF | isobutyl chloroformate |
| Iva | isovaline |
| MPTA | dimethylphosphinothioyl azide |
| MPTO | 3-dimethylphosphinothioyl-2(3H)-oxazolone |
| NDPP | norborn-5-ene-2,3-dicarboximidodiphenylphosphate |
| PTF | benzyltriphenylphosphonium dihydrogen trifluoride |
| PyAOP | [(7-azabenzotriazol-1-yl)oxy]tris-(pyrrolidino)phosphonium hexafluorophosphate |
| PyBOP | benzotriazol-1-yloxytri(pyrrolidino)phosphonium hexafluorophosphate |
| PyBroP | bromotri(pyrrolidino)phosphonium hexafluorophosphate |
| PyCloP | chlorotri(pyrrolidino)phosphonium hexafluorophosphate |
| PyClU | chloro-1,1,3,3-bis(tetramethylene)-formamidinium hexafluorophosphate |
| PyDOP | [(3,4-dihydro-4-oxo-1,2,3-benzotriazin-3-yl)oxy]tris-(pyrrolidino)phosphonium hexafluorophosphate |
| PyFOP | [[6-(trifluoromethyl)benzotriazol-1-yl]oxy]tris(pyrrolidino)phosphonium hexafluorophosphate |
| PyNOP | [(6-nitrobenzotriazol-1-yl)oxy]tris-(pyrrolidino)phosphonium hexafluorophosphate |
| PyTOP | (pyridyl-2-thio)tris(pyrrolidino)-phosphonium hexafluorophosphate |
| SOMP | 5-(succinimidyloxy)-3,4-dihydro-1-methyl 2H-pyrrolium hexachloroantimonate |
| TATU | O-(7-azabenzotriazol-1-yl)-1,1,3,3-tetramethyluronium tetrafluoroborate |
| TBTU | O-benzotriazol-1-yl-1,1,3,3-tetramethyluronium tetrafluoroborate |
| TDBTU | 2-(3,4-dihydro-4-oxo-1,2,3-benzotriazin-3-yl)-1,1,3,3-tetramethyluronium tetrafluoro borate |
| TEMP | 2,3,5,6-tetramethylpyridine |
| TFFH | tetramethylfluoroformamidinium hexafluorophosphate |
| Ths-Cl | 5-methyl-1,3,4-thiadiazole-2-sulfonyl chloride |
| TNTU | 2-(5-norbornene-2,3-dicarboximido)-1,1,3,3-tetramethyluronium tetrafluoro borate |
| TODT | S-(1-oxido-2-pyridinyl)-1,3-dimethyl-1,3-trimethylenethiouronium tetrafluoro borate |
| TOTT | S-(1-oxido-2-pyridinyl)-1,1,3,3-tetramethylthiouronium tetrafluoroborate |
| TOTU | O-[cyano(ethoxycarbonyl)methylene-amino]-N,N,N',N'-tetramethyluronium tetrafluoroborate |
| TSTU | 2-succinimido-1,1,3,3-tetramethyluronium tetrafluoroborate |

The compound of formula (VI), where Z' comprises a hydroxyl group, may be derived from a compound of formula (VII) through alcohol group protection. The compound of formula (VI), where Z' comprises a primary or secondary amino group, may be derived from a compound of formula (VII) through amino group protection. In some cases, the compound of formula (VI), where Z' comprises a carboxylic acid group, may be derived from a compound of formula (VII) using a protection group strategy. The skilled worker will recognise that the compound of formula (VI), where Z' comprises an acid halide, may be derived from the corresponding compound where Z' comprises a carboxylic acid.

Where the bioactive agent has more than one functional group that can be used to covalently couple it to a compound of formula (VI), those skilled in the art will be able to select appropriate reaction conditions, reagents and/or protecting groups to promote the desired coupling reaction.

Techniques, equipment and reagents well known in the art can advantageously be used to prepare the monomer - bioactive agent conjugates in accordance with the invention.

Examples of general strategies for synthesising monomer - bioactive agent conjugates of formula (II), which employ protecting group strategies, are represented in Scheme 1 below (where D is a releasable bioactive agent; and D' is that part of the releasable bioactive moiety other than the hydroxyl, amine, carboxylic acid, etc):

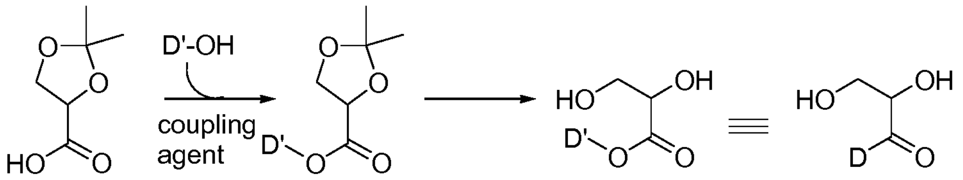
D'-OH
coupling
agent

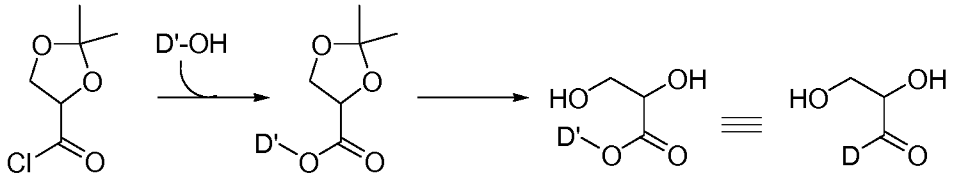
D'-OH

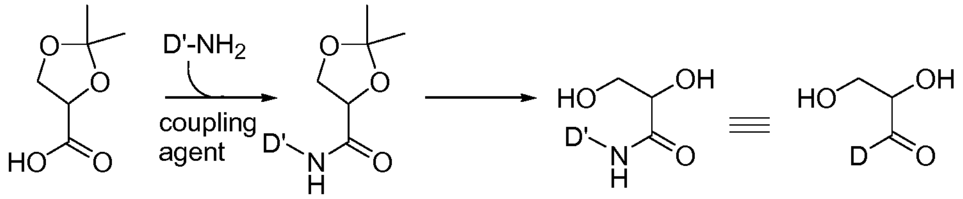
D'-NH2
coupling
agent

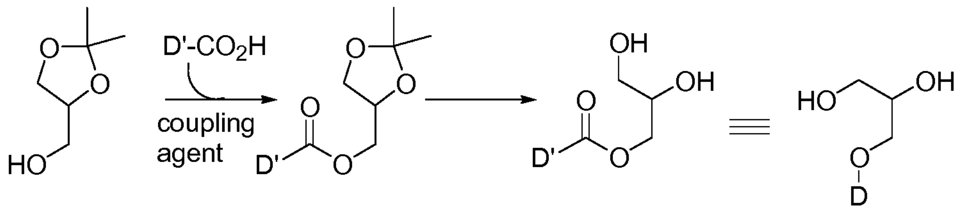
D'-CO2H
coupling
agent

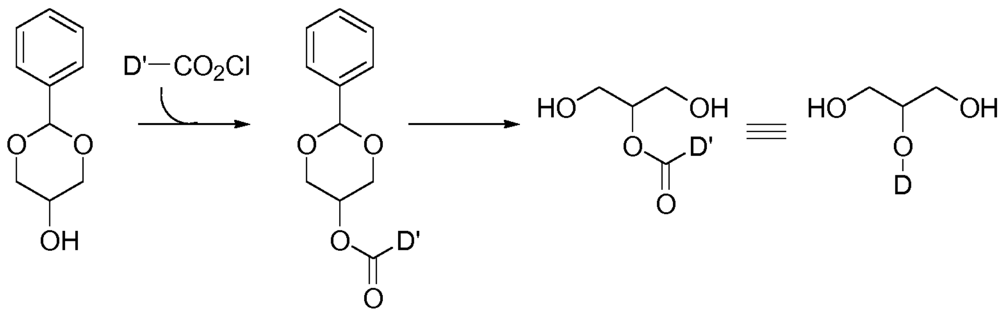
D'—CO2Cl

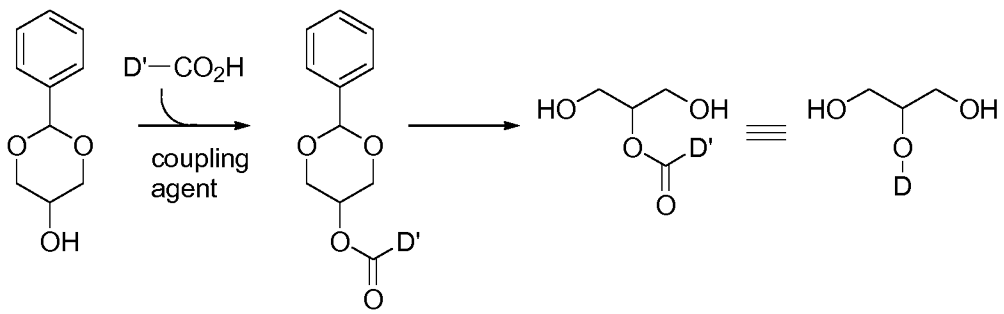
D'—CO2H
coupling
agent

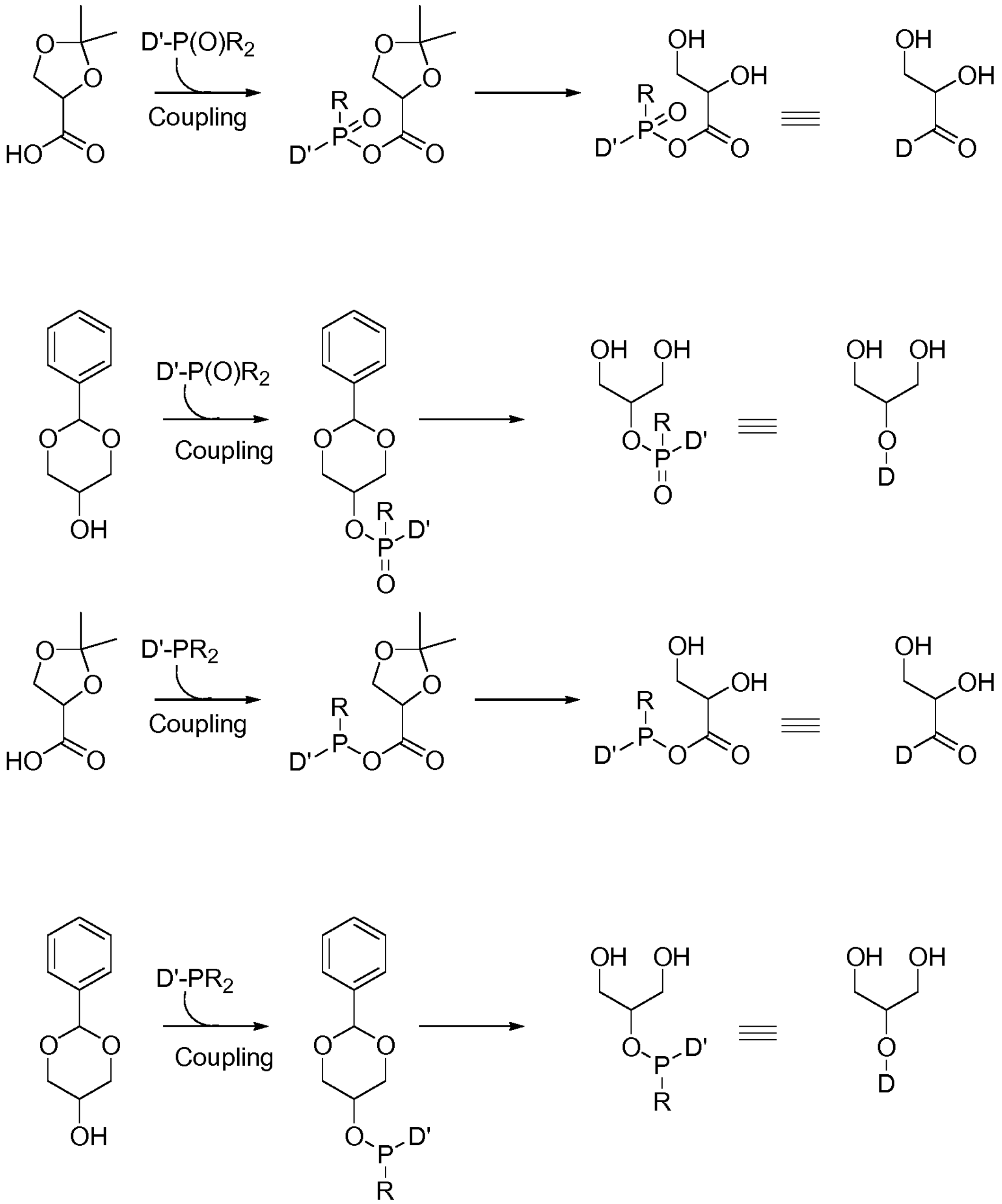


**Scheme 1:** General strategies for synthesising monomer – bioactive agent conjugates of formula (II).

The R group may be substituted with one or more groups comprising a releasable bioactive agent (D), as shown in the following structure derived from pentaerythritol:

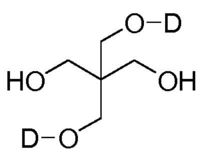

Where there is more than one bioactive agent within the monomer - bioactive agent conjugate (or polymer - bioactive agent conjugate) of the invention, each bioactive agent may be the same or different, but typically each bioactive agent will be the same.

It will be appreciated that in the conjugates shown in Scheme 1, each of the linking groups is either a -O- or

-C(O)- group. The Scheme will of course be equally applicable for other linking groups (Z) herein described.

Examples of general strategies for synthesising monomer - bioactive agent conjugates of formula (II), which employ protecting group strategies and use diacid-based linking groups, are represented in Scheme 2 below (where p is an integer from eg 1 to 18, D is a releasable bioactive agent; and D' is that part of the releasable bioactive moiety other than the hydroxyl, amine, carboxylic acid, etc):

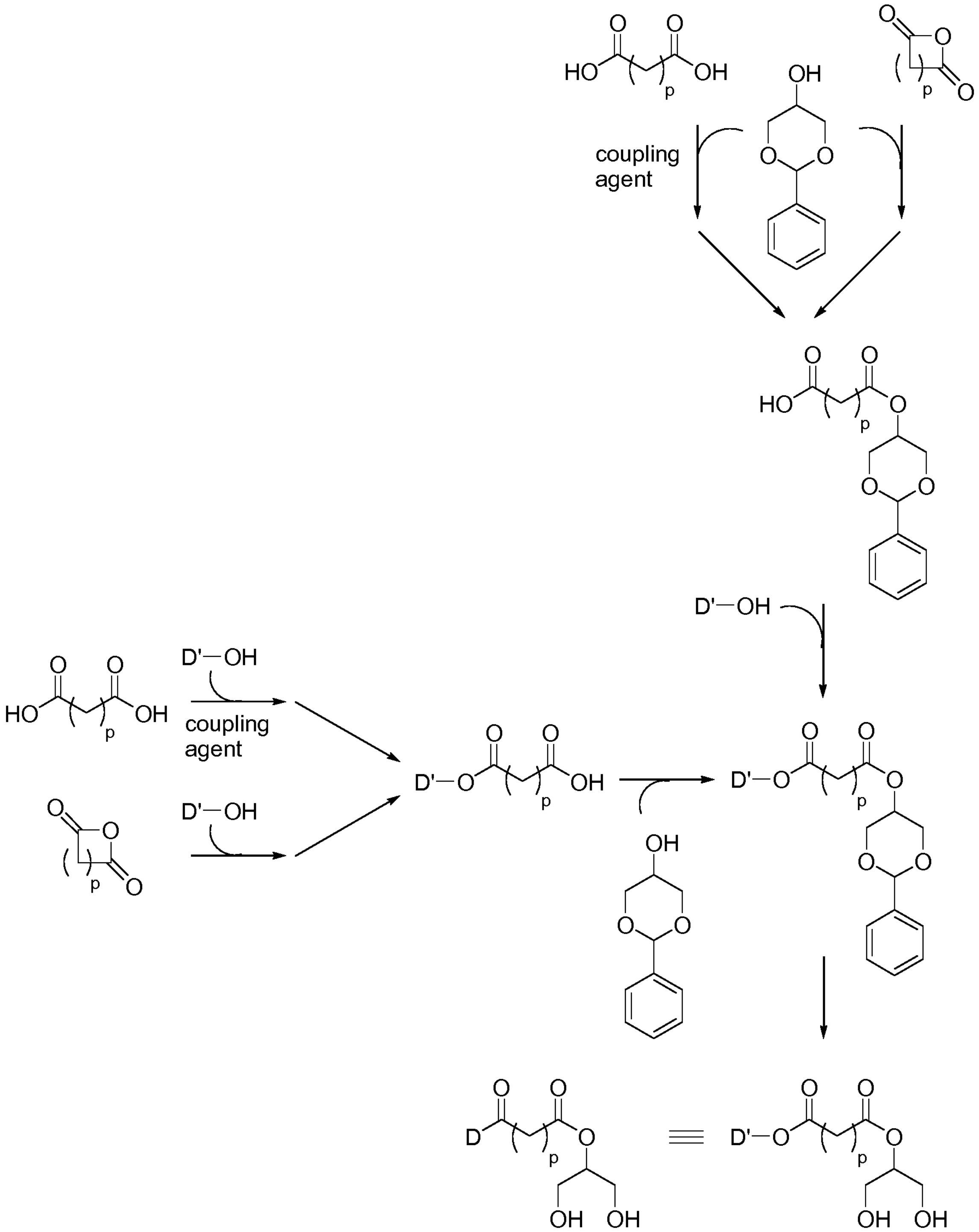


**Scheme 2:** General strategies for synthesising monomer – bioactive agent conjugates of formula (II)

With reference to the aspect of the present invention that provides a process for the preparation of a monomer

- bioactive agent conjugate of formula (II), Scheme 2 provides strategies for coupling compounds of formula (VI) to D and D-Z". In particular, in the synthesis of the compound of formula (II) denoted by:

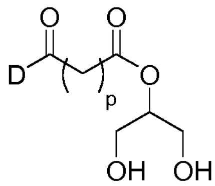

the Z group of the conjugate of formula (II) may be denoted by:

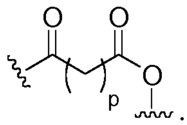

This Z group is formed through either:

i) the reaction of Z' as

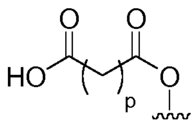

in a compound of formula (VI) with D as D'-OH; or

ii) the reaction of Z' as

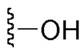

in a compound of formula (VI) with D-Z" as

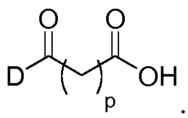

Specific examples of monomer - bioactive agent conjugates are shown below, with the bioactive agent being presented on the left and the monomer - bioactive agent conjugate being presented on the right:

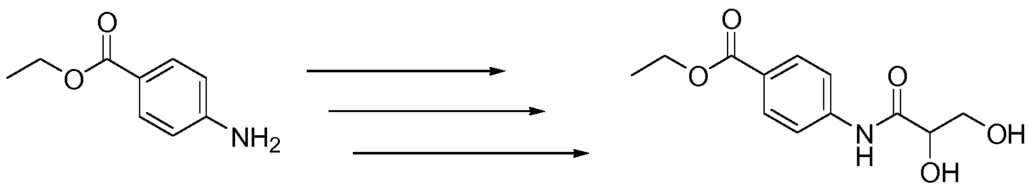

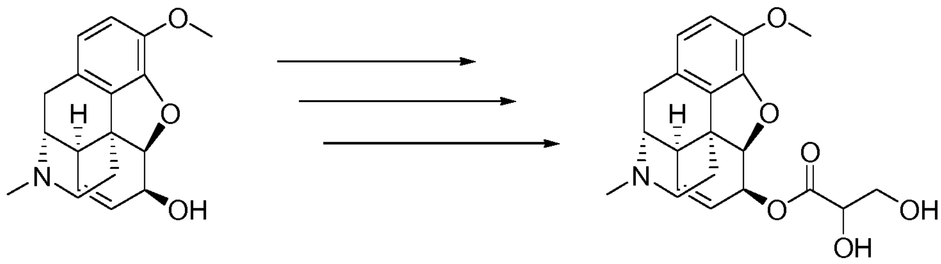

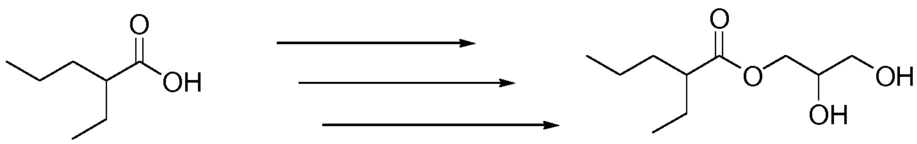

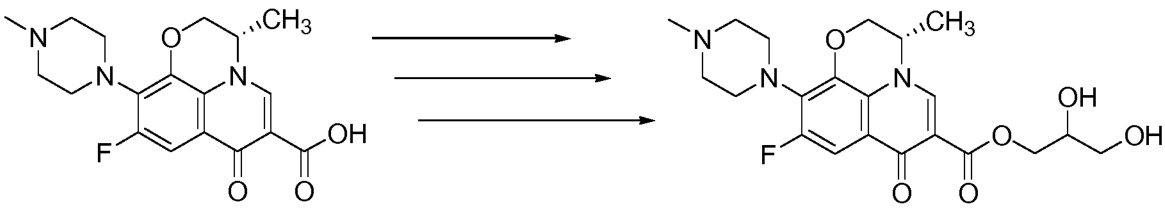

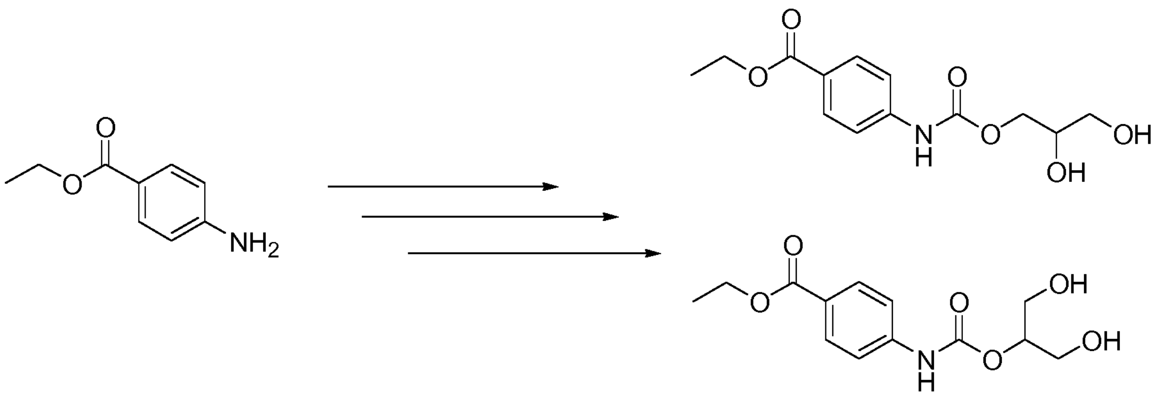

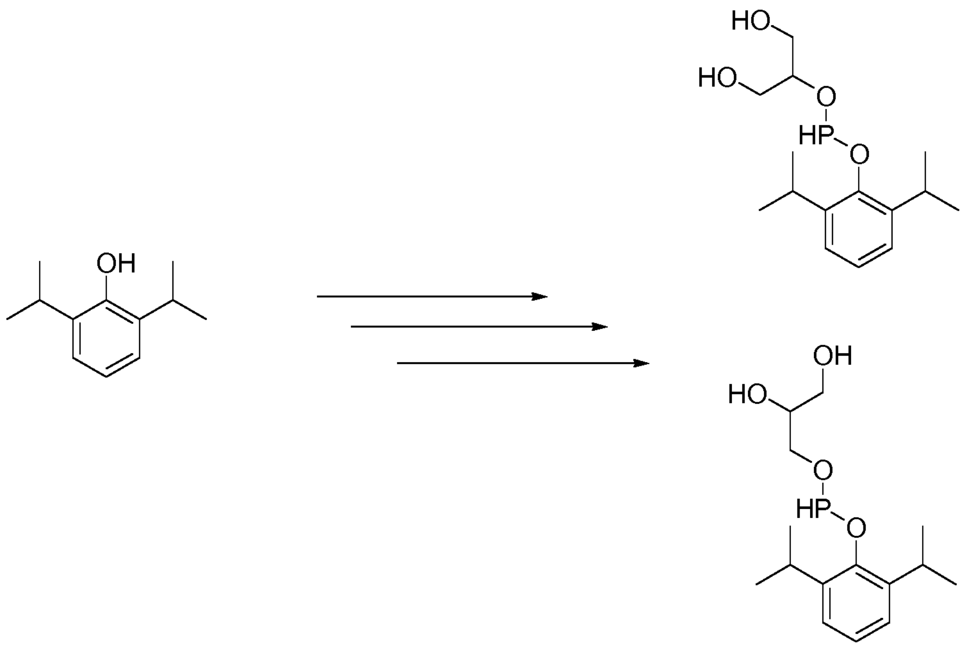

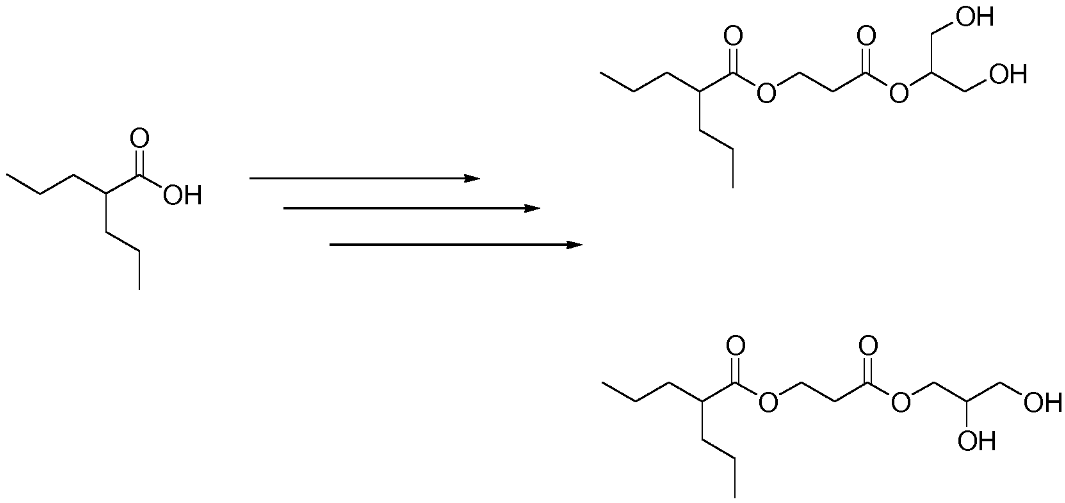

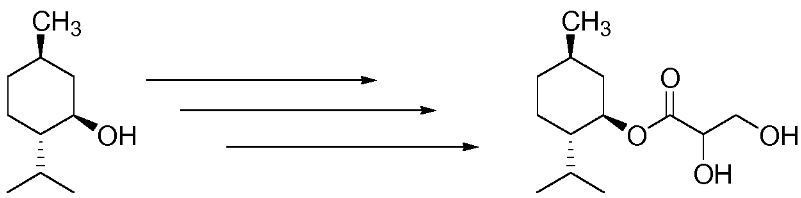

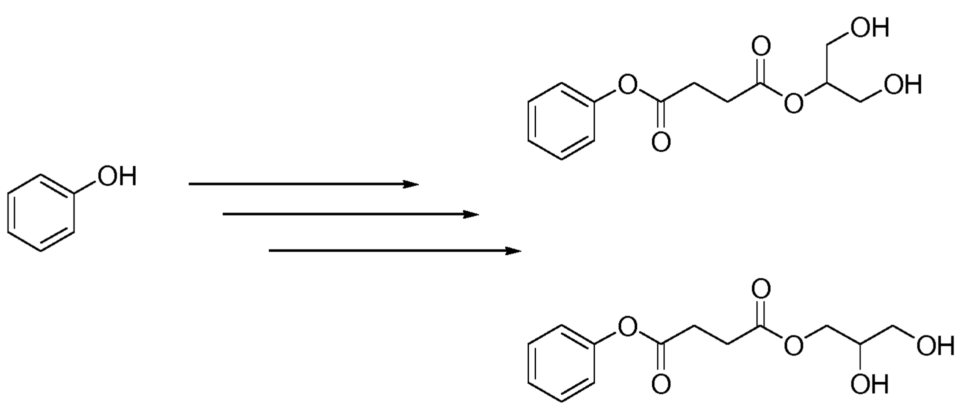

Further specific examples of monomer - bioactive agent conjugates are shown below, with the bioactive agent being presented on the left and the monomer - bioactive agent conjugate being presented on the right. The acetylated latanoprost monomer - bioactive agent conjugates (lower structures) represent examples of bioactive agents conjugated in prodrug form such that the acetyl groups will cleave *in vivo:*

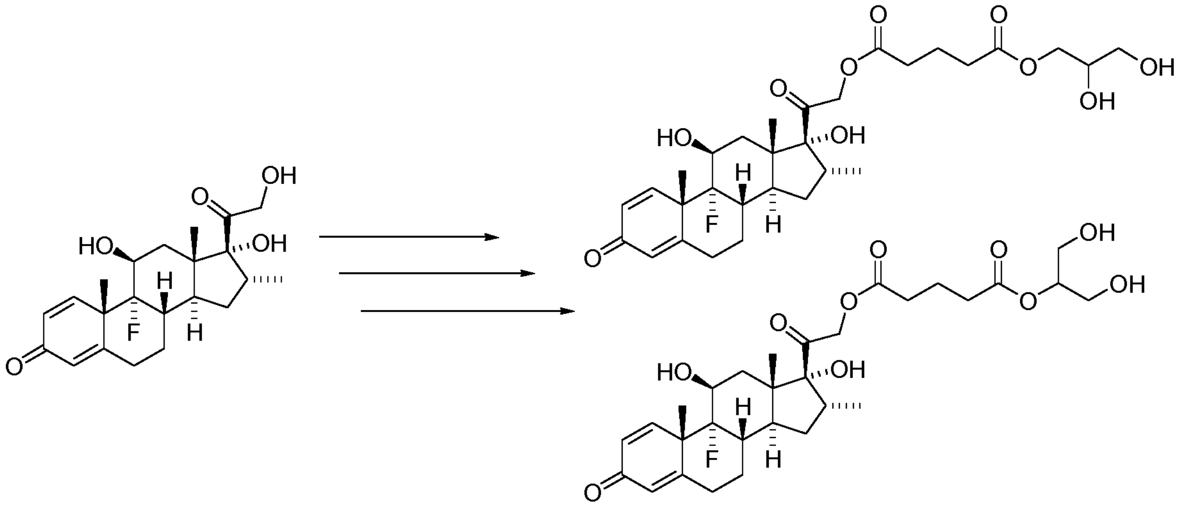

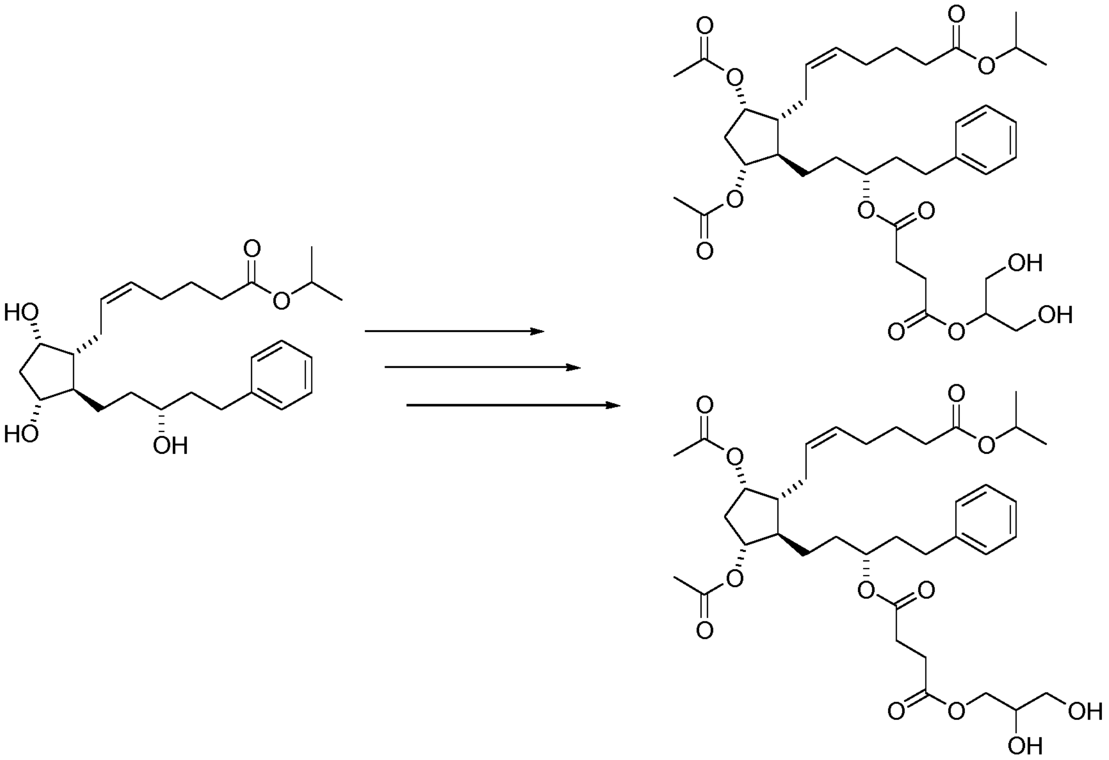

The invention also provides a process for making a polymer - bioactive agent conjugate comprising as part of its polymer backbone a moiety of general formula (I):

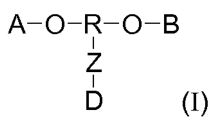
(I)

wherein:

A and B, which may be the same or different, represent the remainder of the polymer backbone and are (i) attached

to the -O-R(ZD)-O- moiety as shown in formula (I) via a bioerodible moiety, and (ii) comprise a poly(urethane-ester) having monomeric units coupled via bioerodible urethane or ester moieties;
R represents a linear or branched optionally substituted hydrocarbon;
Z is a linking group; and
D is a releasable bioactive agent;

said process comprising the step of polymerising a monomer of formula (II):

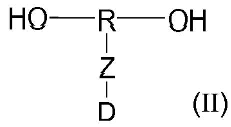

(II)

wherein:

R, Z and D are as defined above;
with a polyisocyanate and a polyester polyol.

The monomers that are polymerised with the monomer - bioactive agent conjugate of formula (II) to form the bioerodible polymer - bioactive agent conjugates of the invention will not only comprise compatible chemical functionality to react with the monomer - bioactive agent conjugate but that reaction will of course give rise to a bioerodible moiety.

Those skilled in the art will also recognise that polymerisation of a diol of formula (II) with a polyisocyanate takes place in the presence of a polyester polyol. The structures of these one or more other types of polyols may or may not comprise one or more bioactive moieties. An example of this second type of polyol is 1,6-hexanediol. The polymer - bioactive agent conjugate so-formed may or may not have a bioactive agent loading of less than 50 mol%. For example where diol of formula (II) is polymerised in the presence of an equimolar amount of 1,6-hexanediol and 2 molar equivalents of diisocyanate, the polyurethane so-formed will typically comprise the residues of the three components in the ratio of 1:1:2. Such conjugates are contemplated by the present invention. Such polymer systems may provide a useful means of modifying the physical properties of the polymer conjugates.

Generally, the polymer - bioactive agent conjugate will comprise at least 10, at least 25 , at least 35, at least 45 or up to 50 mol% of bioactive agent (D), relative to the total number of moles of monomer that form the polymer.

In some embodiments, the polymer - bioactive agent conjugate will comprise up to 60, up to 70 , up to 80, up to 90 and even up to 100 mol% of conjugated bioactive agent (D), relative to the total number of moles of monomer that form the polymer. However, in that case, it will be appreciated that the amount of conjugated bioactive agent greater than 50 mol% will necessarily be derived from moieties other than the -O-R(ZD)-O- moieties. For example, the other monomers which a polymerised with the monomer - bioactive agent conjugate of the invention may also comprise conjugated bioactive agent.

Suitable polyisocyanates that may be used to prepare the polymer - bioactive agent conjugates include aliphatic, aromatic and cycloaliphatic polyisocyanates and combinations thereof. Specific polyisocyanates include, but are not limited to, diisocyanates such as m-phenylene diisocyanate, p-phenylene diisocyanate, 2,4-toluene diisocyanate, 2,6-toluene diisocyanate, 1,6-hexamethylene diisocyanate, 1,4-hexamethylene diisocyanate, 1,3-cyclohexane diisocyanate, 1,4-cyclohexane diisocyanate, hexahydro-toluene diisocyanate and its isomers, isophorone diisocyanate, dicyclohexylmethane diisocyanates, 1,5-napthylene diisocyanate, 4,4'-diphenylmethane diisocyanate, 2,4' diphenylmethane diisocyanate, 4,4'-biphenylene diisocyanate, 3,3'-dimethoxy-4,4'-biphenylene diisocyanate, and 3,3'-dimethyl-diphenylpropane-4,4'-diisocyanate; triisocyanates such as 2,4,6-toluene triisocyanate; higher -isocyanates such as 4,4'-dimethyldiphenylmethane-2,2',5,5'-tetraisocyanate, polymethylene polyphenyl-polyisocyanates and alkyl esters of lysine diisocyanate (for example ethyl ester of lysine diisocyanate - ELDI); and combinations thereof. It will be appreciated that the use of polyisocyanates comprising greater than two isocyanate moieties provides for branched structures.

Techniques, equipment and reagents well known in the art can advantageously be used to prepare the polymer - bioactive agent conjugates in accordance with the invention.

For example, polyurethanes might be prepared batch wise by mixing all components together and waiting until an exotherm occurs followed by casting the mixture into a container. The mixture can be subsequently heated to drive the reaction. When adopting this approach, the components to be mixed might first be made up into two parts before mixing: Part-1 might include a compound of general formula (III) and one or more of: a polyol (e.g. polyester polyol), a chain extender, blowing agent (e.g water), catalyst, and surfactants etc. Part-2 will generally comprise the polyisocyanate. Part-1 or Part-2 can also contain other additives such as fillers, pigments etc.

The polyurethanes might also be prepared as a prepolymer that is subsequently reacted with a chain extender. For example, through suitable adjustment of molar ratios, an isocyanate terminated pre-polymer may be prepared by

mixing Parts -1 and -2 mentioned above. The isocyanate terminated polymer could then be reacted with a chain extender/ branching molecule such as a short chain diol (e.g. 1,4-butanediol) or polyol (such as a triol). Alternatively, through suitable adjustment of molar ratios, the prepolymer could be produced such that it was hydroxy terminated. This hydroxy terminated prepolymer could then be reacted with a polyisocyanate to produce the desired polyurethane.

It may also be desirable for the bioerodible polymers to be substantially amorphous and thus contain a low amount of crystalline segments. Such polymers can provide a more predictable rate of bioactive agent release as well as assisting with the production of materials that are more flexible which can assist with producing coatings as well as assisting with the rate of polymer erosion.

Additional variables such as the choice of co-monomers and the means to produce the polymers can also assist with the production of highly amorphous and/or flexible polymers. For example, using monomers such as caprolactone or polyester polyols such as polycaprolactone diol can decrease the crystallinity and increase the flexibility of the resulting polymer.

For the polyurethanes it may be important to limit the content of hard segment (use conventional definition of hard segment - chain extenders + diisocyanates). One example of a typical class of chain extender are low lolecular weight diols. Examples of such chain extenders include ethylene glycol, propane diol, propylene glycol, butane diol etc. Apart from the potential toxicity of such chain extenders, limiting the hard segments in the polyurethanes also provides a reason to reduce the low molecular weight diol content as described above.

The polyurethane forming reactions can be carried out in a range of different equipment including batch kettles, static mixers, reactive injection moulders or extruders.

It also may be advantageous to heat the reagents prior to or during the reaction process to improve their solubility or to enhance their reactivity. The reaction process may also be conducted in solvent.

Suitable polyacids that may be used to prepare the polymer - bioactive agent conjugates include aliphatic, aromatic and cycloaliphatic polyacids and combinations thereof. Specific polyacids include, but are not limited to the following, oxalic acid, fumaric acid, maleic acid, succinic acid, glutaric acid, adipic acid, pimelic acid, suberic acid, azelaic acid, sebacic acid, phthalic acid, dodecanediacid, isophthalic acid, terephthalic acid, dodecylsuccinic acid, napthalene-2,6-dicarboxylic acid, naphthalene-2,7-dicarboxylic acid, cyclohexane dicarboxylic acid, fumaric acid, itaconic acid, malonic acid, mesaconic acid. Esters, diesters and anhydrides of the above diacids are also suitable in the process of the invention.

Polyesters might be prepared batch wise by mixing all components together with heating and continued stirring. A condensate of the reaction such as water or low molecular weight alcohol (depending if acids or esters are used as the co-monomer) can be removed by distillation. To promote further reaction produce higher molecular weight polyester the temperature may be increased and vacuum applied.

A polycondensation catalyst well known to those skilled in the art can be included in the reaction mixture to increase the rate of polymerisation.

The reaction may also be conducted in an appropriate solvent to help increase the rate of polymerisation. The solvent will generally be selected to have only minimal solubility with the condensate (e.g. water or low molecular weight alcohol). For example the reaction may be carried out in toluene and a toluene / condensate mixture distilled off continuously and the condensate allowed to separate in a Dean - Stark trap.

To further increase the molecular weight of the polyester a second stage reaction in either a wiped film reactor or a solid state reactor may be employed. The need to use such reactors will depend upon the target molecular weight as well as the suitability of the polymer for further reaction.

Where the polyesters are prepared using an carboxylic acid halide monomer, those skilled in the art will appreciate that the condensation reaction is driven by the removal of HX (where X is a halide). For example, if a di-acid chloride co-monomer is with the monomer - bioactive agent conjugate of formula (II), HCl will be liberated from the reaction. Such a reaction may be carried out in solution at an elevated temperature to drive the reaction. It is also possible to add an appropriate base to form a salt with the liberated acid halide. For example an excess of triethyl amine may be included in a reaction mixture containing a 1: 1 molar ratio of a di-acid chloride co-monomer and the monomer - bioactive agent conjugate of formula (II). The reaction will afford the desired polymer - bioactive agent conjugate and a triethylamine hydrochloride salt.

With all such polycondensation reactions, it is possible to some extent to control the molecular weight of the resulting polyester, its degree of branching (through control of monomer functionality) and its end group functionality by adjustment of the molar ratio's and the functionality of the monomers used in the reaction.

For example, in some instances it may be desirable to produce lower molecular weight polyesters that could be used as polymer - bioactive agent conjugate polyester polyols for reaction with polyisocyanates and perhaps other reagents for the production of polyester-urethanes.

Additionally, it may be possible to increase the molecular weight and/or degree of branching of the polyester through the inclusion in the reaction mixture of coupling/branching agents. Examples of such coupling/branching agents include: polyepoxides, polyisocyanates, polyoxazolines. In that case, the "poly" is used to indicate a reactive functionality

of 2 or more (e.g. 2 or more epoxy groups). Having a reactive functionality of 2 will tend to produce higher molecular polymers still with a significant polyester-like character. Agents having a functionality of more than 2 will produce a branched polymer, still with significant polyester-like character.

As discussed in more detail in the Example section below, the synthesis monomer bioactive agent conjugates typically required the optimization of reaction conditions and the alteration of known purification methodologies. The desired hydrolytic instability of the monomer bioactive agent conjugates restricted the usage of several traditional purification methodologies and made the development of alternative pathways necessary.

The presence of any impurities in the monomer bioactive agent conjugates can also affect the molecular weight, and structure of the final polymer as well as the release rate of the bioactive from the polymer conjugate.

Further, the use of the monomer bioactive agent conjugates in the formation of the polymers in some cases required specific polymerisation methodology to be developed to allow the efficient incorporation of the monomer bioactive agent conjugates into the polymer. This included selection of appropriate solvents / heating / mixing/ catalyst / order of monomer additions etc to allow the incorporation of the monomer bioactive agent conjugates as well as to minimise the amount of degradation or premature release of the bioactive agent.

Careful selection of co-monomers / reaction conditions etc may also be required for a given monomer bioactive agent conjugate in order to produce a polymer conjugate with appropriate bioactive agent loading as well as have mechanical properties, bioactive release rate, formability etc.

Additionally, methods were developed to remove impurities from the polymer bioactive agent conjugates in order provide control of the release rate of the bioactive from the polymer conjugate.

Irrespective of the manner in which the bioerodible polymer - bioactive agent conjugates are prepared, as discussed above all repeat units that make up the polymer backbone will be coupled via a bioerodible moiety. Accordingly, any monomer or macoromer used in the preparation of the conjugates shall not contain repeat units that are couples by a non-bioerodible moiety such as an ether.

While the use of polyether segments can provide desirable improvements in the flexibility and in some cases can provide an improvement in the desired rate of release of the bioactive the release of polyethers having a moleculr weight below 1000 g/mol can result in increased toxicity from the degradation products.

In most cases it is possible to use polyester polyols such as polycaprolactone diol to also provide desired increase in the flexibility of the polymer as well as providing improvements in the release of the bioactive agent. However, the polyester polyols can adavatgeously degrade into more benign monomeric components.

Additioanlly, the use of polyester polyols such as DLLA (DL lactide) or PLGA (poly(lactic-co-glycolic acid))can provide an increase rate of degradation of the polymer as well as release of the drug through the autocatalytic effect of the released acidic components.

The polymer-bioactive agent conjugates in accordance with the invention can be incorporated or made into coatings and scaffolds for target *in vitro* and *in vivo* applications in a variety of ways.

For example, the polymer conjugates of the invention may be used in the manufacture of sutures, dental devices, orthopaedic fixation devices, transdermal patches, ligating clips, vascular grafts, stents, and tissue-engineering scaffolds. The polymers may be applied to the outside or inside of the body of the subject in need of treatment.

Coatings containing the polymer-bioactive agent conjugate(s) can advantageously be directly produced using techniques well known in the art including: solution casting, spray coating, melt pressing, transfer moulding, lamination, moulding onto a premade film containing the conjugate, rotomoulding, spin coating, extrusion coating, electrospinning etc.

Premade films for use in subsequent application as coatings can advantageously also be produced using techniques well known in the art including: film extrusion, film blowing, tentering etc.

The premade films may be applied as coatings by: melt pressing, vacuum forming, thermoforming, transfer lamination, adhesive bonding.

Coatings may be included as films, multilayer films, non-uniform or graded coatings as dots, patterns or structures according to some form of mask or template.

Three dimensional scaffolds containing the polymer-bioactive agent conjugate(s) can also be formed in a number of means including:

- Fibre based structures which in turn are knitted, woven, spun bonded or formed into non-woven mats etc. Additionally, fibre structures could be formed with a binder resin into composite structures. The fibres could be formed by melt extrusion, wet spinning or the bioactive conjugate could be over-coated or dispersed within fibres by bicomponent fibre extrusion, dip or spray coating etc.
- Moulded structures could be produced by injection moulding, blow moulding, reactive injection moulding, casting or moulding with subsequent machining etc.
- Porous structures could be made by moulding / extrusion in the presence of the porogen. Additionally porous 3D structures could be produced by moulding or polymerisation in the presence of an extractable material. For example, a polyurethane containing the monomer-bioactive agent conjugate or polymer-bioactive agent conjugate could be

formed by casting around a sufficient content of polystyrene beads. The polystyrene beads could be removed by extraction with an appropriate solvent.

In this specification "optionally substituted" is taken to mean that a group may or may not be substituted or fused (so as to form a condensed polycyclic group) with one, two, three or more of organic and inorganic groups (i.e. the optional substituent) including those selected from: alkyl, alkenyl, alkynyl, carbocyclyl, aryl, heterocyclyl, heteroaryl, acyl, aralkyl, alkaryl, alkheterocyclyl, alkheteroaryl, alkcarbocyclyl, halo, haloalkyl, haloalkenyl, haloalkynyl, haloaryl, halocarbocyclyl, haloheterocyclyl, haloheteroaryl, haloacyl, haloaryalkyl, hydroxy, hydroxyalkyl, hydroxyalkenyl, hydroxyalkynyl, hydroxycarbocyclyl, hydroxyaryl, hydroxyheterocyclyl, hydroxyheteroaryl, hydroxyacyl, hydroxyaralkyl, alkoxyalkyl, alkoxyalkenyl, alkoxyalkynyl, alkoxycarbocyclyl, alkoxyaryl, alkoxyheterocyclyl, alkoxyheteroaryl, alkoxyacyl, alkoxyaralkyl, alkoxy, alkenyloxy, alkynyloxy, aryloxy, carbocyclyloxy, aralkyloxy, heteroaryloxy, heterocyclyloxy, acyloxy, haloalkoxy, haloalkenyloxy, haloalkynyloxy, haloaryloxy, halocarbocyclyloxy, haloaralkyloxy, haloheteroaryloxy, haloheterocyclyloxy, haloacyloxy, nitro, nitroalkyl, nitroalkenyl, nitroalkynyl, nitroaryl, nitroheterocyclyl, nitroheteroayl, nitrocarbocyclyl, nitroacyl, nitroaralkyl, amino ($NH_2$), alkylamino, dialkylamino, alkenylamino, alkynylamino, arylamino, diarylamino, aralkylamino, diaralkylamino, acylamino, diacylamino, heterocyclamino, heteroarylamino, carboxy, carboxyester, amido, alkylsulphonyloxy, arylsulphenyloxy, alkylsulphenyl, arylsulphenyl, thio, alkylthio, alkenylthio, alkynylthio, arylthio, aralkylthio, carbocyclylthio, heterocyclylthio, heteroarylthio, acylthio, sulfoxide, sulfonyl, sulfonamide, aminoalkyl, aminoalkenyl, aminoalkynyl, aminocarbocyclyl, aminoaryl, aminoheterocyclyl, aminoheteroaryl, aminoacyl, aminoaralkyl, thioalkyl, thioalkenyl, thioalkynyl, thiocarbocyclyl, thioaryl, thioheterocyclyl, thioheteroaryl, thioacyl, thioaralkyl, carboxyalkyl, carboxyalkenyl, carboxyalkynyl, carboxycarbocyclyl, carboxyaryl, carboxyheterocyclyl, carboxyheteroaryl, carboxyacyl, carboxyaralkyl, carboxyesteralkyl, carboxyesteralkenyl, carboxyesteralkynyl, carboxyestercarbocyclyl, carboxyesteraryl, carboxyesterheterocyclyl, carboxyesterheteroaryl, carboxyesteracyl, carboxyesteraralkyl, amidoalkyl, amidoalkenyl, amidoalkynyl, amidocarbocyclyl, amidoaryl, amidoheterocyclyl, amidoheteroaryl, amidoacyl, amidoaralkyl, formylalkyl, formylalkenyl, formylalkynyl, formylcarbocyclyl, formylaryl, formylheterocyclyl, formylheteroaryl, formylacyl, formylaralkyl, acylalkyl, acylalkenyl, acylalkynyl, acylcarbocyclyl, acylaryl, acylheterocyclyl, acylheteroaryl, acylacyl, acylaralkyl, sulfoxidealkyl, sulfoxidealkenyl, sulfoxidealkynyl, sulfoxidecarbocyclyl, sulfoxidearyl, sulfoxideheterocyclyl, sulfoxideheteroaryl, sulfoxideacyl, sulfoxidearalkyl, sulfonylalkyl, sulfonylalkenyl, sulfonylalkynyl, sulfonylcarbocyclyl, sulfonylaryl, sulfonylheterocyclyl, sulfonylheteroaryl, sulfonylacyl, sulfonylaralkyl, sulfonamidoalkyl, sulfonamidoalkenyl, sulfonamidoalkynyl, sulfonamidocarbocyclyl, sulfonamidoaryl, sulfonamidoheterocyclyl, sulfonamidoheteroaryl, sulfonamidoacyl, sulfonamidoaralkyl, nitroalkyl, nitroalkenyl, nitroalkynyl, nitrocarbocyclyl, nitroaryl, nitroheterocyclyl, nitroheteroaryl, nitroacyl, nitroaralkyl, cyano, sulfate and phosphate groups.

In some embodiments, it may be desirable that a group (for example the R group) is optionally substituted with a polymer chain. An example of such a polymer chain includes a polyester, polyurethane, or copolymers thereof. Such a polymer chain may, or may not, have one or more bioactive agents appended thereto. For example, the R group of the formulae disclosed herein may be substituted with a polymer chain. The skilled worker will recognise that the R group may therefore represent a point of branching of the polymer backbone within the polymer - bioactive agent conjugate of the present invention. If R is substituted with a polymer chian, that polymer chain should also be bioerodible and not contain any repeat units that are coupled with a non-bioerodible moiety as described herein.

Preferred optional substituents include the aforementioned reactive functional groups or moieties, polymer chains and alkyl, (e.g. $C_{1-6}$ alkyl such as methyl, ethyl, propyl, butyl, cyclopropyl, cyclobutyl, cyclopentyl or cyclohexyl), hydroxyalkyl (e.g. hydroxymethyl, hydroxyethyl, hydroxypropyl), alkoxyalkyl (e.g. methoxymethyl, methoxyethyl, methoxypropyl, ethoxymethyl, ethoxyethyl, ethoxypropyl etc) alkoxy (e.g. $C_{1-6}$ alkoxy such as methoxy, ethoxy, propoxy, butoxy, cyclopropoxy, cyclobutoxy), halo, trifluoromethyl, trichloromethyl, tribromomethyl, hydroxy, phenyl (which itself may be further substituted e.g., by $C_{1-6}$ alkyl, halo, hydroxy, hydroxy$C_{1-6}$ alkyl, $C_{1-6}$ alkoxy, halo$C_{1-6}$alkyl, cyano, nitro $OC(O)C_{1-6}$ alkyl, and amino), benzyl (wherein benzyl itself may be further substituted e.g., by $C_{1-6}$ alkyl, halo, hydroxy, hydroxy$C_{1-6}$alkyl, $C_{1-6}$ alkoxy, halo$C_{1-6}$ alkyl, cyano, nitro $OC(O)C_{1-6}$ alkyl, and amino), phenoxy (wherein phenyl itself may be further substituted e.g., by $C_{1-6}$ alkyl, halo, hydroxy, hydroxy$C_{1-6}$ alkyl, $C_{1-6}$ alkoxy, halo$C_{1-6}$ alkyl, cyano, nitro $OC(O)C_{1-6}$ alkyl, and amino), benzyloxy (wherein benzyl itself may be further substituted e.g., by $C_{1-6}$ alkyl, halo, hydroxy, hydroxy$C_{1-6}$ alkyl, $C_{1-6}$ alkoxy, halo$C_{1-6}$ alkyl, cyano, nitro $OC(O)C_{1-6}$ alkyl, and amino), amino, alkylamino (e.g. $C_{1-6}$ alkyl, such as methylamino, ethylamino, propylamino etc), dialkylamino (e.g. $C_{1-6}$ alkyl, such as dimethylamino, diethylamino, dipropylamino), acylamino (e.g. $NHC(O)CH_3$), phenylamino (wherein phenyl itself may be further substituted e.g., by $C_{1-6}$ alkyl, halo, hydroxy hydroxy$C_{1-6}$ alkyl, $C_{1-6}$ alkoxy, halo$C_{1-6}$ alkyl, cyano, nitro $OC(O)C_{1-6}$ alkyl, and amino), nitro, formyl, -C(O)-alkyl (e.g. $C_{1-6}$ alkyl, such as acetyl), O-C(O)-alkyl (e.g. $C_{1-6}$alkyl, such as acetyloxy), benzoyl (wherein the phenyl group itself may be further substituted e.g., by $C_{1-6}$ alkyl, halo, hydroxy hydroxy$C_{1-6}$ alkyl, $C_{1-6}$ alkoxy, halo$C_{1-6}$ alkyl, cyano, nitro $OC(O)C_{1-6}$alkyl, and amino), replacement of $CH_2$ with C=O, $CO_2H$, $CO_2$alkyl (e.g. $C_{1-6}$ alkyl such as methyl ester, ethyl ester, propyl ester, butyl ester), $CO_2$phenyl (wherein phenyl itself may be further substituted e.g., by $C_{1-6}$ alkyl, halo, hydroxy, hydroxyl $C_{1-6}$ alkyl, $C_{1-6}$ alkoxy, halo $C_{1-6}$ alkyl, cyano, nitro $OC(O)C_{1-6}$ alkyl, and amino), $CONH_2$, CONHphenyl (wherein phenyl itself may be further substituted e.g., by $C_{1-6}$ alkyl, halo, hydroxy, hydroxyl $C_{1-6}$

alkyl, $C_{1-6}$ alkoxy, halo $C_{1-6}$ alkyl, cyano, nitro $OC(O)C_{1-6}$ alkyl, and amino), CONHbenzyl (wherein benzyl itself may be further substituted e.g., by $C_{1-6}$ alkyl, halo, hydroxy hydroxyl $C_{1-6}$ alkyl, $C_{1-6}$ alkoxy, halo $C_{1-6}$ alkyl, cyano, nitro $OC(O)C_{1-6}$ alkyl, and amino), CONHalkyl (e.g. $C_{1-6}$ alkyl such as methyl ester, ethyl ester, propyl ester, butyl amide) CONHdialkyl (e.g. $C_{1-6}$ alkyl) aminoalkyl (e.g., HN $C_{1-6}$ alkyl-, $C_{1-6}$alkylHN-$C_{1-6}$ alkyl- and ($C_{1-6}$ alkyl)$_2$N-$C_{1-6}$ alkyl-), thioalkyl (e.g., HS $C_{1-6}$ alkyl-), carboxyalkyl (e.g., $HO_2CC_{1-6}$ alkyl-), carboxyesteralkyl (e.g., $C_{1-6}$ alkyl$O_2CC_{1-6}$ alkyl-), amidoalkyl (e.g., $H_2N(O)CC_{1-6}$ alkyl-, H($C_{1-6}$ alkyl)N(O)$CC_{1-6}$ alkyl-), formylalkyl (e.g., $OHCC_{1-6}$alkyl-), acylalkyl (e.g., $C_{1-6}$ alkyl(O)$CC_{1-6}$ alkyl-), nitroalkyl (e.g., $O_2NC_{1-6}$ alkyl-), sulfoxidealkyl (e.g., $R^3(O)SC_{1-6}$ alkyl, such as $C_{1-6}$ alkyl(O)$SC_{1-6}$ alkyl-), sulfonylalkyl (e.g., $R^3(O)_2SC_{1-6}$ alkyl- such as $C_{1-6}$ alkyl$(O)_2SC_{1-6}$ alkyl-), sulfonamidoalkyl (e.g., $_2$HRN(O)$SC_{1-6}$ alkyl, H($C_{1-6}$ alkyl)N(O)$SC_{1-6}$ alkyl-).

As used herein, the term "alkyl", used either alone or in compound words denotes straight chain, branched or cyclic alkyl, for example $C_{1-40}$ alkyl, or $C_{1-20}$ or $C_{1-10}$. Examples of straight chain and branched alkyl include methyl, ethyl, n-propyl, isopropyl, *n*-butyl, *sec*-butyl, *t*-butyl, *n*-pentyl, 1,2-dimethylpropyl, 1,1-dimethyl-propyl, hexyl, 4-methylpentyl, 1-methylpentyl, 2-methylpentyl, 3-methylpentyl, 1,1-dimethylbutyl, 2,2-dimethylbutyl, 3,3-dimethylbutyl, 1,2-dimethylbutyl, 1,3-dimethylbutyl, 1,2,2-trimethylpropyl, 1,1,2-trimethylpropyl, heptyl, 5-methylhexyl, 1-methylhexyl, 2,2-dimethylpentyl, 3,3-dimethylpentyl, 4,4-dimethylpentyl, 1,2-dimethylpentyl, 1,3-dimethylpentyl, 1,4-dimethyl-pentyl, 1,2,3-trimethylbutyl, 1,1,2-trimethylbutyl, 1,1,3-trimethylbutyl, octyl, 6-methylheptyl, 1-methylheptyl, 1,1,3,3-tetramethylbutyl, nonyl, 1-, 2-, 3-, 4-, 5-, 6- or 7-methyloctyl, 1-, 2-, 3-, 4- or 5-ethylheptyl, 1-, 2- or 3-propylhexyl, decyl, 1-, 2-, 3-, 4-, 5-, 6-, 7- and 8-methylnonyl, 1-, 2-, 3-, 4-, 5- or 6-ethyloctyl, 1-, 2-, 3- or 4-propylheptyl, undecyl, 1-, 2-, 3-, 4-, 5-, 6-, 7-, 8- or 9-methyldecyl, 1-, 2-, 3-, 4-, 5-, 6- or 7-ethylnonyl, 1-, 2-, 3-, 4- or 5-propyloctyl, 1-, 2- or 3-butylheptyl, 1-pentylhexyl, dodecyl, 1-, 2-, 3-, 4-, 5-, 6-, 7-, 8-, 9- or 10-methylundecyl, 1-, 2-, 3-, 4-, 5-, 6-, 7- or 8-ethyldecyl, 1-, 2-, 3-, 4-, 5- or 6-propylnonyl, 1-, 2-, 3- or 4-butyloctyl, 1-2-pentylheptyl, tridecyl, tetradecyl, pentadecyl, hexadecyl, heptadecyl, octadecyl, nonoadecyl, eicosyl and the like. Examples of cyclic alkyl include mono- or polycyclic alkyl groups such as cyclopropyl, cyclobutyl, cyclopentyl, cyclohexyl, cycloheptyl, cyclooctyl, cyclononyl, cyclodecyl and the like. Where an alkyl group is referred to generally as "propyl", butyl" etc, it will be understood that this can refer to any of straight, branched and cyclic isomers where appropriate. An alkyl group may be optionally substituted by one or more optional substituents as herein defined.

As used herein, term "alkenyl" denotes groups formed from straight chain, branched or cyclic hydrocarbon residues containing at least one carbon to carbon double bond including ethylenically mono-, di- or polyunsaturated alkyl or cycloalkyl groups as previously defined, for example $C_{2-40}$ alkenyl, or $C_{2-20}$ or $C_{2-10}$. Thus, alkenyl is intended to include propenyl, butylenyl, pentenyl, hexaenyl, heptaenyl, octaenyl, nonaenyl, decenyl, undecenyl, dodecenyl, tridecenyl, tetradecenyl, pentadecenyl, hexadecenyl, heptadecenyl, octadecenyl, nondecenyl, eicosenyl hydrocarbon groups with one or more carbon to carbon double bonds. Examples of alkenyl include vinyl, allyl, 1-methylvinyl, butenyl, iso-butenyl, 3-methyl-2-butenyl, 1-pentenyl, cyclopentenyl, 1-methyl-cyclopentenyl, 1-hexenyl, 3-hexenyl, cyclohexenyl, 1-heptenyl, 3-heptenyl, 1-octenyl, cyclooctenyl, 1-nonenyl, 2-nonenyl, 3-nonenyl, 1-decenyl, 3-decenyl, 1,3-butadienyl, 1,4-pentadienyl, 1,3-cyclopentadienyl, 1,3-hexadienyl, 1,4-hexadienyl, 1,3-cyclohexadienyl, 1,4-cyclohexadienyl, 1,3-cycloheptadienyl, 1,3,5-cycloheptatrienyl and 1,3,5,7-cyclooctatetraenyl. An alkenyl group may be optionally substituted by one or more optional substituents as herein defined.

As used herein the term "alkynyl" denotes groups formed from straight chain, branched or cyclic hydrocarbon residues containing at least one carbon-carbon triple bond including ethylenically mono-, di- or polyunsaturated alkyl or cycloalkyl groups as previously defined, for example, $C_{2-40}$ alkenyl, or $C_{2-20}$ or $C_{2-10}$. Thus, alkynyl is intended to include propynyl, butylynyl, pentynyl, hexaynyl, heptaynyl, octaynyl, nonaynyl, decynyl, undecynyl, dodecynyl, tridecynyl, tetradecynyl, pentadecynyl, hexadecynyl, heptadecynyl, octadecynyl, nondecynyl, eicosynyl hydrocarbon groups with one or more carbon to carbon triple bonds. Examples of alkynyl include ethynyl, 1-propynyl, 2-propynyl, and butynyl isomers, and pentynyl isomers. An alkynyl group may be optionally substituted by one or more optional substituents as herein defined.

An alkenyl group may comprise a carbon to carbon triple bond and an alkynyl group may comprise a carbon to carbon double bond (i.e. so called ene-yne or yne-ene groups).

As used herein, the term "aryl" (or "carboaryl)" denotes any of single, polynuclear, conjugated and fused residues of aromatic hydrocarbon ring systems. Examples of aryl include phenyl, biphenyl, terphenyl, quaterphenyl, naphthyl, tetrahydronaphthyl, anthracenyl, dihydroanthracenyl, benzanthracenyl, dibenzanthracenyl, phenanthrenyl, fluorenyl, pyrenyl, idenyl, azulenyl, chrysenyl. Preferred aryl include phenyl and naphthyl. An aryl group may be optionally substituted by one or more optional substituents as herein defined.

As used herein, the terms "alkylene", "alkenylene", and "arylene" are intended to denote the divalent forms of "alkyl", "alkenyl", and "aryl", respectively, as herein defined.

The term "halogen" ("halo") denotes fluorine, chlorine, bromine or iodine (fluoro, chloro, bromo or iodo). Preferred halogens are chlorine, bromine or iodine.

The term "carbocyclyl" includes any of non-aromatic monocyclic, polycyclic, fused or conjugated hydrocarbon residues, preferably $C_{3-20}$ (e.g. $C_{3-10}$ or $C_{3-8}$). The rings may be saturated, e.g. cycloalkyl, or may possess one or more

double bonds (cycloalkenyl) and/or one or more triple bonds (cycloalkynyl). Particularly preferred carbocyclyl moieties are 5-6-membered or 9-10 membered ring systems. Suitable examples include cyclopropyl, cyclobutyl, cyclopentyl, cyclohexyl, cycloheptyl, cyclooctyl, cyclononyl, cyclodecyl, cyclopentenyl, cyclohexenyl, cyclooctenyl, cyclopentadienyl, cyclohexadienyl, cyclooctatetraenyl, indanyl, decalinyl and indenyl.

The term "heterocyclyl" when used alone or in compound words includes any of monocyclic, polycyclic, fused or conjugated hydrocarbon residues, preferably $C_{3-20}$ (e.g. $C_{3-10}$ or $C_{3-8}$) wherein one or more carbon atoms are replaced by a heteroatom so as to provide a non-aromatic residue. Suitable heteroatoms include O, N, S, P and Se, particularly O, N and S. Where two or more carbon atoms are replaced, this may be by two or more of the same heteroatom or by different heteroatoms. The heterocyclyl group may be saturated or partially unsaturated, i.e. possess one or more double bonds. Particularly preferred heterocyclyl are 5-6 and 9-10 membered heterocyclyl. Suitable examples of heterocyclyl groups may include azridinyl, oxiranyl, thiiranyl, azetidinyl, oxetanyl, thietanyl, 2H-pyrrolyl, pyrrolidinyl, pyrrolinyl, piperidyl, piperazinyl, morpholinyl, indolinyl, imidazolidinyl, imidazolinyl, pyrazolidinyl, thiomorpholinyl, dioxanyl, tetrahydrofuranyl, tetrahydropyranyl, tetrahydropyrrolyl, tetrahydrothiophenyl, pyrazolinyl, dioxalanyl, thiazolidinyl, isoxazolidinyl, dihydropyranyl, oxazinyl, thiazinyl, thiomorpholinyl, oxathianyl, dithianyl, trioxanyl, thiadiazinyl, dithiazinyl, trithianyl, azepinyl, oxepinyl, thiepinyl, indenyl, indanyl, 3H-indolyl, isoindolinyl, 4H-quinolazinyl, chromenyl, chromanyl, isochromanyl, pyranyl and dihydropyranyl.

The term "heteroaryl" includes any of monocyclic, polycyclic, fused or conjugated hydrocarbon residues, wherein one or more carbon atoms are replaced by a heteroatom so as to provide an aromatic residue. Preferred heteroaryl have 3-20 ring atoms, e.g. 3-10. Particularly preferred heteroaryl are 5-6 and 9-10 membered bicyclic ring systems. Suitable heteroatoms include, O, N, S, P and Se, particularly O, N and S. Where two or more carbon atoms are replaced, this may be by two or more of the same heteroatom or by different heteroatoms. Suitable examples of heteroaryl groups may include pyridyl, pyrrolyl, thienyl, imidazolyl, furanyl, benzothienyl, isobenzothienyl, benzofuranyl, isobenzofuranyl, indolyl, isoindolyl, pyrazolyl, pyrazinyl, pyrimidinyl, pyridazinyl, indolizinyl, quinolyl, isoquinolyl, phthalazinyl, 1,5-naphthyridinyl, quinozalinyl, quinazolinyl, quinolinyl, oxazolyl, thiazolyl, isothiazolyl, isoxazolyl, triazolyl, oxadialzolyl, oxatriazolyl, triazinyl, and furazanyl.

The term "acyl" either alone or in compound words denotes a group containing the agent C=O (and not being a carboxylic acid, ester or amide) Preferred acyl includes C(O)-$R^x$, wherein $R^x$ is hydrogen or an alkyl, alkenyl, alkynyl, aryl, heteroaryl, carbocyclyl, or heterocyclyl residue. Examples of acyl include formyl, straight chain or branched alkanoyl (e.g. $C_{1-20}$) such as, acetyl, propanoyl, butanoyl, 2-methylpropanoyl, pentanoyl, 2,2-dimethylpropanoyl, hexanoyl, heptanoyl, octanoyl, nonanoyl, decanoyl, undecanoyl, dodecanoyl, tridecanoyl, tetradecanoyl, pentadecanoyl, hexadecanoyl, heptadecanoyl, octadecanoyl, nonadecanoyl and icosanoyl; cycloalkylcarbonyl such as cyclopropylcarbonyl cyclobutylcarbonyl, cyclopentylcarbonyl and cyclohexylcarbonyl; aroyl such as benzoyl, toluoyl and naphthoyl; aralkanoyl such as phenylalkanoyl (e.g. phenylacetyl, phenylpropanoyl, phenylbutanoyl, phenylisobutylyl, phenylpentanoyl and phenylhexanoyl) and naphthylalkanoyl (e.g. naphthylacetyl, naphthylpropanoyl and naphthylbutanoyl]; aralkenoyl such as phenylalkenoyl (e.g. phenylpropenoyl, phenylbutenoyl, phenylmethacryloyl, phenylpentenoyl and phenylhexenoyl and naphthylalkenoyl (e.g. naphthylpropenoyl, naphthylbutenoyl and naphthylpentenoyl); aryloxyalkanoyl such as phenoxyacetyl and phenoxypropionyl; arylthiocarbamoyl such as phenylthiocarbamoyl; arylglyoxyloyl such as phenylglyoxyloyl and naphthylglyoxyloyl; arylsulfonyl such as phenylsulfonyl and napthylsulfonyl; heterocycliccarbonyl; heterocyclicalkanoyl such as thienylacetyl, thienylpropanoyl, thienylbutanoyl, thienylpentanoyl, thienylhexanoyl, thiazolylacetyl, thiadiazolylacetyl and tetrazolylacetyl; heterocyclicalkenoyl such as heterocyclicpropenoyl, heterocyclicbutenoyl, heterocyclicpentenoyl and heterocyclichexenoyl; and heterocyclicglyoxyloyl such as thiazolyglyoxyloyl and thienylglyoxyloyl. The $R^x$ residue may be optionally substituted as described herein.

The term "sulfoxide", either alone or in a compound word, refers to a group -S(O)$R^y$ wherein $R^y$ is selected from hydrogen, alkyl, alkenyl, alkynyl, aryl, heteroaryl, heterocyclyl, carbocyclyl, and aralkyl. Examples of preferred $R^y$ include $C_{1-20}$alkyl, phenyl and benzyl.

The term "sulfonyl", either alone or in a compound word, refers to a group $S(O)_2$-$R^y$, wherein $R^y$ is selected from hydrogen, alkyl, alkenyl, alkynyl, aryl, heteroaryl, heterocyclyl, carbocyclyl and aralkyl. Examples of preferred $R^y$ include $C_{1-20}$alkyl, phenyl and benzyl.

The term "sulfonamide", either alone or in a compound word, refers to a group S(O)N$R^y$$R^y$ wherein each $R^y$ is independently selected from hydrogen, alkyl, alkenyl, alkynyl, aryl, heteroaryl, heterocyclyl, carbocyclyl, and aralkyl. Examples of preferred $R^y$ include $C_{1-20}$alkyl, phenyl and benzyl. In a preferred embodiment at least one $R^y$ is hydrogen. In another form, both $R^y$ are hydrogen.

The term, "amino" is used here in its broadest sense as understood in the art and includes groups of the formula N$R^A$$R^B$ wherein $R^A$ and $R^B$ may be any independently selected from hydrogen, alkyl, alkenyl, alkynyl, aryl, carbocyclyl, heteroaryl, heterocyclyl, aralkyl, and acyl. $R^A$ and $R^B$, together with the nitrogen to which they are attached, may also form a monocyclic, or polycyclic ring system e.g. a 3-10 membered ring, particularly, 5-6 and 9-10 membered systems. Examples of "amino" include $NH_2$, NHalkyl (e.g. $C_{1-20}$alkyl), NHaryl (e.g. NHphenyl), NHaralkyl (e.g. NHbenzyl), NHacyl (e.g. NHC(O)$C_{1-20}$alkyl, NHC(O)phenyl), Nalkylalkyl (wherein each alkyl, for example $C_{1-20}$, may be the same or different)

and 5 or 6 membered rings, optionally containing one or more same or different heteroatoms (e.g. O, N and S).

The term "amido" is used here in its broadest sense as understood in the art and includes groups having the formula $C(O)NR^AR^B$, wherein $R^A$ and $R^B$ are as defined as above. Examples of amido include $C(O)NH_2$, C(O)NHalkyl (e.g. $C_{1-20}$alkyl), C(O)NHaryl (e.g. C(O)NHphenyl), C(O)NHaralkyl (e.g. C(O)NHbenzyl), C(O)NHacyl (e.g. C(O)NHC(O)$C_{1-20}$alkyl, C(O)NHC(O)phenyl), C(O)Nalkylalkyl (wherein each alkyl, for example $C_{1-20}$, may be the same or different) and 5 or 6 membered rings, optionally containing one or more same or different heteroatoms (e.g. O, N and S).

The term "carboxy ester" is used here in its broadest sense as understood in the art and includes groups having the formula $CO_2R^z$, wherein $R^z$ may be selected from groups including alkyl, alkenyl, alkynyl, aryl, carbocyclyl, heteroaryl, heterocyclyl, aralkyl, and acyl. Examples of carboxy ester include $CO_2C_{1-20}$alkyl, $CO_2$aryl (e.g.. $CO_2$phenyl), $CO_2$aralkyl (e.g. $CO_2$ benzyl).

The term "heteroatom" or "hetero" as used herein in its broadest sense refers to any atom other than a carbon atom which may be a member of a cyclic organic group. Particular examples of heteroatoms include nitrogen, oxygen, sulfur, phosphorous, boron, silicon, selenium and tellurium, more particularly nitrogen, oxygen and sulfur.

It is understood that the compounds of the present invention (including monomers and polymers) may exist in one or more stereoisomeric forms (eg enantiomers, diastereomers). The present invention includes within its scope all of these stereoisomeric forms either isolated (in for example enantiomeric isolation), or in combination (including racemic mixtures).

The invention will now be described with reference to the following non-limiting examples:

## EXAMPLES

### General

Proton NMR spectra were obtained on Bruker AV400 and Bruker AV200 spectrometers, operating at 400 MHz and 200 MHz respectively. All spectra were obtained at 23°C unless specified. Chemical shifts are reported in parts per million (ppm) on the $\delta$ scale and relative to the chloroform peak at 7.26 ppm (1H). Oven dried glassware was used in all reactions carried out under an inert atmosphere (either dry nitrogen or argon). All starting materials and reagents were obtained commercially unless otherwise stated. Removal of solvents "under reduced pressure" refers to the process of bulk solvent removal by rotary evaporation (low vacuum pump) followed by application of high vacuum pump (oil pump) for a minimum of 30 min. Analytical thin layer chromatography (TLC) was performed on plastic-backed Merck Kieselgel KG60F254 silica plates and visualised using short wave ultraviolet light, potassium permanganate or phosphomolybdate dip. Flash chromatography was performed using 230-400 mesh Merck Silica Gel 60 following established guidelines under positive pressure. Tetrahydrofuran and dichloromethane were obtained from a solvent dispensing system under an inert atmosphere. All other reagents and solvents were used as purchased.

Molecular weights of polymers were characterized by gel permeation chromatography (GPC) performed in tetrahydrofuran (THF) or dimethylformamide (DMF) 1.0 mL/min, 25°C using a Waters GPC instrument, with a Waters 2414 Refractive Index Detector, a series of four Polymer Laboratories PLGel columns (3 × 5 μm Mixed-C and 1×3 μm Mixed-E), and Empower Pro Software. The GPC was calibrated with narrow polydispersity polystyrene standards (Polymer Laboratories EasiCal, MW from 264 to 256000), and molecular weights are reported as polystyrene equivalents.

Acid number and hydroxy number were performed using the methods outlined below

### Acid Value Determination for Biodegradable Polymers

#### References

ASTM D1980-87 (vol 6.03). Standard Test Method for Acid Value of Fatty Acids and Polymerised Fatty Acids - superseded by ASTM D1980-87(1998) (vol 6.03).

Membranes of Polyurethanes Containing Crystalline Soft Segments: Oxygen Permeability and Morphology, Oh, H.-J.; Kim, W.-Y.; Jeong, Y.-S.; Lee, Y.-S., Bull. Korean Chem. Soc., 2001, 22(2), 194-8.

#### Method

This test method determines the total amount of residual acid groups present in the polymers and any free acid groups present, reported as acid value (mg KOH/g polymer).

#### Reagents

Chloroform and methanol, AR grade.

KOH pellets, AR grade.
Phenolphthalein indicator (1% in MeOH).
Potassium hydrogen phthalate ($KHC_8H_4O_4$), dry at 120 °C for 2 hr before use.

**NOTE: all glassware should be clean and oven dried before use**

Preparation and Standardisation of 0.1 M KOH solution (do in triplicate)
Dissolve 5.61 g of KOH in methanol to make a 1 L solution. (2/4/06) - dissolve 33g of KOH in carbon dioxide free water then filter. Add 71 mL water and 900 mL methanol Weigh out approximately but accurately, 0.74g of dried $KHC_8H_4O_4$ into a conical flask. Add 100 mL of carbon dioxide-free water and 3 drops of the phenolphthalein indicator and mix well. Titrate with the 0.1 M KOH solution.

$$M = \frac{m/204.23}{v}$$

M = molarity of methanolic KOH solution (mol/L)
m = mass of $KHC_8H_4O_4$ used (g)
204.23 = molecular weight of $KHC_8H_4O_4$ (g/mol)
v = titre (L)

Procedure (do in triplicate)

Weigh out approximately but accurately 0.5 g to 1 g of sample into a 100 mL round-bottomed flask.
Degas under high vacuum (≤ 0.5 mmHg) for 1 hr.
Dissolve the sample in 50 to 100 mL of neutralised chloroform*. It may be necessary to magnetically stir the mixture for a few minutes to achieve complete dissolution.

**[0295]** * Use the same volume of solvent for the triplicate set. Methanol may be used if the sample does not dissolve in chloroform although it is slightly more acidic. Neutralise the chloroform before dissolving the sample by adding 5 drops of the indicator solution and adding dilute KOH solution (1 part 0.1 M KOH solution to 10 parts MeOH) to the phenolphthalein endpoint. Do the same for the methanol but add 0.5 mL of the indicator instead of 5 drops as the colour change is more difficult to observe.

Add 0.5 mL of the phenolphthalein indicator solution to the sample mixture and titrate immediately with the 0.1 M KOH solution to the first pink colour that persists for 30 seconds.

Calculation

$$\text{(a) Acid Value (mg KOH/g polymer)} = \frac{VM \times 56.1}{W}$$

v = sample titre (mL)
M = molarity of the KOH solution (mol/L)
56.1 = molecular weight of KOH (g/mol)
W = weight of the sample used (g)

$$\text{(b) Molecular weights} = \frac{56.1 \times n \times 1000}{(OH + Acid)\ \text{values}}$$

n = functionality of polymer (2 for linear, 4 for star etc.)

**Hydroxyl Value Determination for Biodegradable Polymers**

References

N-Methylimidazole as a Catalyst for Acetylation of Hydroxyl Terminated Polymers, Dee, L. A., Biggers, G. L., Fiske, M. E., Anal. Chem., 1980, 52, 572-3.
ASTM D2849-69 (Sections 31 to 39). Standard Methods of Testing: Urethane Foam Polyol Raw Materials. Method A - Acetic Anhydride Pressure Bottle Section. - standard withdrawn in 1987, no replacement.
ASTM E200-97 (Sections 74 to 79). Standard Practice for Preparation, Standardisation and Storage of Standard and Reagent Solutions for Chemical Analysis - superseded by ASTM E200-97(2001)e1 (vol 15.05).
Hydroxy-Terminated Poly (e-Caprolactone-co-Valerolactone) Oligomers: Synthesis, Characterisation and Polyurethane Network Formation, Storey, R. F., Herring, K. R., Hoffman, D. C., J. Polymer Science (Part A: Polymer Chemistry), 1991, 29, 1759-77.

Method

This test method determines the total amount of hydroxyl end groups present in a polymer sample, reported as hydroxyl value (mg KOH/g polymer).

The OH groups are acetylated with acetic anhydride in 1,2-dichloroethane at 100 °C using N-methylimidazole as catalyst. The excess acetic anhydride is hydrolysed with water and the total acetic acid produced is back titrated with standard KOH solution. The hydroxyl content is calculated from the difference between the blank and sample titres.

If the samples contain free acids, these are also titrated with KOH during the back titration. Therefore if the samples contain significant amounts of acidity or alkalinity, it must be corrected by carrying out acidity or alkalinity determinations.

Reagents

Acetic anhydride (AA), AR grade.
1,2-Dichloroethane (DCE), distil (bp 83.4 °C, 760 mmHg) and store at room temperature. N-Methylimidazole (NMIM), dry over KOH overnight, distil under reduced pressure (bp 197-198 °C, 760 mmHg), store under $N_2$, keep refrigerated.
Chloroform and methanol, AR grade.
Thymol blue indicator (10% in MeOH).
0.5 M KOH in MeOH.
Benzoic acid, grind and dry at 80 °C for at least 2 hours before use.

**NOTE: All glassware should be clean and oven dried before use.**

Preparation and Standardisation of 0.5 M Methanolic KOH solution (do in triplicate) Dissolve 28.05 g of AR grade KOH in methanol to make a 1 L solution.TRY THIS - dissolve 66g KOH in 60 mL water, filter. 79 mL solution obtained. Add 200 mL water then 2.5L methanol

Accurately weigh out 0.735-0.745 g dry benzoic acid into a conical flask. Add 1 mL degassed water, 7 mL MeOH, 1 mL NMIM, 50 mL $CHCl_3$ and mix well.

Add 3 drops of the thymol blue indicator solution and titrate with the 0.5 M KOH solution to the endpoint (colour change from yellow to blue-violet).

$$M = \frac{m/122.12}{v}$$

M = molarity of methanolic KOH solution (mol/L)
m = mass of benzoic acid used (g)
122.12 = molecular weight of benzoic acid (g/mol)
v = titre (L)

Acetylation of Samples/Procedure

Prepare a stock solution (SS) of AA in DCE (1:6 ratio by volume) and keep in a dark bottle. This must be

prepared fresh each time the procedure is carried out.

Samples (do in triplicate):

Place 3 - 4 milliequivalents of polymer* into a 250 mL round-bottomed flask.
Degas the sample at high pressure (≤ 0.5 mmHg) for 1 hr.
Add 20 mL of DCE, followed by 4 mL of the SS and 4 mL of NMIM.
Attach a condenser and drying tube and heat the mixture in a 110 °C oil bath (which has been turned on for at least 1 hr prior), with stirring for 15 minutes.
Cool the mixture in an ice bath, then add 3 mL of distilled water to hydrolyse the excess acetic anhydride and heat the mixture again as above for 5 minutes.
Cool the mixture in an ice bath and then rinse the condenser down with 160 mL chloroform and 35 mL methanol into the reaction vessel.
Add 5 drops of thymol blue indicator and titrate with the 0.5 M KOH solution to the final endpoint (colour change from orange to blue-green to bright blue).

*Weight (W) of sample required for hydroxyl number determination

$$= \frac{3 \times 10^{-3}\,\text{mol} \times \text{Mn}}{n}$$

Mn = molecular weight of sample obtained from GPC trace
n = OH functionality of polymer (2 for dihydroxy, 4 for tetrahydroxy etc.)

Blank (do once per stock solution):

As above, omitting steps 1 - 2. The blank mixture should turn a green-yellow colour during step 4.

Calculations

$$\text{Hydroxyl number (mg KOH/g polymer)} = \frac{[(B - A)M \times 56.1]}{W}$$

A = sample titre (mL)
B = blank titre (mL)
M = molarity of the KOH solution (mol/L)
W = weight of sample used (g)

$$\text{Molecular weights} = \frac{56.1 \times n \times 1000}{(\text{OH} + \text{Acid})\ \text{values}}$$

n = functionality of polymer (2 for linear, 4 for star, etc.)

**Valproic Acid (2-propylpentanoic acid)**

Example 1: Valproic Acid Monoglyceride (VA-MG) - *For comparative Examples*

(a) 2-propylpentanoic acid, 2, 2-dimethyl-1,3-dioxolane-4-yl methyl ester

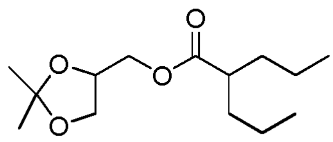

2-propylpentanoic acid (10.00 g; 0.07 mol) was dissolved in dichloromethane (anhydrous) (500 ml), under an argon atmosphere. 2,2-dimethyl-1,3-dioxolane-4-methanol (solketal) (11.00 g; 0.08 mol; 1.2 equiv) and p-dimethylaminopyridine (1.02 g; 8.30 mmol; 0.12 equiv) were added. The solution was cooled to 0°C and a solution of 1,3-dicyclohexylcarbodiimide (17.17 g; 0.08 mol; 1.20 equiv) in dichloromethane (anhydrous) (100 ml) was added dropwise over 30 minutes. A white precipitate formed during the addition. The reaction mixture was allowed to warm to ambient temperature and stirred at ambient temperature for 16 hours. The reaction mixture was cooled in a dry ice/acetone bath (-78°C) followed by filtration to remove the urea precipitate. The solvent was removed in vacuo to obtain the crude product as a clear, colourless oil (23.29 g) which was purified by column chromatography (silica gel, 5% ethyl acetate/petroleum ether 40-60) to obtain a colourless oil (14.40 g, 80 %).
$^1$H-NMR ($CDCl_3$, 200 MHz): δ[ppm] = 4.30 (quintet, 1H, J=5.8Hz); 4.03-4.17 (m, 3H); 3.74 (dd, 1H, J=6.1Hz, J=8.3Hz); 2.34-2.47 (m, 1H); 1.19-1.69 (m,14H); 0.89 (t, 6H, J=7.1Hz)

(b) 2, 3-dihydroxypropyl 2-propylpentanoate (VA-MG)

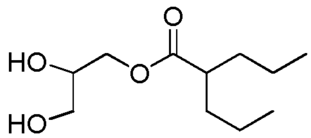

2-propylpentanoic acid 2,2-dimethyl-1,3-dioxolane-4-yl methyl ester was dissolved in 95% (v/v) aqueous ethanol (200 ml). Amberlyst 15 (wet) ion exchange resin (sulfonic acid) (6.40 g) and antibumping granules were added. The reaction mixture was refluxed for 5 hours without stirring. The reaction mixture was cooled to ambient temperature, followed by filtration and removal of the solvent in vacuo to obtain a pale brown oil (12.89 g) which was purified by column chromatography (silica gel, 50% ethyl acetate/petroleum ether 40-60) to obtain a colourless oil (10.40 g, 86%).
$^1$H-NMR ($CDCl_3$, 200 MHz): δ[ppm] = 4.27-4.11 (m, 2H); 3.80-4.01 (m, 1H); 3.52-3.76 (m, 2H); 2.52 (d, 1H, J=5.0Hz); 2.34-2.49 (m, 1H); 2.09 (t, 1H, J=6.0Hz); 1.20-1.70 (m, 8H); 0.90 (t, 6H, J=6.9Hz)

Example 2: 3-(1,3-dihydroxypropan-2-yloxy)-3-oxopropyl 2-propylpentanoate

a) 3-hydroxypropyl 2-propylpentanoate

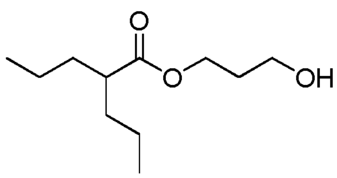

2-propylpentanoic acid (10.0 g, 69.3 mmol) was mixed with propane-1-3-diol (42.1 g, 555.0 mmol) and methylsulfonic acid (5 drops) in toluene (200 ml) and the mixture was heated under reflux using a Dean Stark trap for 12 h. After that, toluene was removed under reduced pressure and the crude mixture was dissolved in dichlormethane (200 ml) and extracted with water (pH 5,3 x 200 ml). The combined organic layers were dried over sodium sulfate and the volatiles were removed under reduced pressure giving a clear oil (14.0 g, 60.0 mmol, 99%).
$^1$H-NMR ($CDCl_3$, 200 MHz): δ[ppm] = 4.24 (tr, 2H, J = 6.1 Hz), 3.68 (tr, 2H, J = 6.1 Hz), 2.37-2.31 (m, 1H), 1.93-1.77 (m, 2H), 1.69-1.16 (m, 8H), 0.89 (tr, 6H, 6.9 Hz)

b) 3-(2-propylpentanoyloxy)propanoic acid

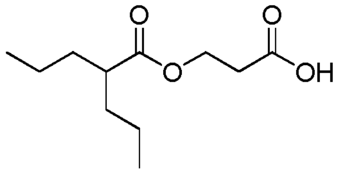

3-hydroxypropyl 2-propylpentanoate (2.08 g, 10.3 mmol) was dissolved in 120 ml of acetone. 3.1 ml of a solution of Jones Reagent (prepared by dissolving 26.72 g of chromium trioxide in 23 ml of concentrated sulfuric acid, and then diluting the mixture to 100 ml with water) was added. Propan-2-ol (5 ml) was added to the reaction mixture which was filtered through a pad of celite. The filtrate was washed with 0.01 M HCl solution (3 x 50 ml), dried over sodium sulfate, filtered and concentrated under reduced pressure. The crude product (2.05 g, 9.5 mmol, 92 %)was used in the next step without any further purification.
$^1$H-NMR ($CDCl_3$, 200 MHz): δ[ppm] = 4.35 (tr, 2H, J = 6.3 Hz), 2.70 (tr, 2H, J = 6.3 Hz), 2.47-2.28 (m, 1H), 1.70-1.15 (m, 8H), 0.88 (tr, 6H, J = 6.9 Hz)

c) 3-oxo-3-(2-phenyl-1,3-dioxan-5-yloxy)propyl 2-propylpentanoate

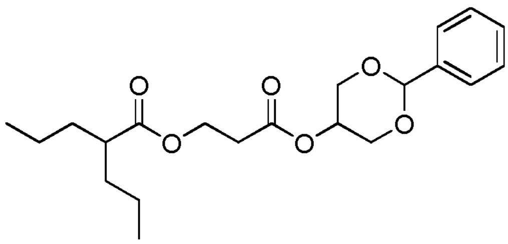

3-(2-propylpentanoyloxy)propanoic acid (2.05 g, 9.5 mmol) was dissolved in anhydrous $CH_2Cl_2$ (200 ml) under argon. 1,3-O-benzylideneglycerol (2.50 g, 13.67 mmol triethylamine (4.04 g, 40.0 mmol) and O-benzotriazol-1-yl-N, N, N', N'-trimethyluronium hexafluorophosphate (4.2 g, 11.0 mmol) were added successively. The reaction mixture was stirred under argon for 72 h. The reaction mixture was washed with saturated $NaHCO_3$ solution (1 x 90 ml), water (1 x 90 ml) and dried over $Na_2SO_4$. The organic solvent was removed under reduced pressure giving a pale, pink solid (2.99 g, 7.9 mmol, 83 %).
$^1$H-NMR ($CDCl_3$, 200 MHz): δ[ppm] = 7.63-7.29 (m, 5H), 5.55 (m, 5H), 4.75-4.66 (m, 1H), 4.42-4.06 (m, 4H), 3.01-2.78 (m, 2H), 2.74-2.24 (m, 3H), 2.07-1.18 (m, 8H), 0.92 (tr, 6H, J = 6.9 Hz)

d) 3-(1,3-dihydroxypropan-2-yloxy)-3-oxopropyl 2-propylpentanoate

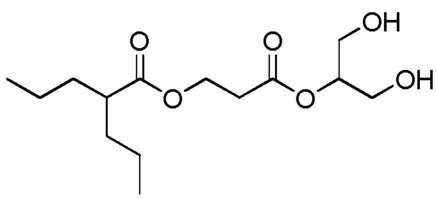

3-oxo-3-(2-phenyl-1,3-dioxan-5-yloxy)propyl 2-propylpentanoate (2.99 g, 7.9 mmol) was dissolved in ethanol (90 ml) under argon. Palladium catalyst (10 wt % Pd/C) (300 mg) was added, the flask was evacuated and allowed to stir 1 atm of hydrogen gas at room temperature for 16 hours. The crude reaction mixture was passed through a glass micro fiber on a sintered funnel. The volatiles were removed under reduced pressure yielding 2.01 g (6.95 mmol, 88%)

of a clear oil. The product was used without any further purification
$^1$H-NMR ($CDCl_3$, 400 MHz): δ[ppm] = 4.99-4.90 (m, 1H), 3.86-3.80 (m, 4H), 2.80-2.70 (m, 2H), 2.48-2.37 (m, 2H), 1.89-1.80 (m, 2H), 1.68-1.54 (m, 2H), 1.50-1.22 (m, 6H), 0.91 (tr, 6H, J = 7.2 Hz)

Example 3: Production of a mixture of 1,2-dihydroxy-5,14-dioxo-4,15-dioxa-6,13-diazanonadecan-19-yl 2-propylpentanoate and 1-hydroxy-2-(hydroxymethyl)-4,13-dioxo-3,14-dioxa-5,12-diazaoctadecan-18-yl 2-propylpentanoate

a) 4-hydroxybutyl 2-propylpentanoate

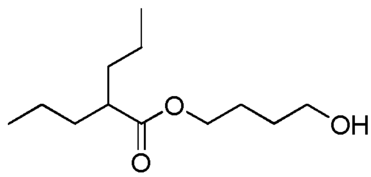

2-propylpentanoic acid (40.16 g, 278.5 mmol) was mixed with butane-1-4-diol (114.65 g, 1272.2 mmol) and methyl-sulfonic acid (5 drops) in toluene (3500 ml) and the mixture was heated under reflux using a Dean Stark trap for 12 h. After that, toluene was removed under reduced pressure and the crude mixture was dissolved in chloroform (200 ml) and extracted with water (pH 5, 3 x 200 ml). The combined organic layers were dried over sodium sulfate and the volatiles were removed under reduced pressure giving a clear oil (99%).

The product was analysed by NMR and found to be VA-BDO

b) 4-(6-isocyanatohexylcarbamoyloxy)butyl 2-propylpentanoate

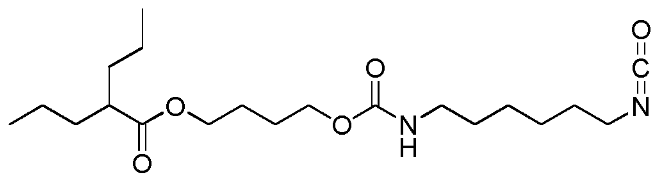

Solution A: 4-hydroxybutyl 2-propylpentanoate (VA-BDO) (10.4518g 48.3) mmol) was dried under vacuum and placed in a dried flask fitted with a magnetic stir bar and closed with a subaseal. The flask was also fitted with a dry nitrogen gas purge line. Approximately 100 mL of chloroform which had been previously dried over molecular sieve was introduced to the flask.

Solution B: hexamethylene diisocyanate (HDI) (7.9001g 47.0 mmol) which had been distilled under vacuum was placed in a round bottom flask fitted with a magnetic stir bar and closed with a subaseal. The flask was also fitted with a dry nitrogen gas purge line. Approximately 120 mL of chloroform which had been previously dried over molecular sieve was introduced to the flask. Additionally 5 drops of Tin 2-ethyl hexanoate was added to the flask as a catalyst.

The content of Solution A was slowly added over a 15 minute period to Solution B with stirring.

The mixture was stirred overnight at room temperature under dry nitrogen. The reaction mixture / solvent = Solution C

A 10 mL subsample was removed and the chloroform removed by under vacuum by rotovap. The compound was analysed by NMR in CDCl3 and found to contain a high proportion of 4-(6-isocyanatohexylcarbamoyloxy)butyl 2-propylpentanoate (VA-BDO-HDI.)

c) Mixture of 1,2-dihydroxy-5,14-dioxo-4,15-dioxa-6,13-diazanonadecan-19-yl 2-propylpentanoate and 1-hydroxy-2-(hydroxymethyl)-4,13-dioxo-3,14-dioxa-5,12-diazaoctadecan-18-yl 2-propylpentanoate

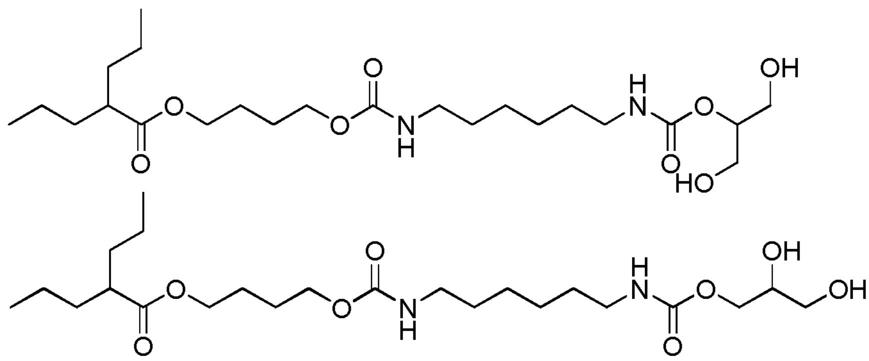

The remaining amount if Solution C from was added slowly over 15 mins to Solution D - which consisted of glycerol (4.4499g 48.3 mmol) which was dried overnight at 110C under vacuum) and 100 mL dried chloroform.

The amount of glycerol in solution D was adjusted to give a 1:1 mole ratio of VA-BDO-HDI to Glycerol, with a slight excess of glycerol.

The solution was stirred for 24 hours at 90C under a nitrogen purge. At various timepoints 4 Ml samples were removed and the chloroform was removed by rotovap and the products were analysed by NMR.

Once high conversion was confirmed by NMR the solution was cooled, more chloroform added to make up to 200mL and and extracted with water (pH 5, 3 x 200 ml). The combined organic layers were dried over sodium sulfate and the volatiles were removed under reduced pressure giving a white solid

A subsample was dissolved in d-DMSO for analysis by NMR. The sample appears to be a mixture of SN1 and SN2 coupling of the VA-BDO-HDI to Glycerol.

The product was dried and stored under nitrogen for use in the production of polymers.

**Ciprofloxacin (1-cyclopropyl-6-fluoro-4-oxo-7-[4'-N-[(tert-butyloxy) carbonyl] piperazin] - 1-yl-quinoline-3-carboxylic acid)**

Example 4:. Ciprofloxacin Monoglyceride (2,3-dihydroxypropyl 1-cyclopropyl-6-fluoro-4-oxo-7-(piperazin-1-yl)-1,4-dihydroquinoline-3-carboxylate)

a) 7-(4-(benzyloxycarbonyl)piperazin-1-yl)-1-cyclopropyl-6-fluoro-4-oxo-1,4-dihydroquinoline-3-carboxylic acid

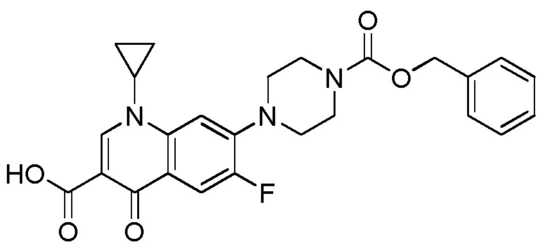

Ciprofloxacin (1-cyclopropyl-6-fluoro-4-oxo-7-piperazin-1-yl)-1,4-quinoline-3-carboxylic acid (0.33 g, 1.0 mmol), was taken up in 2N NaOH solution (10 ml) and cooled to 0°C (ice/water bath). Benzyloxycarbonyl chloride (0.40 ml, 0.48 g, 2.8 mmol) was added dropwise. The reaction mixture was stirred at 0°C for 1 h and then gradually warmed to room temperature and stirred for another 16 h. After that the reaction mixture was brought to pH 5 through dropwise addition of 2N HCl solution. The reaction mixture was extracted with $CHCl_3$ (3 x 50 ml). The combined organic layers were dried over $Na_2SO_4$ and the solvent was removed under reduced pressure. The crude product was purified through crystallisation from acetonitrile to give a colourless solid (0.23 g, 0.49 mmol, 49 %)
$^1$H-NMR ($CDCl_3$, 400 MHz): δ[ppm] = 8.78 (s, 1H, H-2); 8.10-8.00 (m, 1H, H-5); 7.44-7.28 (m, 6H, 1H of H-8+5H of ArH); 5.17 (s, 2H, $ArCH_2$); 3.81-3.63 (m, 4H, $2xCH_2$ of piperazine); 3.56-3.46 (m, 1H of cyclopropane); 3.38-3.23 (m, 4H, $2xCH_2$ of piperazine); 1.44-1.33 (m, 2H, $CH_2$ of cyclopropane); 1.24-1.13 (m, 2H, $CH_2$ of cyclopropane)

b) (2,2-dimethyl-1,3-dioxolan-4-yl)methyl 7-(4-(benzyloxycarbonyl)piperazin-1-yl)-1-cyclopropyl-6-fluoro-4-oxo-1,4-dihydroquinoline-3-carboxylate

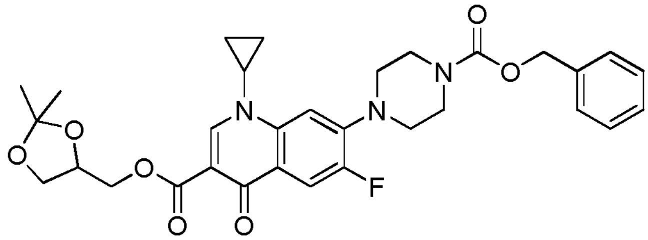

7-(4-(benzyloxycarbonyl)piperazin-1-yl)-1-cyclopropyl-6-fluoro-4-oxo-1,4-dihydroquinoline-3-carboxylic acid (0.47 g, 1.0 mmol) was dissolved in anhydrous $CH_2Cl_2$ (47 ml) under argon. 2,2-dimethyl-1,3-dioxolane-4-methanol (0.20 g, 1.5 mmol) triethylamine (0.40 g, 4.0 mmol) and O-benzotriazol-1-yl-N,N,N',N'-trimethyluronium hexafluorophosphate (0.42 g, 1.1 mmol) were added successively. The reaction mixture was stirred under argon for 72 h. The reaction mixture was washed with saturated $NaHCO_3$ solution (1 x 50 ml), water (1 x 50 ml) and dried over $Na_2SO_4$. The organic solvent was removed under reduced pressuregiving a pale, pink solid (0.54 g, 0.93 mmol, 93 %).
$^1$H-NMR ($CDCl_3$, 400 MHz): δ[ppm] = 8.56 (s, 1H, H-2); 8.10-7.97 (m, 1H, H-5); 7.42-7.13 (m, 6H, 5xArH+1H of H-8); 5.16 (s, 2H, $ArCH_2O$); 4.52-4.40 (m, 1H, CH); 4.40-4.30 (m, 2H, 1H of $CH_2O$+1H of $CH_2O$); 4.08-3.96 (m, 1H, 1H of $CH_2O$); 3.86-3.81 (m, 1H, 1H of $CH_2O$); 3.62-3.51 (m, 4H, 2x$CH_2$ of piperazine); 3.37-3.33 (m, 1H, CH of cyclopropane); 3.31-3.14 (m, 4H, 2x$CH_2$ of piperazine); 1.44 (s, 3H, $CH_3$); 1.36 (s, 3H, $CH_3$); 1.34-1.26 (m, $CH_2$ of cyclopropane); 1.26-1.03 (m, $CH_2$ of cyclopropane)
MS (MeCN) 580 [M+ 1] 602 [M+23]

c) 2,3-dihydroxypropyl 7-(4-(benzyloxycarbonyl)piperazin-1-yl)-1-cyclopropyl-6-fluoro-4-oxo-1,4-dihydroquinoline-3-carboxylate

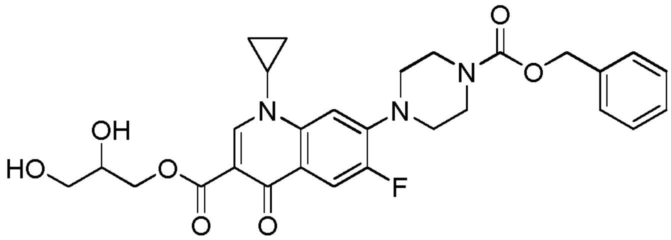

(2,2-dimethyl-1,3-dioxolan-4-yl)methyl 7-(4-(benzyloxycarbonyl)piperazin-1-yl)-1-cyclopropyl-6-fluoro-4-oxo-1,4-dihydroquinoline-3-carboxylate (2.85 g, 4.9 mmol) was dissolved in 95 % aqueous ehanol (100 ml). After addition of amberlyst 15 (wet) ion exchange resion and anti bumping granulates the reaction mixture was refluxed for 5 h. The ion exchange resion and the bumping-granulates were removed through filtration and the solvent was removed under reduced pressure to give the crude product as a yellow solid. The crude product was purified through column chromatography ($Al_2O_3$ 1% MeOH in $CHCl_3$) to yield the pure product as a pale yellow solid (1.66 g, 63 %).
$^1$H-NMR ($CDCl_3$, 400 MHz): δ[ppm] = 8.52 (s, 1H, H-2); 7.97-7.77 (m, 1H, H-5); 7.67-7.51 (m, 1H, H-8); 7.51-7.26 (m, 5H, ArH); 5.17 (s, 2H, $ArCH_2$); 4.52-4.16 (m, 2H, 1H of $CH_2O$+1H of $CH_2O$); 3.97-3.82 (m, 1H, CHO); 3.82-3.43 (m, 6H, 4H of 2x$CH_2$ piperazine+1H of $CH_2O$+1H of CH cyclopropane); 3.19-2.98 (m, 4H, 2x$CH_2$ piperazine); 1.53-1.00 (m, 4H, 2x$CH_2$ cyclopropane)
MS (MeCN) 540 [M+ 1] 562 [M+23]

d) 2,3-dihydroxypropyl 1-cyclopropyl-6-fluoro-4-oxo-7-(piperazin-1-yl)-1,4-dihydroquinoline-3-carboxylate

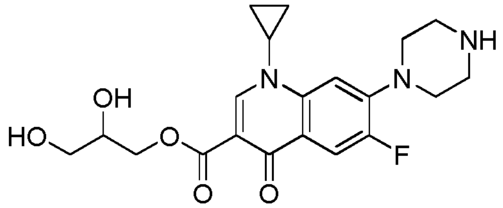

2,3-dihydroxypropyl 7-(4-(benzyloxycarbonyl)piperazin-1-yl)-1-cyclopropyl-6-fluoro-4-oxo-1,4-dihydroquinoline-3-carboxylate (1.66 g, 3.1 mmol) was dissolved in ethanol (100 ml). Hydrogenation in a Thales Nano H-Cube Hydrogenator (10 % Pd/C cartridge, 11 bar) and evaporation of the organic solvent gave the crude product as a yellow solid. Crystallisation from acetone gave the crude product as a colourless solid (0.71 g, 57 %).
$^1$H-NMR ($d_6$-acetone), 500 MHz): δ[ppm] = 8.72 (s, 1H, H-2); 7.91-7.80 (m, 1H, H-5); 7.55-7.43 (m, 1H, H-8); 4.42-4.20 (m, 2H, 1H of $CH_2O$+1H of $CH_2O$); 4.00-3.90 (m, 1H, CH); 3.82-3.75 (m, 1H, 1H of $CH_2O$); 3.70-3.5 (m, 6H, 4H of 2x$CH_2$ piperazine+1H of $CH_2O$+1H CH of cyclopropane); 3.15-3.02 (m, 4H, 2x$CH_2$ of piperazine); 1.48-1.30 (m, 2H, $CH_2$ of cyclopropane); 1.25-1.10 (m, 2H, $CH_2$ of cyclopropane
MS (MeCN) 406 [M+ 1] 428 [M+23]

Example 6: 2,3-dihydroxypropyl 1-cyclopropyl-6-fluoro-4-oxo-7-(4-propylpiperazin-1-yl)-1,4-dihydroquinoline-3-carboxylate

a) 1-cyclopropyl-6-fluoro-4-oxo-7-(4-propylpiperazin-1-yl)-1,4-dihydroquinoline-3-carboxylic acid

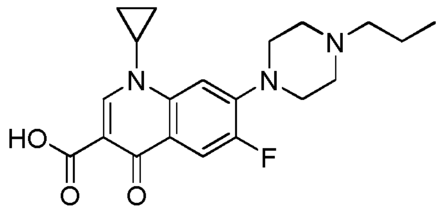

1-cyclopropyl-6-fluoro-4-oxo-7-(piperazin-1-yl)-1 ,4-dihydroquinoline-3-carboxylic acid (5.00 g, 15.10 mmol) was taken up in dioxane/water (1:1, 100 ml). 1-iodopropane (3.08 g, 18.10 mmol), and sodiumhydrogencarbonate (3.81 g, 45.30 mmol) was added and heated at 80 °C for 16 h. The crude reaction mixture was cooled to room temperature and acidified with 1M HCl to pH 6. The reaction mixture was extracted with chloroform (3 x 100 ml), the combined organic layer were dried over sodium sulfate and the volatiles were removed under reduced pressure yielding 3.87 g (69 %) of pure product.
$^1$H-NMR ($CDCl_3$, 400 MHz): δ[ppm] = 8.77 (s, 1H, H-2); 8.07-7.97 (m, 1H, H-5); 7.41-7.30 (m, 1H, H-8); 3.57-3.46 (m, CH of cyclopropane ring); 3.41-3.24 (m, 4H, 2x$CH_2$ of piperazine); 2.74-2.58 (m, 2x$CH_2$ of piperazine); 2.44-2.33 (m, 2H, $CH_2N$); 1.65-1.45 (m, 2H, $CH_2$); 1.45-1.28 (m, 2H, $CH_2$ of cyclopropane ring); 1.28-1.12 (m, 2H, $CH_2$ of cyclopropane ring); 0.94 (t, 3H, $CH_3$)
MS ($CH_2Cl_2$) 374 [M+ 1] 747 [2M+1]

b) (2,2-dimethyl-1,3-dioxolan-4-yl)methyl 1-cyclopropyl-6-fluoro-4-oxo-7-(4-propylpiperazin-1-yl)-1,4-dihydroquinoline-3-carboxylate

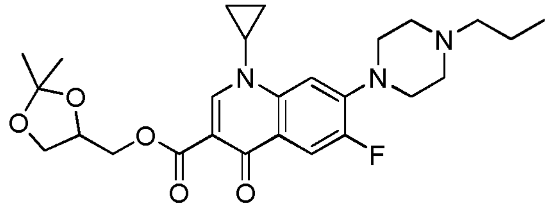

1-cyclopropyl-6-fluoro-4-oxo-7-(4-propylpiperazin-1-yl)-1,4-dihydroquinoline-3-carboxylic acid (8.86 g, 23.70 mmol) was dissolved in anhydrous dichloromethane (370 ml) under argon. 2,2-dimethyl-1,3-dioxolane-4-methanol (4.71 g, 35.60 mmol), triethylamine (9.59 g, 94.80 mmol) and HBTU (9.90 g, 26.10 mmol) were added and the reaction mixture was stirred at room temperature for three days (exclusion of light). The reaction mixture was washed with saturated $NaHCO_3$ solution (500 ml), aqueous (pH 5) hydrochloric acid (500 ml) and water (500 ml) successively. The organic layer was dried over $Na_2SO_4$ and the solvent was removed under reduced pressure, yielding 10.28 g (89 %) of a yellow solid.
$^1$H-NMR ($CDCl_3$, 400 MHz): δ[ppm] = 8.53 (s, 1H, H-2); 8.09-7.95 (m,1H, H-5); 7.32-7.17 (m, H-8); 4.51-4.41 (m, 1H, CH); 4.41-4.31 (m, 2H, 1H of $CH_2O$+ 1H of $CH_2O$); 4.19-4.09 (m, 1H, $CH_2O$); 3.98-3.86 (m, 1H, $CH_2O$); 3.50-3.36 (m, 1H of cyclopropane ring); 3.36-3.15 (m, 4H, 2x$CH_2$ of piperazine); 2.76-2.61 (m, 4H, 2x$CH_2$ of piperazine); 2.49-2.31 (m, 2H, $CH_2N$); 1.72-1.48 (m, 2H, $CH_2CH_2CH_3$); 1.44 (s, 3H, $CH_3$), 1.36 (s, 3H, $CH_3$); 1.33-1.25 (m, 2H, $CH_2$ of cyclopropane); 1.16-1.03 (m, 2H, $CH_2$ of cyclopropane); 0.93 (t, 3H, $CH_3$)

c) 2,3-dihydroxypropyl 1-cyclopropyl-6-fluoro-4-oxo-7-(4-propylpiperazin-1-yl)-1,4-dihydroquinoline-3-carboxylate

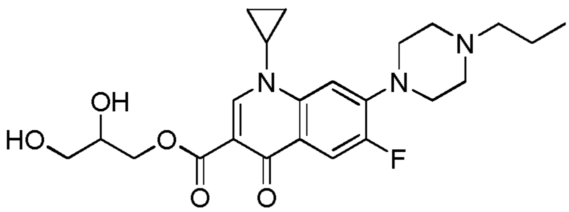

(2,2-dimethyl-1,3-dioxolan-4-yl)methyl 1-cyclopropyl-6-fluoro-4-oxo-7-(4-propylpiperazin-1-yl)-1,4-dihydroquinoline-3-carboxylate (2.45 g, 5.03 mmol) was dissolved in anhydrous dichloromethane (245 ml) under argon. A 1 M solution of boron trichloride in dichloromethane (6.3 ml, 6.30 mmol) was added dropwise and the reaction mixture was stirred at room temperature for 1 h. Methanol (25 ml) was added to the reaction mixture and the volatiles were removed under reduced pressure. The crude product was purified through flash column chromatography (5% MeOH in $CHCl_3$) giving a yellow oil (1.58 g, 3.53 mmol, 70 %).
MS (MeOH): 448.3 [M+1]

**Levofloxacin (9-fluoro-3-methyl-10-(4-methylpiperazin-1-yl)-7-oxo-3,7-dihydro-2H-[1,4]oxazino[2,3,4-ij]quinoline-6-carboxylic acid**

Example 7: 2,3-dihydroxypropyl 9-fluoro-3-methyl-10-(-4-methylpiperazin-1-yl)-7-oxo-3,7-dihydro-2H-[1,4]oxazino[2,3,4-ij]quinoline-6-carboxylate

a) (2,2-dimethyl-1,3-dioxolan-4-yl)methyl 9-fluoro-3-methyl-10-(4-methylpipirazin-1-yl)-7-oxo-3,7-dihydro-2H-[1,4]oxazino[2,3,4-ij]quinoline-6-carboxylate

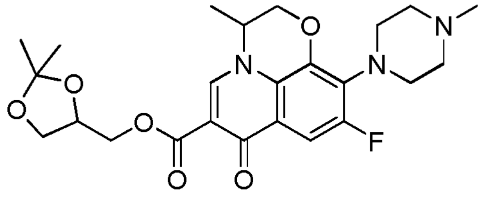

(9-fluoro-3-methyl-10-(4-methylpiperazin-1-yl)-7-oxo-3,7-dihydro-2H-[1,4]oxazino[2,3,4-ij]quinoline-6-carboxylic acid (10.00 g, 27.7 mmol), was dissolved in $CH_2Cl_2$ (500 ml) under argon. 2,2-dimethyl-1,3-dioxolane-4-methanol (5.49 g, 41.5 mmol) triethylamine (11.21 g, 110.8 mmol) and O-benzotriazol-1-yl-N,N, N', N'-trimethyluronium hexafluorophosphate (11.56 g, 30.5 mmol) were added successively. The reaction mixture was stirred under argon for 72 h. The reaction mixture was washed with saturated $NaHCO_3$ solution (1 x 500 ml), water (1 x 50 ml) and dried over $Na_2SO_4$. The organic solvent was removed under reduced pressuregiving a white solid. The crude product was crystallized from

acetonitrile giving the product in 44 % yield (5.81 g).

$^1H$-NMR ($CDCl_3$, 400 MHz): δ[ppm] = 8.29 (s, 1H, H-5); 7.65 (m, 1H, H-8); 4.53-4.41 (m, 1H, CH); 4.41-4.25 (m, 5H, 1H of $CH_2O$, 1H of $CH_2O$, 2H of $CH_2O$, 1H of CHN); 4.21-4.09 (m, 1H, 1H of $CH_2O$); 3.96-3.85 (m, 1H of $CH_2O$); 3.43-3.23 (m, 4H, 2x$CH_2$ piperazine); 2.65-2.45 (m, 4H 2x$CH_2$ piperazine); 2.36 (s, 3H, $NCH_3$); 1.58 (d, 3H, $CH_3$); 1.44 (s, 3H, $CH_3$), 1.37 (s, 3H, $CH_3$).

MS (EtOH) 475 [M+1].

b) 4-(6-((2,3-dihydroxypropoxy)carbonyl)-9-fluoro-3-methyl-7-oxo-3,7-dihydro-2H-[1,4]oxazino[2,3,4-ij]quinolin-10-yl)-1-methylpiperazin-1-ium 2,2,2-trifluoroacetate

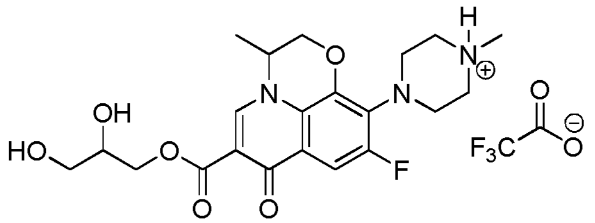

(2,2-dimethyl-1,3-dioxolan-4-yl)methyl 9-fluoro-3-methyl-10-(4-methylpipirazin-1-yl)-7-oxo-3,7-dihydro-2H-[1,4]oxazino[2,3,4-ij]quinoline-6-carboxylate (1.00 g, 2.1 mmol), was dissolved in $CH_2Cl_2$ (42 ml) under argon and cooled to 0°C. Trifluoro acetic acid (2.00 ml, 2.96 g, 26.0 mmol) was added and the reaction mixture was allowed to stir at 0°C for 4 h and then over night at room temperature. The reaction mixture was flushed through a plug of $Al_2O_3$ (10% MeOH in $CHCl_3$) (2x). After that the organic solvents were collected and removed under reduced pressure iving the product as a yellow gum (0.24 g, 104 %).

$^1H$-NMR ($CDCl_3$, 400 MHz): δ[ppm] = 8.76 (s, H-5); 7.63-7.50 (m, H-8); 4.74-4.61 (m, 1H, H-3 of benzoxazine ring); 4.61-4.50 (m, 1H, $NHCHCH_2O$); 4.50-4.33 (m, 2H, 1H of $NHCHCH_2O$+1H of $CH_2OCO$); 4.33-4.24 (m, 1H, $CH_2OCO$); 4.03-3.93 (m, 1H, CHOH); 3.73-3.53 (m, 6H, 2x$CH_2$ piperazine +$CH_2OH$); 3.43-3.22 (m, 4H, 2x$CH_2$ piperazine); 3.01 (s, 3H, +$NHCH_3$); 1.55 (d, 3H, $CH_3$).

MS (MeOH) 436 [M+1]; 458 [M+23]

c) 2,3-dihydroxypropyl 9-fluoro-3-methyl-10-(-4-methylpiperazin-1-yl)-7-oxo-3,7-dihydro-2H-[1,4]oxazino[2,3,4-ij]quinoline-6-carboxylate

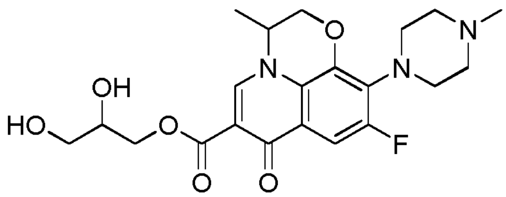

4-(6-((2,3-dihydroxypropoxy)carbonyl)-9-fluoro-3-methyl-7-oxo-3,7-dihydro-2H-[1,4]oxazino[2,3,4-ij]quinolin-10-yl)-1-methylpiperazin-1-ium 2,2,2-trifluoroacetate (0.50 g, 0.91 mmol) was dissolved in methanol under argon and cooled to 0°C (ice/salt bath). 1 equiv. of NaOH dissolved in methanol (25 ml) was added slowly until the solution reached pH 7 - pH 8. The reaction mixture was passed through a plug of $Al_2O_3$ (10% MeOH in $CHCl_3$). The organic solvent was removed under reduced pressure giving a yellow solid (0.35 g, 88 %)

$^1H$-NMR ($CDCl_3$, 400 MHz): δ[ppm] = 8.67 (s, H-5); 7.56-7.40 (m, H-8); 4.71-4.56 (m, 1H, H-3 of benzoxazine ring); 4.56-4.33 (m, 3H, 2H of $NHCHCH_2O$+1H of $CH_2OCO$); 4.33-4.21 (m, 1H, $CH_2OCO$); 4.02-3.91 (m, 1H, CHOH); 3.57-3.52 (m, 2H, $CH_2OH$); 3.52-3.34 (m, 4H, 2x$CH_2$ piperazine); 2.85-2.56 (m, 4H, 2x$CH_2$ piperazine); 2.43 (s, 3H, $NHCH_3$); 1.52 (d, 3H, $CH_3$).

MS (MeOH) 436 [M+ 1] 458 [M+23]

Example 8: (S)-1,3-dihydroxypropan-2-yl 9-fluoro-3-methyl-10-(4-methylpiperazin-1-yl)-7-oxo-3,7-dihydro-2H-[1,4]oxazino[2,3,4-ij]quinoline-6-carboxylate

a) (S)-2-phenyl-1,3-dioxan-5-yl 9-fluoro-3-methyl-10-(4-methylpiperazin-1-yl)-7-oxo-3,7-dihydro-2H-[1,4]oxazino[2,3,4-ij]quinoline-6-carboxylate

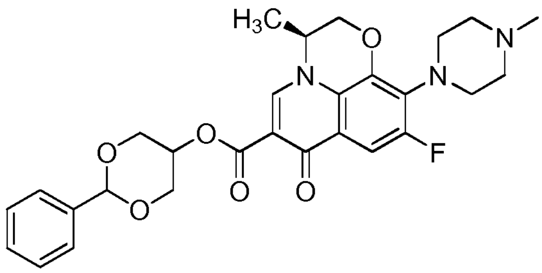

(S)-9-fluoro-3-methyl-10-(4-methylpiperazin-1-yl)-7-oxo-3,7-dihydro-2H-[1,4] oxazino [2,3,4-ij] quinoline-6-carboxylic acid (4.18 g, 11.56 mmol) was dissolved in anhydrous dichloromethane (210 ml) under argon. 1,3-O-benzylideneglycerol (2.50 g, 13.67 mmol), triethylamine (4.68 g, 46.24 mmol) and HBTU (4.38 g, 11.56 mmol) were added successively. The reaction mixture was stirred at room temperature (exclusion of light) for 3 days. The reaction mixture was washed with saturated $NaHCO_3$ solution (500 ml), aqueous (pH 5) hydrochloric acid (500 ml) and water (500 ml) successively. The organic layer was dried over $Na_2SO_4$ and the solvent was removed under reduced pressure. The crude product was purified through flash column chromatography ($SiO_2$/10 % MeOH in $CHCl_3$) yielding 4.32 g, 71 %) of a yellow, crystalline solid.
$^1$H-NMR ($CDCl_3$, 400 MHz): $\delta$[ppm] = 8.22 (s, 1H, H-5); 7.64-7.52 (m, 1H, H-8); 7.46-7.31 (m,4H, ArH); 7.10 (d, 1H, ArH); 5.65 (s, 1H, CHOCO); 4.91 (s, 1H, ArCH); 4.64-4.01 (m, 7H, 1H of CHN+2H of $CH_2O$+4H of 2x$CH_2O$); 3.38-3.22 (m, 2x$CH_2$ of piperazine ring); 2.62-2.43 (m, 4H, 2x$CH_2$ of piperazine ring); 2.36 (s, $NCH_3$); 0.95 (d, 3H, $CHCH_3$)
MS (MeOH) 524 [M+1] 546 [M+23]

b) (S)-1,3-dihydroxypropan-2-yl 9-fluoro-3-methyl-10-(4-methylpiperazin-1-yl)-7-oxo-3,7-dihydro-2H-[1,4]oxazino[2,3,4-ij]quinoline-6-carboxylate

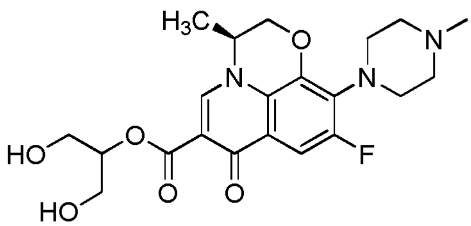

(S)-2-phenyl-1,3-dioxan-5-yl 9-fluoro-3-methyl-10-(4-methylpiperazin-1-yl)-7-oxo-3,7-dihydro-2H-[1,4]oxazino[2,3,4-ij]quinoline-6-carboxylate (0.50 g, 9.55 mmol), was dissolved in a mixture of dichloromethane/methanol (2:1.5, 87.5 ml) under argon. Palladium catalyst (10 wt % Pd/C) (180 mg) was added, the flask was evacuated and allowed to stir 1 atm of hydrogen gas at room temperature for 16 hours. The crude reaction mixture was passed through a glass microfiber on a sintered funnel. The volatiles were removed under reduced pressure yielding 370 mg (88%) of a yellow gum
$^1$H-NMR ($CDCl_3$, 400 MHz): $\delta$[ppm] = 8.72 (s, 1H), 7.57 (dd, 1H, $J^1$ = 12.6 Hz, $J^2$ = 1.8 Hz), 4.67-4.60 (m, 1H), 4.55-4.49 (m, 1H), 4.41-4.35 (m, 2H), 4.31-4.25 (m, 1H), 4.00-3.92 (m, 1H), 3.69-3.63 (m, 2H), 3.46-3.33 (m, 4H), 2.71-2.60 (m, 4H), 2.40 (s, 3H), 1.53 (d, 3H, J = 6.8 Hz)

**Benzocaine** (ethyl 4-aminobenzoate)

Example 9 (Comparative): Ethyl 4-((1,3-dihydroxypropan-2-yloxy)carbonylamino)benzoate

a) 2-phenyl-1,3-dioxan-5-yl carbonochloridate

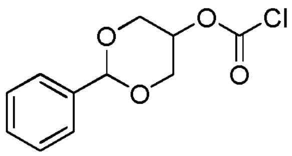

Triphosgene (bis(trichloromethyl)carbonate (13.35 g, 45.0 mmol) was added under argon at room temperature to a solution of 1,3-O-benzylideneglycerol (18.02 g, 100.0 mmol) in dry $CH_2Cl_2$ (300 ml). The reaction mixture was cooled to -40°C and a solution of pyridine (10.9 ml, 135.0 mmol) was added over 35 min under stirring. Upon completion of the addition the reaction mixture was stirred for 30 min at -40°C. Then it was allowed to warm to 0°C over 60 min and subsequently warmed to rt over 3.5 h under stirring. The reaction product was used in the next step without any further purification
$^1$H-NMR ($CDCl_3$, 400 MHz): δ[ppm] = 7.42-7.33 (m, 2H), 7.31-7.21 (m, 3H), 5.50 (s, 1H), 4.72-4.70 (m, 1H), 4.34-4.28 (m, 2H), 4.18-4.09 (m, 2H)

b) Ethyl 4-((2-phenyl-1,3-dioxan-5-yloxy)carbonylamino)benzoate

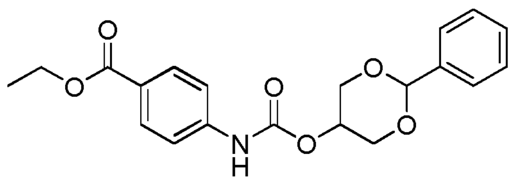

Ethyl 4-aminobenzoate (benzocaine) (16.53 g, 0.10 mol) was dissolved in dry $CH_2Cl_2$ (160 ml) under argon. The reaction mixture was cooled to 0°C and triethylamine (16.7 ml, 0.12 mol) was added. 2-phenyl-1,3-dioxan-5-yl carbonochloridate (224.27 g, 0.10 mol) (as a solution in 300 ml $CH_2Cl_2$ - obtained in the previous reaction step) was added gradually over 40 min. The reaction mixture was stirred for 1 h at 0°C and then gradually warmed to room temperature and allowed to stirr over night. The crude reaction mixture was was washed with 1M aqueous HCl (2 x 300 ml), saturated $NaHCO_3$ solution (2 x 300 ml) and water (2 x 300 ml) successively. The organic layer was dried over $Na_2SO_4$ and the solvent was removed under reduced pressure. The crude product was purified through flash column chromatography ($SiO_2$/$CHCl_3$) yielding (25.9 g, 70.0 mmol, 71 %) of a colourless crystalline solid.
$^1$H-NMR ($CDCl_3$, 400 MHz): δ[ppm] = 8.00 (d, 2H, J = 8.8 Hz), 7.55-7.50 (m, 2H), 7.44 (d, 2H, J = 8.8 Hz), 7.41-7.36 (m, 3H), 7.11 (s, 1H), 5.62 (s, 1H), 4.41-4.33 (m, 4H), 4.25-4.22 (m, 2H), 1.38 (tr, 3H, J = 7.1 Hz)

c) Ethyl 4-((1,3-dihydroxypropan-2-yloxy)carbonylamino)benzoate

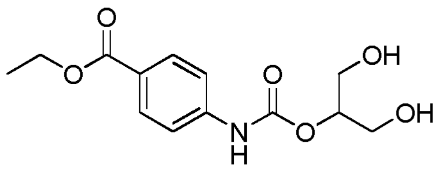

Ethyl 4-((2-phenyl-1,3-dioxan-5-yloxy)carbonylamino)benzoate (4.70 g, 12.7 mmol) was dissolved in ethanol (500 ml) under argon and Pd/C (2.48 g, 2.3 mmol) was added. The flask was flushed with hydrogen and allowed to stirr

under 1 atm $H_2$ at room temperature for 16 h. The crude reaction mixture was passed through a plug of celite and the solvent was removed under reduced pressure yielding (3.14 g, 11.2 mmol, 88 %). The product was used without any further purification.
$^1$H-NMR ($CDCl_3$, 400 MHz): δ[ppm] = 8.00 (d, 2H, J = 8.8 Hz), 7.46 (d, 2H, J = 8.8 Hz), 7.17 (s, 1H), 4.97-4.93 (m, 1H), 4.35 (quart, J = 7.1 Hz), 3.99-3.86 (m, 4H), 2.62-2.11 (br, 2H), 1.38 (tr, 3H, J = 7.1 Hz)

**Menthol (1R,2S,5R)-2-isopropyl-5-methylcyclohexanol)**

Example 10 (Comparative): (1R,2S,5R)-2-isopropyl-5-methylcyclohexyl 2,3-dihydroxypropanoate

a) Sodium (R)-(+)-2,2-dimethyl-1,3-dioxolane-4-carboxylate

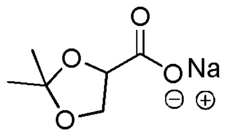

Methyl (R)-(+)-2,2-dimethyl-1,3-dioxolane-4-carboxylate (1.00 g, 6.2 mmol) was dissolved in 80 % aqueous 1,4-dioxane. 1.2 M aqueous NaOH solution was added (5.2 ml) and the reaction mixture was stirred at room temperature for 3 h. After that the solvents were removed under reduced pressure and a colourless solid was obtained (1.03 g, 6.1 mmol, 98 %). The product was used immediately in the next reaction step without further purification.

b) (R)-(+)-2,2-dimethyl-1,3-dioxolane-4-carboxylic acid

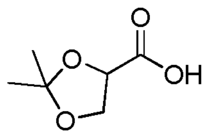

Sodium (R)-(+)-2,2-dimethyl-1,3-dioxolane-4-carboxylate (1.03 g, 6.1 mmol) was dissolved in a mixture of water (1.2 ml) and ethyl acetate (1.2 ml) and cooled to 0°C. 2 M aqueous $H_3PO_4$ solution (7 ml) was added until the reaction mixture reached pH 2. After that the reaction mixture was saturated with NaCl and the reaction mixture was extracted with ethyl acetate (3 x 20 ml). The organic layer was dried over $Na_2SO_4$ and the solvent was removed under reduced pressure. The product (0.73 g, 4.9 mmol, 81 %) was used in the next step without any further purification.
$^1$H-NMR ($CDCl_3$, 400 MHz): δ[ppm] = 8.99-8.13 (br, 1H), 4.64-4.61 (m, 1H), 4.32-4.25 (m, 1H), 4.22-4.17 (m, 1H), 1.52 (s, 3H), 1.41 (s, 3H)

c) (1R,2S,5R)-2-isopropyl-5-methylcyclohexyl 2,2-dimethyl-1,3-dioxolane-4-carboxylate

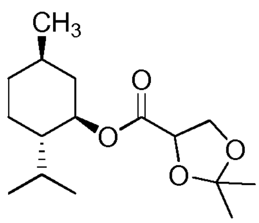

(R)-(+)-2,2-dimethyl-1,3-dioxolane-4-carboxylic acid (2.86 g, 19.6 mmol) was dissolved in anhydrous $CH_2Cl_2$ (120 ml) under argon. Menthol (1R,2S,5R)-2-isopropyl-5-methylcyclohexanol) (3.67 g, 23.5 mmol), triethylamine (7.93 g, 78.4 mmol) and O-benzotriazol-1-yl-N,N,N',N'-trimethyluronium hexafluorophosphate (8.18 g, 21.6 mmol) were added

successively. The reaction mixture was stirred under argon for 72 h. The reaction mixture was washed with saturated $NaHCO_3$ solution (3 x 50 ml), aqueous HCl (pH 5) (3 x 50 ml) and water (3 x 50 ml) and dried over $Na_2SO_4$. The organic solvent was removed under reduced pressuregiving a yellow oil. (0.54 g, 0.93 mmol, 93 %). The crude product was purified through flash column chromatography ($SiO_2$/15% - 25% gradient of ethyl acetate in hexane) yielding 3.16 (11.2 mmol, 57 %) of a yellow oil.
$^1$H-NMR ($CDCl_3$, 400 MHz): δ[ppm] = 4.82-4.69 (m, 1H), 4.59-4.50 (m, 1H), 4.25-4.18 (m, 1H), 4.10 -4.01 (m, 1H), 2.03-1.94 (m, 1H), 1.89-1.77 (m, 1H), 1.70-1.62 (m, 2H), 1.55-1.35 (m, 8H), 1.09-0.95 (m, 2H), 0.92-0.79 (m, 7H), 0.74 (d, J = 7.0 Hz)

d) (1R,2S,5R)-2-isopropyl-5-methylcyclohexyl 2,3-dihydroxypropanoate

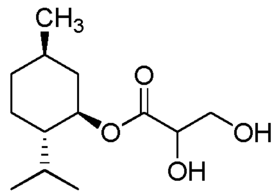

(1R,2S,5R)-2-isopropyl-5-methylcyclohexyl 2,2-dimethyl-1,3-dioxolane-4-carboxylate (1.00 g, 35.2 mmol) was dissolved in 98 % aqueous ethanol (10 ml). Amberlyst 15 (wet) ion exchange resin (0.30 g) and bumping granulate were added. The reaction mixture was refluxed for 4 h without stirring (under argon). After that, the reaction was brought to room temperature, the resin and bumping granulate were removed and the solvent was removed under reduced pressure. The product was obtained in quantitative yield (0.94 g)
$^1$H-NMR ($CDCl_3$, 400 MHz): δ[ppm] = 4.86-4.74 (m, 1H),4.26-4.19 (m, 1H), 3.92-3.87 (m, 1H), 3.85-3.79 (m, 1H), 3.25-3.14 (m, 1H), 2.22-2.08 (m, 1H), 2.06-1.96 (m, 1H), 1.93-1.79 (m, 1H), 1.74-1.65 (m, 2H), 1.56-.

Example 11 (Comparative): 1,3-dihydroxypropan-2-yl (2S,5R)-2-isopropyl-5-methylcyclohexyl phosphonate

a) Dichloro((1R,2S,5R)-2-isopropyl-5-methylcyclohexyloxy)phosphine

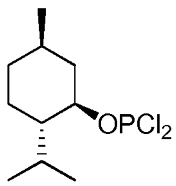

The phosphorus trichloride (56.0ml, 87.89g, 0.640mol) was added to dichloromethane (anhydrous) under argon gas to obtain a solution. The solution was cooled to -30°C and (-)-menthol (1R,2S,5R) (10.00g, 0.0640mol) was added portion wise over 10mins. The solution was allowed to warm to ambient temperature and was allowed to stir at ambient temperature for 16 hours. The solvent was removed in vacuo to obtain a colourless oil (16.46g). The crude product was used directly in the subsequent step.

b) (2S,5R)-2-isopropyl-5-methylcyclohexyl 2-phenyl-1,3-dioxan-5-yl phosphonate

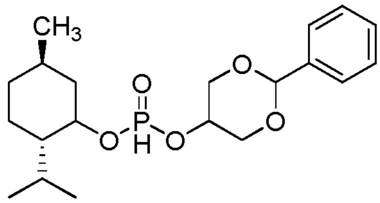

The dichloro(2S,5R)-2-isopropyl-5-methylcyclohexyloxyphosphine (8.23g, 0.0320mol) was dissolved in dichloromethane (anhydrous) (100ml) under argon gas. Cooled to 0°C. Added a solution of cis-1,3-benzylideneglycerol (7.21g, 0.0400mol) and tert-butanol (2.97g, 0.040mol) (1:1) in dichloromethane (anhydrous) dropwise over 30 mins. Then added triethylamine (8.9ml, 6.47g, 0.0640mol) dropwise. Allowed to stir at 0°C for 10mins. And then allowed to gradually warm to ambient temperature. And removed solvent *in vacuo* to obtain a colourless crystalline solid (26.67g).

The crude product was chromatographed on silica gel using EtOc/pet spirit 60-80 to obtain the product fraction as a colourless oil (1.29g)

$^1$H-NMR ($CDCl_3$, 400 MHz): $\delta$[ppm] = 7.93-7.92 (m, 0.5H, P-H), 7.58-7.29 (m, 5H), 6.17-6.16 (m, 0.5H, P-H), 5.56 (s, 1H), 4.53-3.98 (m, 6H), 2.31-1.89 (m, 3H), 1.88-0.57 (m, 15H)

c) 1,3-dihydroxypropan-2-yl (2S,5R)-2-isopropyl-5-methylcyclohexyl phosphonate

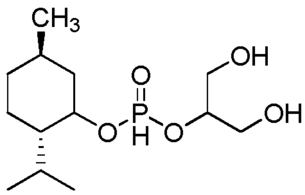

The The dichloro(2S,5R)-2-isopropyl-5-methylcyclohexyl-2-phenyl-1,3-dioxan-5-yl phosphonate (0.97g; 2.54mmol) was dissolved in 80% (v/v) aqueous acetic acid (10ml) and allowed to stir at 70°C for 3hrs. The solvent was removed *in vacuo* followed by pumping under high vacuum to obtain an oil (0.69 g, 2.37 mmol).

$^1$H-NMR ($CDCl_3$, 400 MHz): $\delta$[ppm] = 7.89-7.87 (m, 0.5H, P-H), 6.01-5.89 (m, 0.5H, P-H), 4.42-3.28 (m, 8H), 2.29-0.51 (m, 18H)

**Phenol**

Example 12 (Comparative): 1,3-dihydroxypropan-2-yl phenyl succinate

a) 4-oxo-4-phenoxybutanoic acid

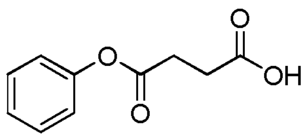

Phenol (1.88 g, 20.0 mmol) was dissolved in a solution of sodium carbonate (anhydrous) (1.06 g, 10.0 mmol) in water (20 ml) and cooled to 0°C. Succinic anhydrid (2.00 g, 20.0 mmol) was added and the suspension was allowed to stirr at 0°C for 1 h. The reaction mixture was gradually warmed to room temperature and stirred over night. The clear solution was cooled to 0°C and acidified to pH 0 (addition of 1M aqueous HCl). The reaction mixture was extracted with chloroform (3 x 25 ml), dried over $Na_2SO_4$, filtered and the solvent removed under reduced pressure to obtain a white solid (1.63 g, 8.4 mmol, 42%). The product was used without any further purification.

$^1$H-NMR ($CDCl_3$, 200 MHz): $\delta$[ppm] = 7.44-7.03 (m, 5H), 2.95-2.77 (m, 4H)

b) Phenyl 2-phenyl-1,3-dioxan-5-yl succinate

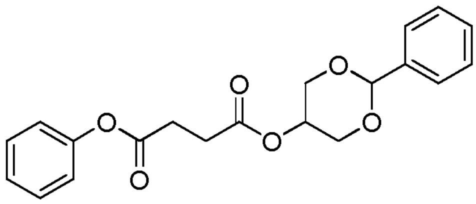

4-oxo-4-phenoxybutanoic acid (1.00 g, 5.15 mmol) was dissolved in anhydrous $CH_2Cl_2$ (55 ml) under argon. Menthol 1,3-O-benzylideneglycerol (1.11 g, 6.18 mmol), triethylamine (2.09 g, 20.6 mmol) and O-benzotriazol-1-yl-N,N, N',N'-trimethyluronium hexafluorophosphate (2.15 g, 5.67 mmol) were added successively. The reaction mixture was stirred under argon for 72 h. The reaction mixture was washed with saturated $NaHCO_3$ solution (3 x 50 ml), aqueous HCl (pH 5) (3 x 50 ml) and water (3 x 50 ml) and dried over $Na_2SO_4$. The organic solvent was removed under reduced pressuregiving a yellow oil. (0.54 g, 0.93 mmol, 93 %). The crude product was purified through flash column chromatography ($SiO_2$/10% MeOH in chloroform) yielding 0.98 g (2.58 mmol, 50 %) of a yellow solid.
$^1$H-NMR ($CDCl_3$, 400 MHz): δ[ppm] = 7.54-7.48 (m, 2H), 7.41-7.31 (m, 5H), 7.23-7.19 (m, 1H), 7.13-7.06 (m, 2H), 5.57 (s, 1H), 4.78 (m, 1H), 4.33-4.29 (m, 2H), 4.20-4.16 (m, 2H), 2.97-2.86 (m, 4H)

c) 1,3-dihydroxypropan-2-yl phenyl succinate

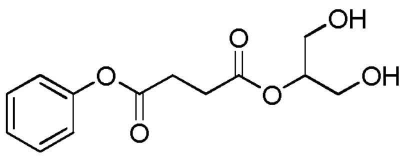

Phenyl 2-phenyl-1,3-dioxan-5-yl succinate (0.11 g, 0.3 mmol) was dissolved in ethanol (20 ml) under argon and Pd/C (0.09 g, 0.1 mmol) was added. The flask was flushed with hydrogen and allowed to stirr under 1 atm $H_2$ at room temperature for 16 h. The crude reaction mixture was passed through a plug of celite and the solvent was removed under reduced pressure yielding (0.9 g, 0.26mmol, 89 %). The product was used without any further purification.
$^1$H-NMR ($CDCl_3$, 400 MHz): δ[ppm] = 7.43-7.33 (m, 2H), 7.25-7.21 (m, 1H), 7.12-7.05 (m, 2H), 4.97 (quint, 1H, J = 4.4 Hz), 3.88-3.80 (m, 4H), 2.95-2.91 (m, 2H), 2.82-2.75 (m, 2H), 2.58-1.60 (br, 2H)

(6) Synthesis of the protected monoglyceride - linker-building blocks

(a) Synthesis of protected monoglycerides with a diacid linker

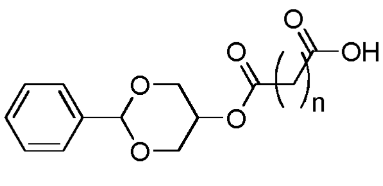

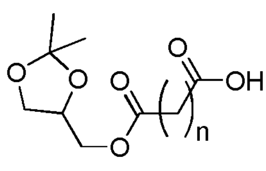

1,3-Benzylideneglycerol and solketal ((2,2-dimethyl-1,3-dioxolan-4-yl)methanol) are each separately dissolved in organic solvent and each reacted with a diacid (succinic, glutaric, adipic, pimelic, suberic, azelaic, sebacic) under appropriate conditions.

(b) Synthesis of protected monoglycerides with an omega hydroxy acid linker

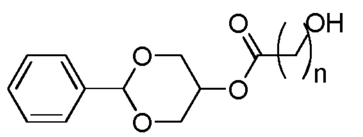

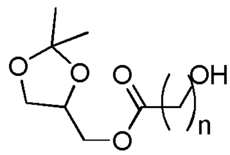

1,3-Benzylideneglycerol and solketal are each separately dissolved in organic solvent and each reacted with an omega hydroxy acid under appropriate conditions.

(c) Synthesis of protected monoglycerides with a chloroformate linker

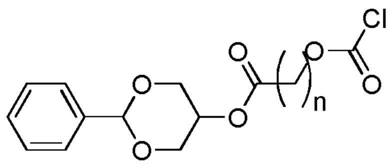

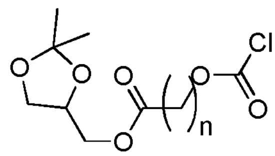

1,3-Benzylideneglycerol and solketal are each separately dissolved in organic solvent and each reacted with an omega hydroxy acid under appropriate conditions. The product of this conversion is reacted with triphosgene to give the protected monoglycerides with a chloroformate linker.

(d) Synthesis of chloroformate functionalised protected monoglycerides

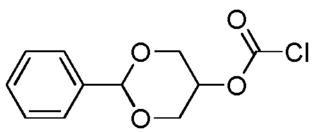

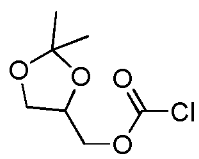

1,3-Benzylideneglycerol and solketal are each separately dissolved in organic solvent and each reacted with triphosgene under appropriate conditions.

(7) Synthesis of the monoglyceride-linker-bioactive agent-conjugates

(a) Coupling of hydroxy containing bioactive agents with protected monoglycerides with a diacid linker

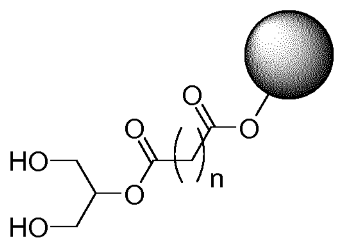

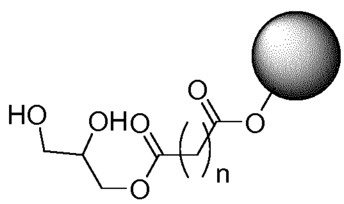

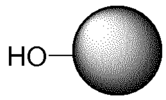 ≡ Bioactive agent

A hydroxy containing bioactive agent - such as Codeine, Fluconazole, Latanoprost or Dexamethasone - (after conversion into a prodrug by protecting additional functional groups with appropriate bioerodable protection groups) is reacted with protected monoglycerides with a diacid linker. The monoglyceride protection groups are removed under appropriate conditions giving the monoglyceride functionalised (at the primary or secondary alcohol positions of the glyceride) bioactive agent monomers.

(b) Coupling of hydroxy containing bioactive agents with protected monoglycerides with an omega hydroxy acid linker

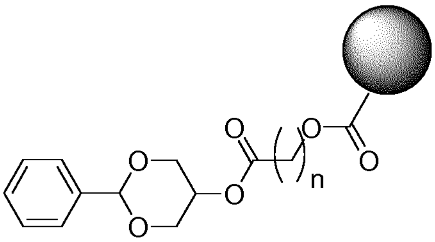

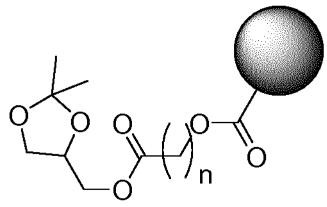

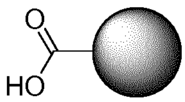 ≡ Bioactive agent

A carboxy acid containing bioactive agent - such as Ciprofloxacin, Levofloxacin or Valproic acid - (after conversion into a prodrug by protecting additional functional groups with appropriate bioerodable protection groups) is reacted with protected monoglycerides with a omega hydroxy acid linker. The monoglyceride protection groups are removed under appropriate conditions giving the monoglyceride functionalised (at the primary or secondary alcohol positions of the glyceride) bioactive agent monomers.

(c) Coupling of amine containing bioactive agents with protected monoglycerides with a chloroformate linker

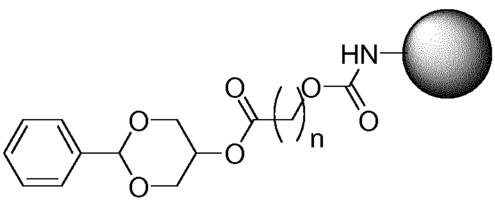

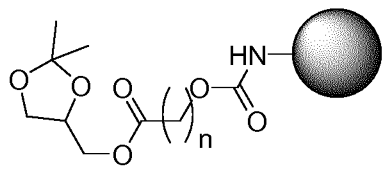

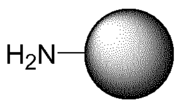 ≡ Bioactive agent

An amine containing bioactive agent - such as Benzocaine - (after conversion into a prodrug by protecting additional functional groups with appropriate bioerodable protection groups) is reacted with protected monoglycerides with a chloroformate linker. The monoglyceride protection groups are removed under appropriate conditions giving the monoglyceride functionalised (at the primary or secondary alcohol positions of the glyceride) bioactive agent monomers.

(d) Coupling of amine containing bioactive agents with chloroformate functionalised protected monoglycerides

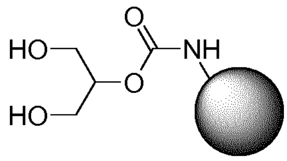

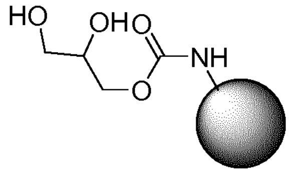

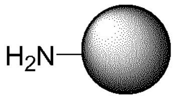 ≡ Bioactive agent

An amine containing bioactive agent - such as Benzocaine - (after conversion into a prodrug by protecting additional functional groups with appropriate bioerodable protection groups) is reacted with chloroformate functionalised protected monoglycerides. The monoglyceride protection groups are removed under appropriate conditions giving the monoglyceride functionalised (at the primary or secondary alcohol positions of the glyceride) bioactive agent monomers.

(e) Coupling of amine containing bioactive agent with carboxylic acid functionalised protected monoglyceride

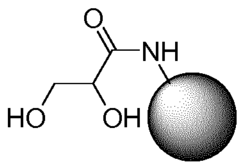

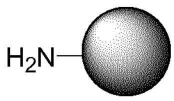 ≡ Bioactive agent

An amine containing bioactive agent - such as Benzocaine - (after conversion into a prodrug by protecting additional functional groups with appropriate bioerodable protection groups) is converted into the corresponding isocyanate. Reaction of the isocyanate with a carboxylic acid functionalised protected monoglyceride produces (via release of $CO_2$) the corresponding amide derivative which is subsequently deprotected.

f) Conversion of an amine containing bioactive agent to an aromatic carbamate containing functionalised protected monoglyceride

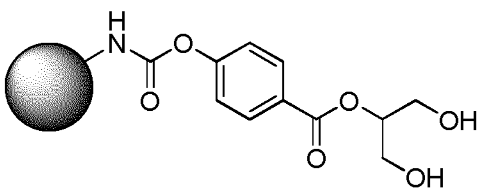

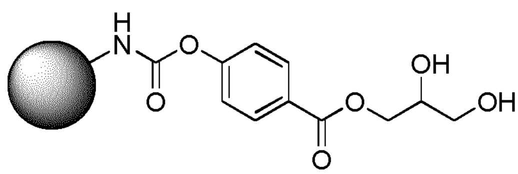

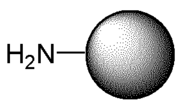 ≡ Bioactive agent

An amine containing bioactive agent - after conversion into a prodrug by protecting additional functional groups with appropriate bioerodable protection groups - is converted into the corresponding functionalised protected monoglyceride containing an aromatic carbamate.

(8) Synthesis of bioactive agent-linker conjugates

(a) Synthesis of diacid-bioactive agent conjugates

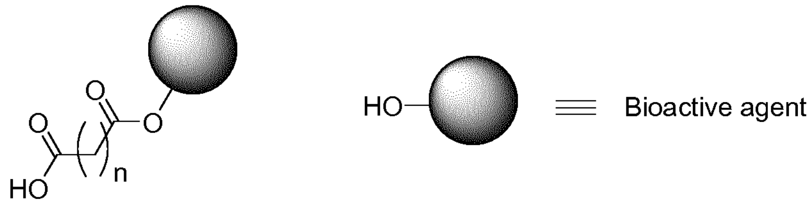


A hydroxy containing bioactive agent - such as Codeine, Fluconazole, Latanoprost or Dexamethasone - (after conversion into a prodrug by protecting additional functional groups with appropriate bioerodable protection groups) is reacted with a diacid or a diacid derivative.

(b) Synthesis of acid/omega hydroxy-bioactive agent conjugates

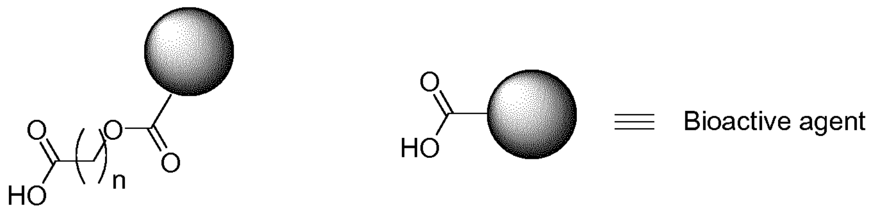


An carboxy acid containing bioactive agent - such as Ciprofloxacin, Levofloxacin or Valproic acid - (after conversion into a prodrug by protecting additional functional groups with appropriate bioerodable protection groups) is reacted with an acid/omega hydroxy linker or a derivative thereof.

(c) Synthesis of an acid/chloroformate-bioactive agent conjugates

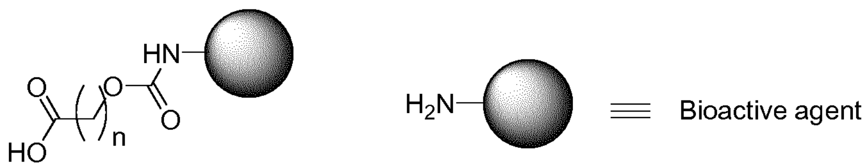


An amine containing bioactive agent - such as Benzocaine - (after conversion into a prodrug by protecting additional functional groups with appropriate bioerodable protection groups) is reacted with a an acid/chloroformate linker or a derivative thereof.

d) Conversion of an carboxy acid containing bioactive agent to functionalised protected monoglyceride containing a spacer group.

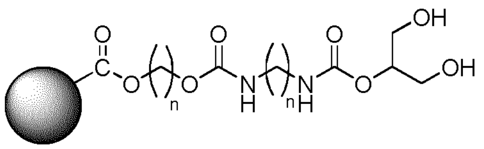

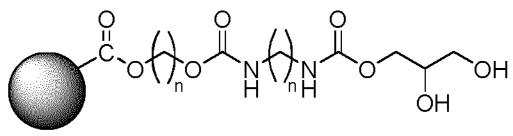

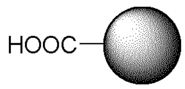 ≡ Bioactive agent

An carboxy acid containing bioactive agent - after conversion into a prodrug by protecting additional functional groups with appropriate bioerodable protection groups - is converted into the corresponding functionalised protected monoglyceride with a spacer group that can be modified to meet the required polymer properties and release kinetics.

e) Conversion of an alcohol containing bioactive agent to functionalised protected monoglyceride containing a spacer group.

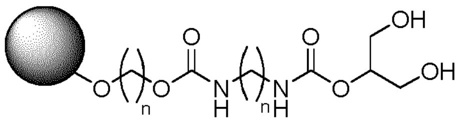

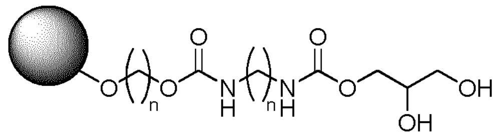

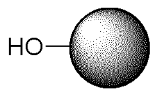 ≡ Bioactive agent

An alcohol containing bioactive agent - after conversion into a prodrug by protecting additional functional groups with appropriate bioerodable protection groups - is converted into the corresponding functionalised protected monoglyceride with a spacer group that can be modified to meet the required polymer properties and release kinetics.

(9) Synthesis of monoglyceride-linker-bioactive agent-conjugates

(a) Coupling of diacid-bioactive agent conjugates with protected monoglycerides

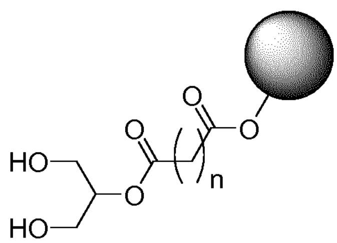

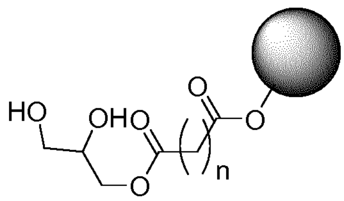

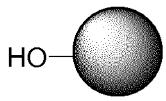 ≡ Bioactive agent

A diacid-bioactive agent conjugate, containing a bioactive agent such as Codeine, Fluconazole, Latanoprost or Dexamethasone - (after conversion into a prodrug by protecting additional functional groups with appropriate bioerodable protection groups) is reacted with protected monoglycerides. The monoglyceride protection groups are removed under appropriate conditions giving the monoglyceride functionalised (at the primary or secondary alcohol positions of the glyceride) bioactive agent monomers.

(b) Coupling of an omega hydroxy acid-bioactive agent conjugate with protected monoglycerides

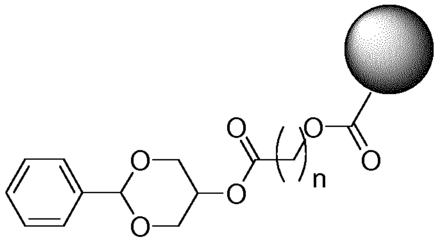

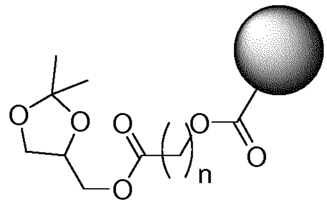

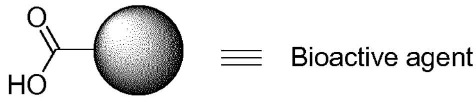


An omega hydroxy acid-bioactive agent conjugate containing a bioactive agent - such as Ciprofloxacin, Levofloxacin or Valproic acid - (after conversion into a prodrug by protecting additional functional groups with appropriate bioerodable protection groups) is reacted with protected monoglycerides. The monoglyceride protection groups are removed under appropriate conditions giving the monoglyceride functionalised (at the primary or secondary alcohol positions of the glyceride) bioactive agent monomers.

(c) Coupling of an acid/chloroformate-bioactive agent conjugates with protected monoglycerides

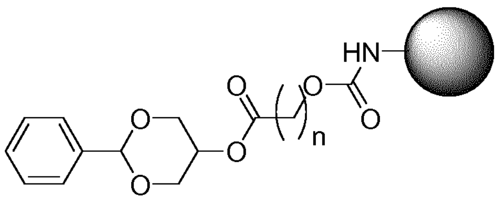

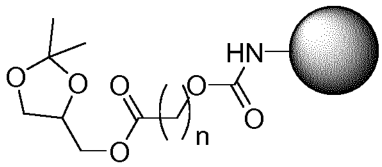

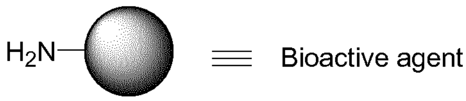


An an acid/chloroformate-bioactive agent conjugate containing a bioactive agent - such as Benzocaine - (after conversion into a prodrug by protecting additional functional groups with appropriate bioerodable protection groups) is reacted with protected monoglycerides. The monoglyceride protection groups are removed under appropriate conditions giving the monoglyceride functionalised (at the primary or secondary alcohol positions of the glyceride) bioactive agent monomers.

Conversion of a phosphate containing bioactive agent to functionalised protected monoglyceride (as phosphate ester) with and/or without a spacer group.

X=spacer - as described in the previous text, R=protection group

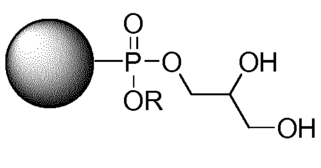

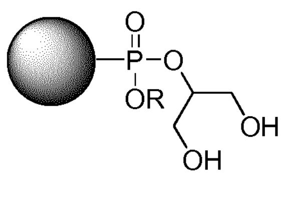

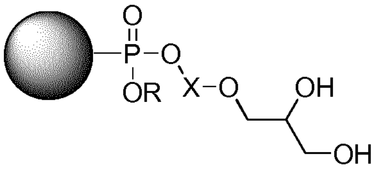 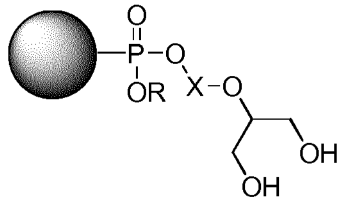

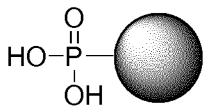 ≡ Bioactive agent

A phosphate containing bioactive agent - after conversion into a prodrug by protecting additional functional groups with appropriate bioerodable protection groups - is converted into the corresponding functionalised protected monoglyceride (as phosphate ester) with or without a spacer group that can be modified to meet the required polymer properties and release kinetics.

**Polymer Production**

This section describes how the functionalised bioactive-monomers conjugates have been covalently linked to the polymer backbone and formed part of the bioactive-polymer conjugates containing the selected bioactive as a pendant attachment. The pendant attached bioactive is able to be released by the breakdown of the covalent bonds through hydrolysis and the degradation of the linkages attaching the bioactive to the polymer.

**Synthesis of Polyols**

(a) DLLA 1000

The DLLA 1000 was produced by the polycondensation reaction of an 88% DL Lactic acid solution (Fluka) using 1-4 butane diol (Aldrich) as the initiator and 2 Ethyl Hexanoate (Aldrich) as the catalyst. The polycondensation reaction was undertaken in a stainless steel vessel (Buchi BEP280 reactor). The reagents were added to the reaction vessel [1-4 BDO; 167.5g; DL-Lactic acid solution 2418g; 2 Ethyl Hexanoate; 0.12% , 2.4g] and the mixture was stirred at 350prm while bubbling nitrogen gas through the solution (3mL/min). A tube was attached to the reactor outlet to collect the water evolved.

Heating was applied (jacket oil temperature 145°C) and the mixture was left to stir for 2 days. Approximately 280mL of water from collected from the reaction mixture.

Samples were taken and the acid number determined by titration using the methods outlined above. The low acid number obtained [0.09 mg/KOH / g] was used to indicate the reaction was completed. The mixture was cooled and subsamples taken for GPC, NMR and hydroxyl number analysis using the methods outlined above.

(b) PLGA (50:50) 1000

Purasorb DL Lactide (1000g) and Purasorb Glycolide (805.5g) was added to the 3L stainless steel Buchi reactor. The reactor was heated under a nitrogen blanket without stirring to a jacket oil temperature of 145°C. The stirrer was started and the jacket temperature adjusted to 120°C.

To the stirred reaction mixture was added 156.3g of 1-4 BDO and 2.2g 2-Ethyl Hexanoate. An exotherm was observed with the reaction temperature rising to 143°C in 8 minutes. The mixture was heated for 40hrs at a stirring rate of 300rpm under a nitrogen blanket.

Samples were taken and the acid number determined by titration using the methods outlined above.

**Polyurethane and Polyester-Urethane Formulations**

Polylactic Acid (Mw 1000 g/mol) and polycaprolactone diol (Mw 1000 g/mol) were both dried under high vacuum overnight at 75°C before use. Hexamethylene diisocyanate (HDI) and Ethyl-lysine diidocyanate (ELDI) were distilled before use.

### Polyurethane Bulk Synthesis Method

The required amounts of DLLA, PLGA or PCLD (ERA Chemicals Pty Ltd) polyester polyols were weighed into a beaker and kept warmed in a pre-heated nitrogen purged oven. The required amount of bioactive-monomer conjugate was first dried under vacuum with mild heat then mix added to the reaction beaker containing the DLLA, PLGA or PCLD components and retuned to an oven to equilibrate.

The required amount of diisocyanate HDI was weighed into a syringe for addition to the other components. Three drops of Dibutyltin Dilaurate (DBTDL) or 2 Ethyl Hexanoate ((Sn2EH) catalyst were first added to the bioactive-monomer conjugate and/polyol mixture. The monomer/s and catalyst were mixed well before either the HDI or ELDI was added via a syringe. The whole mixture was mixed and stirred vigorously with a spatula until the mixture thickened considerably before it was poured onto a baking tray. The tray was then placed in an oven overnight at 80°C to cure the polymer.

Optimisation of the molecular weight was achieved by varying the HDI or ELDI index. The molecular weights of the polymers were characterised by Gel Permeation Chromatography (GPC) using THF or DMF as the solvent. Reaction completion and structural integrity were confirmed by means of $^1$H NMR.

### Polyurethane Solution Synthesis Method

Bioactive-monomer conjugates were dried under vacuum with mild heat then added to dry DMF (typically > 1g) in a small round bottle flask. Two drops of DBTDL or Sn2EH were added and the mixture warmed in an oil bath at 85°C before injecting in a slight excess of HDI or ELDI. The mixture was typically found to have thickened considerably after stirring overnight at 85°C. The DMF solvent was removed and the material was then analysed or further purified.

### Polyurethane Precipitation Purification Method

The resulting PU was dried to remove any DMF present. The polymer was then dissolved in a minimal amount of DMSO then precipitated from solution into acetonitrile. Any unreacted bioactive-monomer conjugate remained dissolved in the DMF/Acetonitrile solution.

### Aliphatic Polyurethanes containing Valproic Acid Monoglyceride (VA-MG)

<u>Comparative Example 1: PU polymer bioactive-coniugate containing 50mole% VA-MG</u>

A polymer-bioactive conjugate containing 50mole% VA-MG was formed by the reaction of 1:1 molar ratio of VA-MG and HDI according to the Polyurethane Bulk Synthesis Method. The VA-MG synthesised in Example 1 above was dried under vacuum. The VA-MG (1.005g, 4.6mmol) was added to a dried 150ml polypropylene (PP) beaker. DBTDL (dibutyl tin dilaurate) catalyst was added to the beaker (0.0041 g). The mixture was stirred and heated in the beaker at 70°C for 5 mins. HDI was then added by syringe (1.003g 5.9 mmol) with stirring. The mixture was then poured into a Teflon coated tray and allowed to cure for 4 hours in an oven at 70°C. Films of the polymer were pressed using a melt press set at 90°C. Teflon coated metal press plates were used. The thickness was controlled using a 200 micron shim plate. The sample was pressed for 5 mins at 90°C and then cooled to room temperature using tap water (flowing through the press platterns).

The polymer film was found to be clear, flexible and tough. The nominal loading the valproic acid in the polymer is approximately 30wt%.

Example 13: Flexible PU polymer bioactive-conjugate containing 33mole% VA-MG

A polymer-bioactive conjugate containing 33mole% VA-MG was formed by the reaction of VA-MG, PCLD1000 and HDI according Polyurethane Bulk Synthesis Method in the proportions 34mole%, 15mole% and 51mole%, respectively. PCLD 1000 (2.508g, 2.524 mmol) and VA-MG (1.185g, 5.428 mmol) were added to a dried glass beaker and mixed thoroughly with a spatula. Three drops of DBTDL (dibutyltin dilaurate) catalyst was then added to the polyols mixture. HDI (1.393g, 8.281 mmol) was delivered by a syringe and vigorous mixing was applied. The hot mixture was then poured onto a Teflon coated tray and placed in an oven to cure overnight at 85°C. Through isocyanate index optimisation, polyurethanes of high molecular weights and strength were synthesised from this formulation. A 250 micron thick film prepared by the process described above was clear, tough. The loading of VA-MG achieved by this formulation is approximately 23.3 wt%.

**Aliphatic Polyurethanes containing Levofloxacin Monoglyceride (LVX-MG)**

Example 20 (Comparative): Polyurethane Bioactive-Polymer conjugate containing > 50wt% LVX-MG

LVFX-MG (0.20g, 0.457 mmol) was dissolved in 2.5 ml of dry DMF solvent. Two drops of the DBTDL catalyst was added to the solution and heated to 65°C in a small round bottle flask with a rubber seal. HDI (0.078g, 0.464 mmol) added via a micro syringe and allowed to react overnight. After removing the DMF, flaky but brittle solid was produced. After precipitation in acetonitrile solvent, a whitish material was collected.

Example 22: Polyurethane Bioactive-Polymer conjugate containing 13wt% bound LVX ( as LVX-MG).

DLLA (1.35g, 1.20 mmol), PCLD (0.38g, 0.384 mmol) and 3 drops of DBTDL was mixed in a beaker and warmed in an oil bath at 65°C. LVFX-MG (0.65g, 1.04 mmol) was dissolved in 3 ml of dry DMF solvent. The two mixtures were then combined before injecting in the HDI (0.52g, 3.09 mmol). Over the course of a few hours, the mixture's viscosity has increased considerably. Transferred the material into a crucible and heated in an oven at 80°C overnight. After removing the solvent, a sticky waxy material was produced.

**Table 2: Bioactive-Polyurethane- Conjugates and Bioactive-Polyester-Urethane Conjugates**

(C) - indicates "comparative"

| Example | Relevant Bioactive Monomer Conjugate Example No and weight (g) | PCLD 1000 (g) | DLLA 1000 (g) | PLGA 50:50 1000 (g) | HDI (g) | ELDI (g) | Catalyst (drops) | Method | Comments |
|---|---|---|---|---|---|---|---|---|---|
| CE-1 | Ex1<br>1.0053 | 0 | 0 | 0 | 1.0037 | 0 | 5 DBTDL | Bulk | Mn = 9,575<br>Mw= 15,332<br>Mp = 15,205<br>PD = 1.60<br>Clear hard polymer |
| 13 | Ex1<br>1.1800 | 2.5086 | 0 | 0 | 1.3900 | 0 | 3 DBTDL | Bulk | Mw 254, 869;<br>Mn 127,312.<br>Clear flexible film |
| 14 | Ex2<br>1.426 | 2.7382 | 0 | 0 | 1.3108 | 0 | 5 DBTDL | Solution | Sticky Clear film |
| 15 | Ex2<br>0.8500 | 1.6205 | 0 | 0 | 0.7713 | 0 | 3 DBTDL | Solution | Rubbery polymer |
| 16 (C) | Ex3<br>3.0358 | 0 | 0 | 0 | 1.0855 | 0 | 5 DBTDL | Bulk | Rubbery polymer |
| 17 | Ex3<br>1.5860 | 2.5071 | 0 | 0 | 0.9844 | 0 | 5 DBTDL | Bulk | Rubbery flexible polymer |
| 18 | Ex3<br>1.5342 | 2.5185 | 0 | 0 | 0.9762 | 0 | 5 DBTDL | Bulk | Flexible polymer |
| 19 (C) | Ex7<br>0.4161 | 0 | 0 | 0 | 0.1652 | 0 | 5 DBTDL | Bulk | Stiff Polymer |
| | LVX-ME *<br>0.1869 | | | | | | | | |
| 20 (C) | Ex7<br>0.2 | 0 | 0 | 0 | 0.078 | 0 | 2 DBTDL | Solution | Flaky brittle polymer |
| 21 (C) | Ex7<br>0.2136<br>LVX-ME *<br>0.1223 | 0 | 0 | 0 | 0.075 | 0 | 2 DBTDL | Solution ppt Acetonitrile | Stiff polymer |

(continued)

(C) - indicates "comparative"

| Example | Relevant Bioactive Monomer Conjugate Example No and weight (g) | PCLD 1000 (g) | DLLA 1000 (g) | PLGA 50:50 1000 (g) | HDI (g) | ELDI (g) | Catalyst (drops) | Method | Comments |
|---|---|---|---|---|---|---|---|---|---|
| 22 | Ex7 0.4511 LVX-ME * 0.1989 | 0.38 | 1.35 | 0 | 0.52 | 0 | 3 DBTDL | Solution | Sticky waxy polymer |
| 23 (C) | Ex7 0.7043 LVX-ME * 0.3018 | 0 | 0 | 0 | 0.2733 | 0 | 5 DBTDL | Solution ppt Acetonitrile | Stiff polymer |
| 24 (C) | Ex8 0.5088 | 0 | 0 | 0 | 0.2002 | 0 | 3 DBTDL | Solution | Stiff Polymer |
| 25 | Ex8 3.022 | 4.9999 | 0 | 0 | 2.0001 | 0 | 3 DBTDL | Solution | Flexible polymer |
| 26 | Ex8 0.8879 | 0 | 0 | 1.5018 | 0.6162 | 0 | 5 DBTDL | Solution | Brittle and hard polymer |
| 27 | Ex8 0.7623 | 1.5140 | 0 | 0 | 0 | 0.7623 | 5 DBTDL | Solution | Soft and waxy polymer |
| 28 | Ex8 0.6034 | 0.5186 | 2.1986 | 0 | 0.6169 | 0 | 5 DBTDL | Solution | stiff polymer |
| 29 | Ex9 0.6529 | 1.2702 | 0 | 0 | 0.5971 | 0 | 5 DBTDL | Solution | Flexible white polymer Mn= 11,979 Mw= 22,898 Pd=1.91 |
| 30 | Ex9 0.5379 | 1.2499 | 0 | 0 | 0 | 0.7121 | 5 DBTDL | Solution | Flexible white polymer Mn= 14,222 Mw= 28,259 Pd=1.98 |

(continued)

(C) - indicates "comparative"

| Example | Relevant Bioactive Monomer Conjugate Example No and weight (g) | PCLD 1000 (g) | DLLA 1000 (g) | PLGA 50:50 1000 (g) | HDI (g) | ELDI (g) | Catalyst (drops) | Method | Comments |
|---|---|---|---|---|---|---|---|---|---|
| 31 | Ex10<br>0.86 | 1.7380 | 0 | 0 | 0.8928 | 0 | 5 DBTDL | Solution | Flexible polymer |
| 32 (C) | Ex11<br>0.70g | 0 | 0 | 0 | 0.5839 | | 4 Sn2EH | Bulk | Yellow sticky flexible polymer |
| 33 | Ex12<br>1.9000 | 3.6861 | 0 | 0 | 1.8119 | 0 | 5 DBTDL | Solution | Flexible polymer |
| 34 | Ex1<br>0.3184<br>Ex8<br>0.6328<br>Ex9<br>0.4106 | 2.4988 | 0 | 0 | 1.1624 | 0 | 10 DBTDL | Solution | Tough flexible polymer |
| 35 | Ex1<br>0.1343<br>Ex8<br>0.2437 | 0.6780 | 0 | 0 | 0.31 | 0 | 5 DBTDL | Solution | Tough flexible polymer |

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

## Claims

1. A bioerodible polymer - bioactive agent conjugate comprising as part of its polymer backbone a moiety of general formula (I):

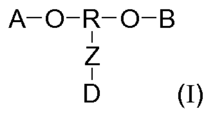

(I)

wherein:

A and B, which may be the same or different, represent the remainder of the polymer backbone and are (i) attached to the -O-R(ZD)-O- moiety as shown in formula (I) via a bioerodible moiety, and (ii) comprise a poly(urethane-ester) having monomeric units coupled via bioerodible urethane or ester moieties;
R represents a linear or branched optionally substituted hydrocarbon;
Z is a linking group; and
D is a releasable bioactive agent.

2. The bioerodible polymer - bioactive agent conjugate according to claim 1, wherein A and B each comprise a copolymer of polyurethane and polyester.

3. The bioerodible polymer - bioactive agent conjugate according to claim 1 or claim 2, which comprises less than 25 mol% of polymerised residues that are derived from a $C_2$ diol, relative to the total number of moles of polymerized diol residues.

4. The bioerodible polymer - bioactive agent conjugate according to any one of claims 1 to 3, wherein D is coupled through Z to R by an ester, amide, anhydride, imide, carbonate, peroxide, peroxyester, thiol, phosphate ester, thioester, sulphate ester, carbamate, azo or boronate ester moiety.

5. The bioerodible polymer - bioactive agent conjugate according to claim 4, wherein Z is selected from -O-; -C(O)-; and optionally substituted: -OC(O)-$C_{1-18}$alkylene-C(O)-; -C(O)O-$C_{1-18}$alkylene-C(O)-; -NR$^a$C(O)-$C_{1-18}$alkylene-C(O)-; -C(O)O-$C_{1-18}$alkylene-O-; -O-$C_{1-18}$alkylene-O-; -O-$C_{1-18}$alkylene-NR$^a$-; -OC(O)-$C_{1-18}$alkylene-NR$^a$-; -C(O)-$C_{1-18}$alkylene-NR$^a$-; -OC(O)-$C_{1-18}$alkylene-O-; -C(O)-$C_{1-18}$alkylene-O-; and-C(O)NR$^a$-$C_{1-18}$alkylene-NR$^a$- where R$^a$ is selected from hydrogen, $C_{1-18}$alkyl, $C_{1-18}$alkenyl, $C_{1-18}$alkynyl, $C_{6-18}$aryl, $C_{3-18}$carbocyclyl, $C_{3-18}$heteroaryl, $C_{3-18}$heterocyclyl, and $C_{7-18}$arylalkyl.

6. The bioerodible polymer - bioactive agent conjugate according to any one of claims 1 to 5, wherein R is a linear or branched optionally substituted hydrocarbon having from 1 to 12 carbon atoms.

7. The bioerodible polymer - bioactive agent conjugate according to any one of claims 1 to 6, wherein the bioactive agent (D) is selected from 5-alpha-reductase inhibitors, amebicides, aminosalicylates, anaesthetics (general and local), analgesics, angiotensin inhibitors, anorexiants, antacid agents, anti-angiogenic agents, antianginal agents, antiarrythmic agents, antiarthritic agents, antibiotics, antibacterial agents, antibodies, anticoagulants, anticonvulsants, antidepressants, antiepileptic agents, antifungals, anthelmintics, antihistamines, antihypertensives, antihyperlipidemic agents, antiinfectives, antiinflammatories, antiemetics, antimalarial, antimetabolites, antimigraine, antimitotics, antiparasitic agents, antiparkinson agents, antipsychotics, antiprotozoals, antitussives, antiulcer agents, antivirals, anxiolytics, bronchodilators, decongestants and expectorants, cancer therapy and related pharmaceuticals, cardiovascular pharmaceuticals, central nervous system pharmaceuticals, benzopidazepines, beta-adrenergic blocking agents, bisphosphonates, calcium channel blockers, carbonic anhydrase inhibitors, chemokine receptor antagonist, coumarins and indadiones, cox-2 inhibitors, contraceptives, cytotoxics, diuretics, diabetes therapies, growth hormones, fertility pharmaceuticals, hematinics, glucose modifying agents, growth promoters, H2 antagonists, heparin and heparin antagonists, hormone replacement therapies, hemostatics, immunosuppressants, immunostimulants, inotropic agents, interferons, hormones and analogs, impotence agents, kinase inhibitors, laxatives, leukotriene modifiers, macrolides, mast cell stabilizers, muscle relaxants/stimulants, mydiratics, neuromuscular blocking agents, obesity therapeutics, ophthalmic pharmaceuticals, osteoporosis drugs, pain therapeutics, peptides and polypeptides, peripheral vasodilators, platelet inhibitors/stimulating agents, prolactin inhibitors, protease inhibitors, protein therapeutics, proton pump inhibtors, radiopharmaceuticals, respiratory pharmaceuticals, sedatives, spermicides, steroids, smoking cessation agents, statins, stimulants and tranquilizers, sulphonamides, thyroid drugs, urinary acidifiers/alkalinisers, and vasodilators.

**8.** The bioerodible polymer - bioactive agent conjugate according to any one of claims 1 to 7, wherein the bioactive agent (D) is selected from fluoroquinolone antibiotics.

**9.** The bioerodible polymer - bioactive agent conjugate according to claim 8, wherein the bioactive agent (D) is selected from alatrofloxacin, balofloxacin, ciprofloxacin, clinafloxacin, danofoxacin, delafloxacin, dextrofloxacin, difloxacin, enoxacin, enrofloxacin, garenoxacin, gatifloxacin, gemifloxacin, grepafloxacin, levofloxacin, lomefloxacin, marbofloxacin, moxifloxacin, nadifloxacin, norfloxacin, ofloxacin, orbifloxacin, pefloxacin, sitafloxacin, sparfloxacin, temafloxacin, tosufloxacin, tosulfloxacin and trovafloxacin.

**10.** The bioerodible polymer - bioactive agent conjugate according to any one of claims 1 to 9, obtainable by polymerising a monomer - bioactive moiety conjugate of formula (II):

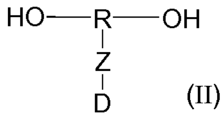

(II)

where:

R represents a linear or branched optionally substituted hydrocarbon;
Z is a spacer moiety; and
D is a releasable bioactive moiety;

with a polyisocyanate and a polyester polyol.

**11.** The bioerodible polymer - bioactive agent conjugate according to claim 10, wherein the polyisocyanate is selected from the group consisting of m-phenylene diisocyanate, p-phenylene diisocyanate, 2,4-toluene diisocyanate, 2,6-toluene diisocyanate, 1,6-hexamethylene diisocyanate, 1,4-hexamethylene diisocyanate, 1,3-cyclohexane diisocyanate, 1,4-cyclohexane diisocyanate, hexahydro-toluene diisocyanate and its isomers, isophorone diisocyanate, dicyclo-hexylmethane diisocyanates, 1,5-napthylene diisocyanate, 4,4'-diphenylmethane diisocyanate, 2,4' diphenylmethane diisocyanate, 4,4'-biphenylene diisocyanate, 3,3'-dimethoxy-4,4'-biphenylene diisocyanate, 3,3'-dimethyl-diphenylpropane-4,4'-diisocyanate, 2,4,6-toluene triisocyanate, 4,4'-dimethyl-diphenylmethane-2,2',5,5'-tetraisocyanate, polymethylene polyphenhyl polyisocyanates and alkyl esters of lysine diisocyanate (preferably ethyl ester of lysine diisocyanate) and combinations thereof.

**12.** The bioerodible polymer - bioactive agent conjugate according to any one of claims 10 or claim 11, wherein the polyester polyol is selected from the group consisting of polycaprolactone diol (PCLD), poly(DL lactide) (DLLA) and poly(lactic acid-co-glycolic acid) (PLGA) and combinations thereof.

**13.** A process for preparing a bioerodible polymer - bioactive agent conjugate according to any one of claims 1 to 9, said process comprising the step of polymerising a monomer - bioactive agent conjugate of formula (II):

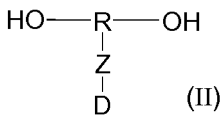

(II)

wherein:

R, Z and D are as defined in any one of claims 1 to 9;

with a polyisocyanate and a polyester polyol.

**14.** A monomer - bioactive agent conjugate that is suitable for use in preparing a bioerodible polymer - bioactive agent conjugate, the monomer - bioactive agent conjugate having a structure of general formula (II):

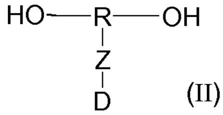

(II)

wherein:

R represents a linear or branched optionally substituted hydrocarbon;
Z is a linking group; and
D is a releasable bioactive agent selected from fluoroquinolone antibiotics.

**15.** A monomer - bioactive agent conjugate according to claim 14, wherein the bioactive agent (D) is selected from alatrofloxacin, balofloxacin, ciprofloxacin, clinafloxacin, danofoxacin, delafloxacin, dextrofloxacin, difloxacin, enoxacin, enrofloxacin, garenoxacin, gatifloxacin, gemifloxacin, grepafloxacin, levofloxacin, lomefloxacin, marbofloxacin, moxifloxacin, nadifloxacin, norfloxacin, ofloxacin, orbifloxacin, pefloxacin, sitafloxacin, sparfloxacin, temafloxacin, tosufloxacin, tosulfloxacin and trovafloxacin.

## Patentansprüche

**1.** Konjugat aus bioerodierbarem Polymer / biologischem Wirkstoff, das als Bestandteil seines Polymergerüsts einen Baustein der allgemeinen Formel (I) umfasst:

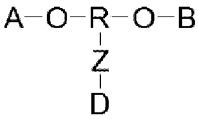

(I)

wobei:

A und B, welche gleichartig oder verschiedenartig sein können, für den übrigen Teil des Polymergerüsts stehen, wobei sie (i) über einen bioerodierbaren Baustein an den -O-R(ZD)-O-Baustein gebunden sind, wie in Formel (I) dargestellt ist, und (ii) einen Polyurethanester umfassen, der Monomereinheiten aufweist, welche über bioerodierbare Urethan- oder Esterbausteine gekuppelt sind;
R für einen geradkettigen oder verzweigten, möglicherweise substituierten, Kohlenwasserstoff steht;
Z für eine Verbindungsgruppe steht; und
D für einen freisetzbaren biologischen Wirkstoff steht.

**2.** Konjugat aus bioerodierbarem Polymer / biologischem Wirkstoff gemäß Anspruch 1, wobei A und B jeweils ein Copolymer aus Polyurethan und Polyester umfassen.

**3.** Konjugat aus bioerodierbarem Polymer / biologischem Wirkstoff gemäß Anspruch 1 oder Anspruch 2, wobei es weniger als 25 Mol-% an polymerisierten Resten umfasst, die sich von einem $C_2$-Diol ableiten, bezogen auf die Gesamtmolanzahl an polymerisierten Diolresten.

**4.** Konjugat aus bioerodierbarem Polymer / biologischem Wirkstoff gemäß einem beliebigen der Ansprüche 1 bis 3, wobei D mittels eines Ester-, Amid-, Anhydrid-, Imid-, Carbonat-, Peroxid-, Peroxyester-, Thiol-, Phosphatester-, Thioester-, Sulfatester-, Carbamat-, Azo- oder Boronatesterbausteins durch Z an R gekuppelt ist.

**5.** Konjugat aus bioerodierbarem Polymer / biologischem Wirkstoff gemäß Anspruch 4, wobei Z aus-O-; -C(O)-; und aus möglicherweise substituiertem:-OC (O)-$C_{1-18}$alkylen-C(O)-; -C(O)-O$C_{1-18}$alkylen-C(O)-;-NR$^a$C(O)-$C_{1-18}$alkylen-C(O)-; -C(O)O-$C_{1-18}$alkylen-O-; -O-$C_{1-18}$alkylen-O-; -O-$C_{1-18}$alkylen-NR$^a$-;

-OC(O)-$C_{1-18}$alkylen-$NR^a$-; -C(O)-$C_{1-18}$alkylen-$NR^a$-; -OC(O)-$C_{1-18}$alkylen-O-;-C(O)-$C_{1-18}$alkylen-O-; und -C(O)$NR^a$-$C_{1-18}$alkylen-$NR^a$-ausgewählt ist, wobei $R^a$ aus Wasserstoff, $C_{1-18}$Alkyl, $C_{1-18}$Alkenyl, $C_{1-18}$Alkinyl, $C_{6-18}$Aryl, $C_{3-18}$Carbocyclyl, $C_{3-18}$Heteroaryl, $C_{3-18}$Heterocyclyl und $C_{7-18}$Arylalkyl ausgewählt ist.

**6.** Konjugat aus bioerodierbarem Polymer / biologischem Wirkstoff gemäß einem beliebigen der Ansprüche 1 bis 5 ausgewählt ist, wobei R für einen geradkettigen oder verzweigten, möglicherweise substituierten, Kohlenwasserstoff mit 1 bis 12 Kohlenstoffatomen steht.

**7.** Konjugat aus bioerodierbarem Polymer / biologischem Wirkstoff gemäß einem beliebigen der Ansprüche 1 bis 6 steht, wobei der biologische Wirkstoff (D) aus den 5-alpha-Reductase-Hemmern, Amöbiziden, Aminosalicylaten, Anästhetika (allgemeiner and lokaler Art), Schmerzmitteln, Angiotensin-Hemmern, Appetitzüglern, säurebindenden Mitteln, Angiogenese-Hemmern, Mitteln gegen Angina pectoris, Mitteln gegen Herzrhythmusstörungen, Mitteln gegen Arthritis, Antibiotika, antibakteriellen Mitteln, Antikörpern, Gerinnungshemmern, Antikonvulsiva, Antidepressiva, Antiepileptika, pilzbekämpfenden Mitteln, Entwurmungsmitteln, Antihistaminika, blutdrucksenkenden Mitteln, Mitteln gegen Hyperlipidämie, infektionsbekämpfenden Mitteln, entzündungshemmenden Mitteln, Antiemetika, malariabekämpfenden Mitteln, Antimetaboliten, migränebekämpfenden Mitteln, Antimitosemitteln, parasitenbekämpfenden Mitteln, Parkinson-bekämpfenden Mitteln, Neuroleptika, protozoenbekämpfenden Mitteln, hustenlindernden Mitteln, geschwürbekämpfenden Mitteln, antiviralen Mitteln, Angstlösern, bronchienerweiternden Mitteln, nasenabschwellenden Mitteln und Hustenlösern, Krebstherapiemitteln und verwandten Arzneimitteln, Herz-Kreislauf-Arzneimitteln, Arzneimitteln für das Zentrale Nervensystem, Benzopidazepinen, Betablockern, Bisphosphonaten, Calciumkanalblockern, Carboanhydrase-Hemmern, Chemokinrezeptor-Antagonisten, Cumarinen und Indadionen, Cox-2-Hemmern, Verhütungsmitteln, Zytotoxinen, harntreibenden Mitteln, Diabetes-Therapiemitteln, Wachstumshormonen, fruchtbarkeitsbeeinflussenden Arzneimitteln, hämatopoesefördernden Mitteln, glucosemodifizierenden Mitteln, wachstumsfördernden Mitteln, H2-Antagonisten, Heparin und Heparin-Antagonisten, Hormonersatztherapie-Mitteln, Hämostatika, Immunsuppressiva, Immunstimulantien, inotropen Mitteln, Interferonen, Hormonen und Analoga, Mitteln gegen Impotenz, Kinase-Inhibitoren, Abführmitteln, Leukotrien-modifizierenden Mitteln, Makroliden, Mastzell-Stabilisatoren, Muskelrelaxantien/stimulatien, Mydriatika, neuromuskulären Blockern, Therapiemitteln gegen Fettleibigkeit, augenmedizinischen Arzneimitteln, Osteoporose-Arzneistoffen, Schmerztherapeutika, Peptiden und Polypeptiden, Mitteln zur Erweiterung peripherer Gefäße, Mitteln zur Hemmung/Stimulierung der Blutplättchen, Prolactin-Hemmern, Protease-Hemmern, Protein-Therapiemitteln, Protonenpumpenhemmern, Radiopharmaka, die Atmung beeinflussenden Arzneimitteln, Sedativa, Spermiziden, Steroiden, Mitteln zur Rauchtabakentwöhnung, Statinen, Stimulantien und Beruhigungsmitteln, Sulfonamiden, Schilddrüsen-Arzneistoffen, Mitteln zur pH-Werteinstellung des Harns und den gefäßerweiternden Mitten ausgewählt ist.

**8.** Konjugat aus bioerodierbarem Polymer / biologischem Wirkstoff gemäß einem beliebigen der Ansprüche 1 bis 7, wobei der biologische Wirkstoff (D) aus den Fluorchinolon-Antibiotika ausgewählt ist.

**9.** Konjugat aus bioerodierbarem Polymer / biologischem Wirkstoff gemäß Anspruch 8, wobei der biologische Wirkstoff (D) aus Alatrofloxacin, Balofloxacin, Ciprofloxacin, Clinafloxacin, Danofoxacin, Delafloxacin, Dextrofloxacin, Difloxacin, Enoxacin, Enrofloxacin, Garenoxacin, Gatifloxacin, Gemifloxacin, Grepafloxacin, Levofloxacin, Lomefloxacin, Marbofloxacin, Moxifloxacin, Nadifloxacin, Norfloxacin, Ofloxacin, Orbifloxacin, Pefloxacin, Sitafloxacin, Sparfloxacin, Temafloxacin, Tosufloxacin, Tosulfloxacin und Trovafloxacin ausgewählt ist.

**10.** Konjugat aus bioerodierbarem Polymer / biologischem Wirkstoff gemäß einem beliebigen der Ansprüche 1 bis 9, wobei es erhalten werden kann, indem ein Konjugat aus Monomer / biologischem Wirkstoff nach Formel (II):

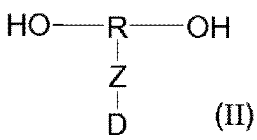

(II)

wobei:

R für einen geradkettigen oder verzweigten, möglicherweise substituierten, Kohlenwasserstoff steht;
Z für einen abstandhaltenden Baustein steht; und
D für einen freisetzbaren biologisch wirksamen Baustein steht;

mit einem Polyisocyanat und einem Polyesterpolyol polymerisiert wird.

**11.** Konjugat aus bioerodierbarem Polymer / biologischem Wirkstoff gemäß Anspruch 10, wobei das Polyisocyanat aus der Gruppe ausgewählt ist, welche aus m-Phenylendiisocyanat, p-Phenylendiisocyanat, 2,4-Toluoldiisocyanat, 2,6-Toluoldiisocyanat, 1,6-Hexamethylendiisocyanat, 1,4-Hexamethylendiisocyanat, 1,3-Cyclohexandiisocyanat, 1,4-Cyclohexandiisocyanat, Hexahydrotoluoldiisocyanat und dessen Isomeren, Isophorondiisocyanat, Dicyclohexylmethandiisocyanaten, 1,5-Naphthylendiisocyanat, 4,4'-Diphenylmethandiisocyanat, 2,4'-Diphenylmethandiisocyanat, 4,4'-Biphenylendiisocyanat, 3,3'-Dimethoxy-4,4'-biphenylendiisocyanat, 3,3'-Dimethyldiphenylpropan-4,4'-diisocyanat, 2,4,6-Toluoltriisocyanat, 4,4'-Dimethyldiphenylmethan-2,2',5,5'-tetraisocyanat, Polymethylenpolyphenylpolyisocyanaten und Alkylestern von Lysindiisocyanat (vorzugsweise Ethylester von Lysindiisocyanat) und deren Kombinationen besteht.

**12.** Konjugat aus bioerodierbarem Polymer / biologischem Wirkstoff gemäß einem beliebigen von Anspruch 10 oder Anspruch 11, wobei der Polyesterpolyol aus der Gruppe ausgewählt ist, die aus Polycaprolactondiol (PCLD), Poly(DL-lactid) (DLLA) und Poly(Milchsäure-co-Glykolsäure) (PLGA) und deren Kombinationen besteht.

**13.** Verfahren zur Herstellung eines Konjugats aus bioerodierbarem Polymer / biologischem Wirkstoff gemäß einem beliebigen der Ansprüche 1 bis 9, wobei das Verfahren den Schritt umfasst, in welchem ein Konjugat aus Monomer / biologischem Wirkstoff nach Formel (II):

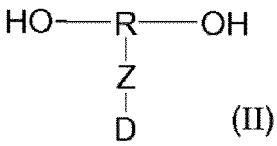
(II)

wobei:

R, Z und D den Begriffsbestimmungen in einem beliebigen der Ansprüche 1 bis 9 entsprechen;
mit einem Polyisocyanat und einem Polyesterpolyol polymerisiert wird.

**14.** Konjugat aus Monomer / biologischem Wirkstoff, das dafür geeignet ist, beim Herstellen eines Konjugats aus bioerodierbarem Polymer / biologischem Wirkstoff verwendet zu werden, wobei das Konjugat aus Monomer / biologischem Wirkstoff eine Struktur nach der allgemeinen Formel (II) aufweist:

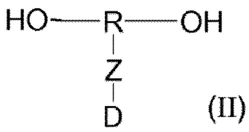
(II)

wobei:

R für einen geradkettigen oder verzweigten, möglicherweise substituierten, Kohlenwasserstoff steht;
Z für eine Verbindungsgruppe steht; und
D für einen freisetzbaren biologischen Wirkstoff steht, der aus den Fluorchinolon-Antibiotika ausgewählt ist.

**15.** Konjugat aus Monomer / biologischem Wirkstoff gemäß Anspruch 14, wobei der biologische Wirkstoff (D) aus Alatrofloxacin, Balofloxacin, Ciprofloxacin, Clinafloxacin, Danofoxacin, Delafloxacin, Dextrofloxacin, Difloxacin, Enoxacin, Enrofloxacin, Garenoxacin, Gatifloxacin, Gemifloxacin, Grepafloxacin, Levofloxacin, Lomefloxacin, Marbofloxacin, Moxifloxacin, Nadifloxacin, Norfloxacin, Ofloxacin, Orbifloxacin, Pefloxacin, Sitafloxacin, Sparfloxacin, Temafloxacin, Tosufloxacin, Tosulfloxacin und Trovafloxacin ausgewählt ist.

## Revendications

**1.** Conjugué polymère bioérodable - agent bioactif comprenant, en tant que partie de son squelette de polymère, un fragment de formule générale (I) :

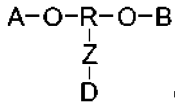

(I)

dans lequel :

A et B, qui peuvent être identiques ou différents, représentent le reste du squelette de polymère et sont (i) liés au fragment -O-R(ZD)-O- tel que décrit dans la formule (I) par l'intermédiaire d'un fragment bioérodable, et (ii) comprennent un a poly(uréthane-ester) ayant des motifs monomères couplés par l'intermédiaire de fragments uréthane ou ester bioérodables ;
R représente un hydrocarbure facultativement substitué linéaire ou ramifié ;
Z est un groupe de liaison ; et
D est un agent bioactif amovible.

2. Conjugué polymère bioérodable - agent bioactif selon la revendication 1, dans lequel A et B comprennent chacun un copolymère de polyuréthane et de polyester.

3. Conjugué polymère bioérodable - agent bioactif selon la revendication 1 ou la revendication 2, qui comprend moins de 25 % en moles de résidus polymérisés qui sont dérivés d'un diol en $C_2$, par rapport au nombre total de moles de résidus diol polymérisés.

4. Conjugué polymère bioérodable - agent bioactif selon l'une quelconque des revendications 1 à 3, dans lequel D est couplé par l'intermédiaire de Z à R par un fragment ester, amide, anhydride, imide, carbonate, peroxyde, peroxyester, thiol, ester de phosphate, thioester, ester de sulfate, carbamate, azoïque ou ester de boronate.

5. Conjugué polymère bioérodable - agent bioactif selon la revendication 4, dans lequel Z est choisi parmi -O- ; -C(O)- ; et, facultativement substitué : -OC(O)-(alkylène en $C_{1-18}$)-C(O)- ; -C(O)O-(alkylène en $C_{1-18}$)-C(O)- ; -$NR^a$C(O)-(alkylène en $C_{1-18}$)-C(O)- ; -C(O)O- (alkylène en $C_{1-18}$)-O- ; -O-(alkylène en $C_{1-18}$)-O- ; -O-(alkylène en $C_{1-18}$)-$NR^a$- ; -OC(O)-(alkylène en $C_{1-18}$)-$NR^a$- ; -C(O)-(alkylène en $C_{1-18}$)-$NR^a$- ; -OC(O)-(alkylène en $C_{1-18}$)-O- ; -C(O)-(alkylène en $C_{1-18}$)-O- ; et -C(O)$NR^a$-(alkylène en $C_{1-18}$)-$NR^a$- où $R^a$ est choisi parmi hydrogène, alkyle en $C_{1-18}$, alcényle en $C_{1-18}$, alcynyle en $C_{1-18}$, aryle en $C_{6-18}$, carbocyclyle en $C_{3-18}$, hétéroaryle en $C_{3-18}$, hétérocyclyle en $C_{3-18}$ et arylalkyle en $C_{7-18}$.

6. Conjugué polymère bioérodable - agent bioactif selon l'une quelconque des revendications 1 à 5, dans lequel R est un hydrocarbure facultativement substitué, linéaire ou ramifié ayant de 1 à 12 atomes de carbone.

7. Conjugué polymère bioérodable - agent bioactif selon l'une quelconque des revendications 1 à 6, dans lequel l'agent bioactif (D) est choisi parmi des inhibiteurs de 5-alpha-réductase, amibicides, aminosalicylates, anesthésiques (généraux et locaux), analgésiques, inhibiteurs d'angiotensine, anorexigènes, agents antiacides, agents antiangiogenèse, agents antiangineux, agents antiarythmiques, agents antiarthritiques, antibiotiques, agents antibactériens, anticorps, anticoagulants, anticonvulsivants, antidépresseurs, agents antiépileptiques, antifongiques, anthelminthiques, antihistaminiques, antihypertenseurs, agents antihyperlipidémiques, anti-infectieux, anti-inflammatoires, antiémétiques, antipaludéens, antimétabolites, antimigraineux, antimitotiques, agents antiparasitaires, agents antiparkinsoniens, antipsychotiques, antiprotozoaires, antitussifs, agents antiulcéreux, antiviraux, anxiolytiques, bronchodilatateurs, décongestionnants et expectorants, thérapie anticancéreuse et agents pharmaceutiques associés, agents pharmaceutiques cardiovasculaires, agents pharmaceutiques du système nerveux central, benzodiazépines, agents bloquants bêta-adrénergiques, biphosphonates, inhibiteurs calciques, inhibiteurs d'anhydrase carbonique, antagoniste de récepteur de chimiokine, coumarines et indadiones, inhibiteurs de cox-2, contraceptifs, cytotoxiques, diurétiques, thérapies du diabète, hormones de croissance, agents pharmaceutiques de fertilité, hématiniques, agents modifiant le glucose, promoteurs de croissance, antagonistes de H2, héparine et antagonistes d'héparine, hormonothérapies substitutives, hémostatiques, immunosuppresseurs, immunostimulants, agents inotropes, interférons, hormones et analogues, agents contre l'impuissance, inhibiteurs de kinases, laxatifs, modificateurs de leucotriènes, macrolides, stabilisateurs de mastocytes, myorelaxants/myostimulants, mydiratiques, agents bloquants neuromusculaires, agents thérapeutiques contre l'obésité, agents pharmaceutiques ophtalmiques, médicaments contre l'ostéoporose, agents thérapeutiques contre la douleur, peptides et polypeptides, vasodilatateurs périphériques, agents inhibiteurs/stimulants plaquettaires, inhibiteurs de prolactine, inhibiteurs de protéase, agents thérapeutiques protéiques, inhibiteurs de pompe à protons, agents radiopharmaceutiques, agents pharmaceutiques

respiratoires, sédatifs, spermicides, stéroïdes, agents de sevrage tabagique, statines, stimulants et tranquillisants, sulfonamides, médicaments thyroïdiens, acidifiants/alcalinisants urinaires et vasodilatateurs.

**8.** Conjugué polymère bioérodable - agent bioactif selon l'une quelconque des revendications 1 à 7, dans lequel l'agent bioactif (D) est choisi parmi des antibiotiques de type fluoroquinolone.

**9.** Conjugué polymère bioérodable - agent bioactif selon la revendication 8, dans lequel l'agent bioactif (D) est choisi parmi les alatrofloxacine, balofloxacine, ciprofloxacine, clinafloxacine, danofoxacine, délafloxacine, dextrofloxacine, difloxacine, énoxacine, enrofloxacine, garénoxacine, gatifloxacine, gémifloxacine, grepafloxacine, lévofloxacine, loméfloxacine, marbofloxacine, moxifloxacine, nadifloxacine, norfloxacine, ofloxacine, orbifloxacine, péfloxacine, sitafloxacine, sparfloxacine, témafloxacine, tosufloxacine, tosulfloxacine et trovafloxacine.

**10.** Conjugué polymère bioérodable - agent bioactif selon l'une quelconque des revendications 1 à 9, pouvant être obtenu par polymérisation d'un conjugué monomère - fragment bioactif de formule (II) :

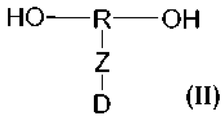

**(II)**

où :

R représente un hydrocarbure facultativement substitué linéaire ou ramifié ;
Z est un fragment espaceur ; et
D est un fragment bioactif amovible ;
avec un polyisocyanate et un polyester polyol.

**11.** Conjugué polymère bioérodable - agent bioactif selon la revendication 10, dans lequel le polyisocyanate est choisi dans le groupe constitué des diisocyanate de m-phénylène, diisocyanate de p-phénylène, diisocyanate de 2,4-toluène, diisocyanate de 2,6-toluène, diisocyanate de 1,6-hexaméthylène, diisocyanate de 1,4-hexaméthylène, diisocyanate de 1,3-cyclohexane, diisocyanate de 1,4-cyclohexane, diisocyanate d'hexahydrotoluène et ses isomères, diisocyanate d'isophorone, diisocyanates de dicyclohexylméthane, diisocyanate de 1,5-napthylène, diisocyanate de 4,4'-diphénylméthane, diisocyanate de 2,4'-diphénylméthane, diisocyanate de 4,4'-biphénylène, diisocyanate de 3,3'-diméthoxy-4,4'-biphénylène, 4,4'-diisocyanate de 3,3'-diméthyl-diphénylpropane, triisocyanate de 2,4,6-toluène, 2,2',5,5'-tétraisocyanate de 4,4'-diméthyl-diphénylméthane, polyméthylène-poly(isocyanates de phényle) et des esters d'alkyle de diisocyanate de lysine (de préférence l'ester éthylique de diisocyanate de lysine) et des combinaisons de ceux-ci.

**12.** Conjugué polymère bioérodable - agent bioactif selon l'une quelconque de la revendication 10 ou la revendication 11, dans lequel le polyester polyol est choisi dans le groupe constitué de polycaprolactone diol (PCLD), poly(DL-lactide) (DLLA) et poly(acide lactique-co-acide glycolique) (PLGA) et des combinaisons de ceux-ci.

**13.** Procédé de préparation d'un conjugué polymère bioérodable - agent bioactif selon l'une quelconque des revendications 1 à 9, ledit procédé comprenant l'étape de polymérisation d'un conjugué monomère - agent bioactif de formule (II) :

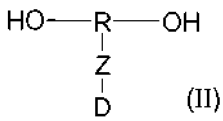

(II)

dans lequel :

R, Z et D sont tels que définis dans l'une quelconque des revendications 1 à 9 ;
avec un polyisocyanate et un polyester polyol.

**14.** Conjugué monomère - agent bioactif qui est adapté pour utilisation dans la préparation d'un conjugué polymère bioérodable - agent bioactif , le conjugué monomère - agent bioactif ayant une structure de formule générale (II) :

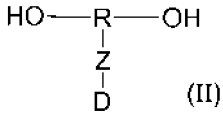

(II)

dans laquelle :

R représente un hydrocarbure facultativement substitué linéaire ou ramifié ;
Z est un groupe de liaison ; et
D est un agent bioactif amovible choisi parmi des antibiotiques de type fluoroquinolone.

**15.** Conjugué monomère - agent bioactif selon la revendication 14, dans lequel l'agent bioactif (D) est choisi parmi les alatrofloxacine, balofloxacine, ciprofloxacine, clinafloxacine, danofoxacine, délafloxacine, dextrofloxacine, difloxacine, énoxacine, enrofloxacine, garénoxacine, gatifloxacine, gémifloxacine, grépafloxacine, lévofloxacine, loméfloxacine, marbofloxacine, moxifloxacine, nadifloxacine, norfloxacine, ofloxacine, orbifloxacine, péfloxacine, sitafloxacine, sparfloxacine, témafloxacine, tosufloxacine, tosulfloxacine et trovafloxacine.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008060780 A
- WO 2006024953 A
- WO 0226867 A
- WO 2005089778 A1


### Non-patent literature cited in the description


- **DAVARAN ; ENTEZAMI.** *Journal of Bioactive and Compatible Polymers,* January 1997, vol. 12, 47-58
- **OUCHI et al.** *British Polymer Journal,* 1990, vol. 23, 221-228
- *Tetrahedron,* 08 March 2004, vol. 60 (11), 2447-2467
- **MARTIN.** Remington's Pharmaceutical Sciences. Mack Publishing Co, 1990
- **T.W. GREENE ; P.G.M. WUTS.** Protective Groups in Organic Chemistry. John Wiley and Sons, 1991
- **OH, H.-J. ; KIM, W.-Y. ; JEONG, Y.-S. ; LEE, Y.-S.** Membranes of Polyurethanes Containing Crystalline Soft Segments: Oxygen Permeability and Morphology. *Bull. Korean Chem. Soc.,* 2001, vol. 22 (2), 194-8
- **DEE, L. A. ; BIGGERS, G. L. ; FISKE, M. E.** N-Methylimidazole as a Catalyst for Acetylation of Hydroxyl Terminated Polymers. *Anal. Chem.,* 1980, vol. 52, 572-3
- *ASTM E200-97 (Sections 74 to 79). Standard Practice for Preparation, Standardisation and Storage of Standard and Reagent Solutions for Chemical Analysis - superseded by ASTM E200-97,* 2001, vol. 15.05, e1
- **STOREY, R. F. ; HERRING, K. R. ; HOFFMAN, D. C.** Hydroxy-Terminated Poly (e-Caprolactone-co-Valerolactone) Oligomers: Synthesis, Characterisation and Polyurethane Network Formation. *J. Polymer Science (Part A: Polymer Chemistry),* 1991, vol. 29, 1759-77